(19) Europäisches Patentamt
European Patent Office
Office européen des brevets

(11) EP 1 668 029 B1

(12) EUROPEAN PATENT SPECIFICATION

(45) Date of publication and mention of the grant of the patent:
**25.12.2013 Bulletin 2013/52**

(21) Application number: **04786063.0**

(22) Date of filing: **14.07.2004**

(51) Int Cl.:
***C07K 14/44*** *(2006.01)* ***C12N 15/30*** *(2006.01)*
***A61K 39/018*** *(2006.01)* ***G01N 33/569*** *(2006.01)*
***C07K 16/20*** *(2006.01)* ***C12N 15/10*** *(2006.01)*

(86) International application number:
**PCT/US2004/022605**

(87) International publication number:
**WO 2005/007691 (27.01.2005 Gazette 2005/04)**

(54) **EAST COAST FEVER VACCINE BASED ON CTL-SPECIFIC SCHIZONT ANTIGENS**

AUF CTL-SPEZIFISCHEN SCHIZONT-ANTIGENEN BASIERENDER EAST-COAST-FIEBER-IMPFSTOFF

VACCIN CONTRE LA THEILERIOSE BOVINE BASEE SUR DES ANTIGENES SCHIZONT SPECIFIQUES A CTL

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **14.07.2003 US 486750 P**

(43) Date of publication of application:
**14.06.2006 Bulletin 2006/24**

(73) Proprietor: **International Livestock Research Institute**
**00100 Nairobi (KE)**

(72) Inventors:
- **TARACHA, Evans**
  **Nairobi (KE)**
- **GLEW, Jane**
  **Birchwood Park**
  **Risley**
  **Warrington WA3 6AT (KE)**
- **GRAHAM, Simon**
  **Nairobi 001000 (KE)**
- **MWANGI, Duncan**
  **Nairobi 00100 (KE)**
- **HONDA, Yoshikazu**
  **Nairobi 00100 (KE)**
- **PELLE, Roger**
  **Nairobi 00100 (KE)**
- **ONUKARI, Nyerhovwo, J.**
  **Nairobi 00100 (KE)**
- **YAMAGE, Matasuke**
  **Nairobi 00100 (KE)**
- **NENE, Vishvanath**
  **Potomac, MD 20854 (US)**

(74) Representative: **Harding, Charles Thomas**
**D Young & Co LLP**
**120 Holborn**
**London**
**EC1N 2DY (GB)**

(56) References cited:
**US-A- 5 273 744**

- **DATABASE EMBL [Online] 2 January 2003 (2003-01-02), "EST629769 TpMugugaSh01 Theileria parva cDNA clone TPFAU35, mRNA sequence." XP002310756 retrieved from EBI accession no. EM_EST:BQ546142 Database accession no. BQ546142**
- **GERHARDS JOACHIM ET AL: "Sequence and expression of a 90-kilodalton heat-shock protein family member of Theileria parva" MOLECULAR AND BIOCHEMICAL PARASITOLOGY, vol. 68, no. 2, 1994, pages 235-246, XP002310752 ISSN: 0166-6851 -& DATABASE EMBL [Online] 21 February 1991 (1991-02-21), "T.parva heat shock protein 90 (hsp90) mRNA, complete cds." XP002323332 retrieved from EBI accession no. EM_INV:TPHSP90 Database accession no. TPHSP90**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE EMBL [Online] 2 January 2003 (2003-01-02), "EST630891 TpMugugaSh01 Theileria parva cDNA clone TPFDB90, mRNA sequence." XP002323333 retrieved from EBI accession no. EM_EST:BQ547264 Database accession no. BQ547264
- GARDNER M J ET AL: "GENOME SEQUENCE OF THE HUMAN MALARIA PARASITE PLASMODIUM FALCIPARUM" NATURE, MACMILLAN JOURNALS LTD. LONDON, GB, vol. 419, 2002, pages 498-511, XP001156336 ISSN: 0028-0836 -& DATABASE UniProt [Online] 1 March 2003 (2003-03-01), "Translation initiation factor eIF-1A, putative." XP002323334 retrieved from EBI accession no. UNIPROT:Q8IHT2 Database accession no. Q8IHT2
- DATABASE EMBL [Online] 2 January 2003 (2003-01-02), "EST630890 TpMugugaSh01 Theileria parva cDNA clone TPFDB90, mRNA sequence." XP002323335 retrieved from EBI accession no. EM_EST:BQ547263 Database accession no. BQ547263
- DATABASE EMBL [Online] 2 January 2003 (2003-01-02), "EST630359 TpMugugaSh01 Theileria parva cDNA clone TPFAY47, mRNA sequence." XP002323336 retrieved from EBI accession no. EM_EST:BQ546732 Database accession no. BQ546732
- DATABASE EMBL [Online] 2 January 2003 (2003-01-02), "EST628661 TpMugugaSh01 Theileria parva cDNA clone TPFAM54, mRNA sequence." XP002323337 retrieved from EBI accession no. EM_EST:BQ545034 Database accession no. BQ545034
- BALLINGALL K T ET AL: "A highly sensitive, non-radioactive assay for T cell activation in cattle: applications in screening for antigens recognised by CD4<+> and CD8<+> T cells" JOURNAL OF IMMUNOLOGICAL METHODS, ELSEVIER SCIENCE PUBLISHERS B.V., AMSTERDAM, NL, vol. 239, no. 1-2, May 2000 (2000-05), pages 85-93, XP004204319 ISSN: 0022-1759
- MORRISON W IVAN ET AL: "Theileriosis: Progress towards vaccine development through understanding immune responses to the parasite" VETERINARY PARASITOLOGY, vol. 57, no. 1-3, 1995, pages 177-187, XP002310754 ISSN: 0304-4017 cited in the application
- MCKEEVER DECLAN J ET AL: "Novel vaccines against Theileria parva: Prospects for sustainability" INTERNATIONAL JOURNAL FOR PARASITOLOGY, vol. 28, no. 5, May 1998 (1998-05), pages 693-706, XP002310753 ISSN: 0020-7519
- NIALL D. MACHUGH ET AL: 'CD8+ T-cell responses to Theileria parva are preferentially directed to a single dominant antigen: Implications for parasite strain-specific immunity' EUROPEAN JOURNAL OF IMMUNOLOGY vol. 39, no. 9, 01 September 2009, pages 2459 - 2469, XP055021150 DOI: 10.1002/eji. 200939227 ISSN: 0014-2980

**Description**

**BACKGROUND INFORMATION**

Theilerioses are a group of disease syndromes affecting cattle, sheep, goats and domestic buffalo caused by tick-borne haemo-protozoan parasites in the genus *Theileria.* The most economically important diseases include Mediterranean fever, East Coast fever (ECF) and malignant theileriosis. Mediterranean fever caused by *Theileria annulata* occurs in North Africa, southern Europe, Near East, Middle East and many parts of Far East Asia with a population of 200 million cattle and buffalo at risk. ECF, caused by T. *parva,* affects 30 million cattle in eastern, central and southern Africa. Malignant theileriosis caused by *T. lestoquardi* affects sheep and goats in southeastern Europe, North Africa, the Near and Middle East and southern Russia and neighbouring States. These parasites belong to the same apicomplexan group as *Plasmodium falciparum, Toxoplasma gondii, Cytoxauzoon spp, Eimeria spp* and *Babesia spp,* with a life-cycle having the arthropod and mammalian components in which sexual and asexual stages develop, respectively. The pathogenic stages of *Theileria* parasites differ. *T. parva* causes a lympho-proliferative disorder in which schizont-infected lymphoblasts are responsible for the pathogenesis of the disease. On the other hand, anaemic disease caused by *T. lestoquardi* and *T. sergenti* is due to piroplasm-infected erythrocytes while both the schizont and piroplasm of *T. annulata* are pathogenic resulting in lympho-proliferative and anaemic syndromes, respectively.

Currently, theilerioses are controlled largely by tick control using acaricides and through "infection and treatment" vaccination protocols, of animals at risk. Such vaccination protocols are deemed effective in that an animal vaccinated in this manner will not develop ECF disease upon exposure to infectious *T. parva.* Due to cost and problems of tick resistance and environmental pollution, control of these diseases through acaricidal destruction of ticks is not sustainable. Vaccination, on the other hand, while effective, presents with certain shortcomings associated with the use of live vaccines. Owing to the ease of transmission of *T. annulata,* infected blood was originally used to immunise cattle by employing parasites of low virulence as the immunizing agent. However, such immunisations were still accompanied by clinically-detectable infection episodes. With the advent of *in vitro* cultivation of *T. annulata* (Sharma et al., 1998) and the development of bulk culture techniques in the 1960s, significant progress was made in realising a more practical immunisation strategy. Currently, passage-attenuated cultures of *T. annulata* are routinely used in national vaccination programs in affected countries. By contrast, similar efforts to immunise cattle against *T. parva* have been unsuccessful. This is attributed to the failure of attenuated *T. parva* parasites to induce immunity. In addition, much higher numbers of *T. parva*-infected cells are required to infect cattle reliably, since the schizonts of *T. parva* transfer at a low frequency and donor cells get rejected before successful transfer. *T. lestoquardi* has also been cultivated *in vitro* and studies have shown that attenuated parasites can be used to immunise animals with a degree of success.

Given the unsuccessful attempts to immunise cattle with attenuated *T. parva,* subsequent efforts have focused on the use of virulent parasites with accompanying chemotherapy. The rationale of this infection and treatment method (ITM) is to allow the infection to establish and suppress development of frank clinical disease by administering theileriacidal drugs. Animals thus immunised were found to be protected against the development of disease when exposed to the homologous parasite. This vaccination strategy has undergone successive refinements including the use of cryopreserved triturated tick stabilites containing sporozoites (the parasite stage infective for cattle lymphocytes) to standardise the immunisation-infection dose, as well as simultaneous drug administration. Further improvement of this immunisation approach has involved the identification and use of a combination of parasite stocks to broaden the immunising spectrum of the vaccine against several field *T. parva* parasite populations. The use of local parasite stocks to immunise in areas where they have been isolated is also practised. ITM immunisation against *T. parva* has been tested extensively under laboratory and field conditions and is now deployed in the affected region to control ECF.

ITM has a number of practical limitations that hinder its application as a sustainable control measure against ECF in those geographical areas most affected by the disease. Being live, it requires a cold refrigerator chain, which is impractical in Africa. ITM can also cause clinical disease if drug application is inadequate and it has the potential to introduce new parasite strains in areas under the vaccination campaign. The cost (US$10-20 per immunisation) of this current vaccine is well beyond the budget of poor farmers with cattle afflicted by ECF, due to both the cost of the drugs and the requirement for a trained veterinarian to administer the vaccine. Because of these concerns associated with the ITM vaccine protocol, a great deal of investment has been put in research work to develop a vaccine that will be sustainable and affordable.

Antigens of many parasitic protozoans that induce a protective antibody response against the development of disease have been identified. For example, the major merozoite surface protein of *Plasmodium* species has been shown to be a target of varying degrees of protective immunity against the asexual blood stages in rodent and human malaria. Vaccination of mice with purified P230, the major merozoite surface protein of the rodent malaria *Plasmodium yoelii,* has resulted in reduced parasitemias in comparison to controls upon intravenous challenge with a lethal dose ofparasitized erythrocytes (Holder et al. 1981. Nature 294:361). Mice have also been protected against *P. yoelii* by passive transfer of a monoclonal antibody (Mab) specific for P230 (Magarian et aL 1984. J. Immunol 132:3131). Mice have also been immunized against (rodent malaria) *Plasmodium chabaudi adami* challenge, by passive immunization with a monoclonal antibody specific for the homologous 250-kDa molecule of this *Plasmodium* species (Lew et al. 1989. Proc. Natl. Acad. Sci. USA 86:3768).

A 67 kDa glycoprotein (p67) from the surface of the *T. parva* sporozoite has been isolated (U.S. Patent Number 5273744) and used in a variety of immunization protocols, with little success reported so far, in the development of pratical levels of immune-mediated disease resistance. However, cattle recovering from a single infection with *T. parva* sporozoites resist infection upon homologous challenge. Such animals have weak antibody and T cell responses to p67. There is a need to identify *T. parva* antigens that can induce antigen-specific class I MHC-restricted $CD8^+$ cytotoxic T lymphocytes (CTLs).

## SUMMARY OF THE INVENTION

The present invention provides an isolated polypeptide, comprising a sequence shown by one of SEQ ID NO: 1, SEQ ID NO: 6, SEQ ID NO: 7 or SEQ ID NO: 9.

In another aspect, the present invention provides a pharmaceutical composition, comprising one or more polypeptides of the present invention and a pharmaceutically acceptable carrier.

In a further aspect, the present invention provides an immunogenic composition, comprising one or more polypeptides of the present invention and, optionally, an adjuvant.

The present invention provides, in a further aspect, an isolated polynucleotide comprising:

(a) a sequence shown by SEQ ID NO: 18, or SEQ ID NO : 23;
(b) a sequence which is at least about 90% identical to the sequence of (a);
(c) a sequence which encodes a polypeptide shown by SEQ ID NO: 1, SEQ ID NO: 6, SEQ ID NO: 7; or SEQ ID NO: 9; or
(d) a complement of any of (a), (b), or (c).

The present invention provides, in another aspect, a pharmaceutical composition comprising the polynucleotide of the present invention and a pharmaceutically acceptable carrier or excipient.

In another aspect, the present invention provides a recombinant construct, comprising a polynucleotide of the present invention, operably linked to an expression control sequence.

In a further aspect, the present invention provides a vector comprising the recombinant construct of the present invention.

The present invention provides, in another aspect, a host cell comprising a vector of the present invention.

In another aspect, the present invention provides a method for producing a polypeptide which stimulates a T. parva-antigen specific cytotoxic lymphocyte (CTL), comprising culturing a host cell of the present inveniton under conditions effective for producing a polypeptide encoded by the polynucleotide, and harvesting the polypeptide.

In a further aspect, the present invention provides an antibody specific for the polypeptide of the present invention.

The present invention provides, in a further aspect, a kit for detecting the presence of *T. parva* in a sample suspected of containing T. *parva,* or for purifying *T. parva* from a sample containing *T. parva,* comprising an antibody of the present invention.

The present invention provides, in another aspect, an *in vitro* method for detecting a pathogenic protozoan infection in a subject, comprising contacting peripheral blood monocytes from the subject with peptide-antigen pulsed cytotoxic T lymphocytes, wherein the cytotoxic T lymphocytes are obtained from an animal to which has been administered:

(i) a polypeptide of the present inventin, under conditions effective for the animal to generate *T. parva*-antigen-specific CTLs; or
(ii) a host cell of the present invention, under conditions effective for the animal to generate *T. parva*-antigen-specific CD4+ helper and CD8+ Cytotoxic T lymphocyte responses.

In another aspect, the present invention provides a method for detecting *T. parva* in a sample suspected of containing *T. parva,* comprising detecting in the sample a polynucleotide of the present invention.

In a further aspect, the present invention provides a method for preparing a polyclonal antibody, comprising immunizing an animal with one or more polypeptides of the present invention or with a host cell of the present invention.

The present invention provides, in another aspect, a method for preparing a monoclonal antibody, comprising:

(a) immunizing an animal with a polypeptide of the present invention, or with a host cell of the present invention,

(b) recovering cells from the animal which produce antibody that binds to the polypeptide,
(c) preparing a hybridoma with the cells isolated in (b), and
(d) recovering a monoclonal antibody from the hybridoma that binds to the polypeptide in (a).

In another aspect, the present invention provides a method for identifying *T. parva* in a sample suspected of containing *T. parva,* comprising contacting the sample with an antibody of the present invention, under conditions effective for the antibody to bind specifically to its cognate antigen, and detecting the presence of bound antibody.

The present description relates, *e.g.*, to methods for the identification of parasite antigens, such as *Theileria parva* antigens, that trigger antigen-specific cytotoxic T lymphocyte (CTLs) responses, for inducing immunoprotection against *T. parva* in bovine species and compositions identified by this method that can be used to generate a protective response in cattle, to resist the development of ECF disease, when challenged with infectious *T. parva* material. More particularly, the method includes steps wherein the stimulation of responding lymphocytes to cells transfected by cDNAs encoding parasite antigen is measured in a high throughput manner, by the release of soluble factors, such as gamma interferon, using either an antibody-elispot assay or a bioassay employing endothelial cells. The use of immortalized skin fibroblast cells from outbred animals that have recovered from exposure, as antigen presenting cells, enables the antigen identification, especially where cloned bovine MHC class I genes are not available for co-transfection into COS cells. In addition, high throughput protocols have been developed that allow the resolution of the identity of individual antigens from candidate cDNA pools. Candidate antigens and epitopes, and the nucleotide sequences that encode or result in the production of these candidate antigens and/or epitopes, identified using these method steps, can be used to stimulate CTLs and to successfully immunize cattle against infection with subsequent exposure to T. parva expressing such antigens.

One aspect of the description is an isolated polypeptide, comprising a sequence represented by one of SEQ ID NO:1 through SEQ ID NO:7; SEQ ID NO:9; or SEQ ID NO: 14 through SEQ ID NO: 17. Such isolated polypeptides are sometimes referred to herein as "polypeptides of the description." In embodiments, an isolated polypeptide of the description is in detectable amounts in isolates of *T. parva;* and/or comprises a *T. parva* antigen.

Another aspect of the invention is a pharmaceutical composition, which comprises one or more polypeptides of the invention and a pharmaceutically acceptable carrier or excipient. Another aspect of the invention is an immunogenic composition, which comprises one or more polypeptides of the invention and, optionally, an adjuvant The immunogenic composition may stimulate cytotoxic T cells specific to the polypeptide; and/or comprise an epitope that stimulates *Thelieria parva (T. parva)*- specific cytotoxic T cells. Another aspect of the description is a vaccine, which comprises one or more polypeptides of the invention and, optionally, an adjuvant. In an embodiment of the description the vaccine protects an animal against *T. parva* infection.

Another aspect of the description is an isolated polynucleotide comprising:

(a) a sequence represented by one of SEQ ID NO:18 through SEQ ID NO: 23 or SEQ ID NO: 28 through SEQ ID NO:31;
(b) a sequence which is at least about 90% identical to a sequence of (a);
(c) a sequence which hybridizes under conditions of high stringency to a polynucleotide which comprises a sequence of (a);
(d) a sequence which encodes a polypeptide represented by SEQ ID NO:1 through SEQ ID NO:7; SEQ ID NO;9; or SEQ ID NO: 14 through SEQ ID NO:17; or
(e) a complement of any of (a), (b), (c) or (d). Such isolated polynucleotides are sometimes referred to herein as "polynucleotides of the description."

Other aspects of the invention include a pharmaceutical composition comprising a polynucleotide of the invention and a pharmaceutically acceptable carrier or excipient; a recombinant construct comprising a polynucleotide of the invention, which is operably linked to an expression control sequence; and a vector comprising such a construct. A vector of the invention may further comprise one or more sequences encoding a selectable marker; and the vector may comprise a plasmid, a bacteriophage, a minichromosome or a eukaryotic virus vector. Another aspect of the description is a host cell (*e.g.*, a prokaryotic cell or a eukaryotic cell) which comprises a polynucleotide or a vector of the invention. Another aspect of the invention is a method for producing a polypeptide which stimulates a *T. parva*-antigen specific cytotoxic lymphocyte (CTL), comprising culturing a host cell of the invention under conditions effective for producing a polypeptide encoded by the polynucleotide, and harvesting the polypeptide.

Another aspect of the invention is an antibody (*e.g.*, a polyclonal antibody or a monoclonal antibody) specific for a polypeptide of the invention. In an embodiment of the invention, the antibody is coupled to a carrier and/or a label.

Another aspect of the invention is a method for preparing a polyclonal antibody, comprising immunizing an animal with one or more polypeptides of the invention, or with cells comprising polynucleotides or vectors of the invention. Another aspect of the invention is a method for preparing a monoclonal antibody, comprising (a) immunizing an animal with a polypeptide of the invention; (b) recovering cells from the animal which produce antibody that binds to the polypeptide; (c) preparing a hybridoma with the cells isolated in (b), and (d) recovering a monoclonal antibody from the hybridoma that binds to the polypeptide in (a). Another aspect of the description is a method for preparing a monoclonal antibody, comprising: (a) immunizing an animal with a host cell comprising a polynucleotide or vector of the invention; (b) recovering cells from the animal which produce antibody that binds to a polypeptide produced by the host cell; (c) preparing hybridomas with the cells isolated in (b), and (d) recovering a monoclonal antibody from the hybridoma that binds to the polypeptide in (b).

Another aspect of the invention is a kit for detecting the presence of *T. parva* in a sample suspected of containing *T. parva,* or for purifying *T. parva* from a sample containing *T. parva,* comprising an antibody of the invention. The kit may further comprise means for performing an enzyme-linked or Western blot assay to detect the presence of *T. parva*; and/or means for binding the antibody to *T. parva* in the sample, and for releasing the organism from the antibody.

Another aspect of the description is a method for protecting an animal against infection by *T. parva,* comprising administering to the animal a polypeptide of the invention, under conditions effective for the animal to generate a protective antibody against the polypeptide, or effective for the animal to generate *T. parva*-antigen-specific CTLs. Another aspect of the description is a method for protecting an animal against infection by *T. parva,* comprising administering to the animal a cell comprising a polynucleotide or vector of the invention, under conditions effective for the animal to generate a protective antibody against a polypeptide encoded by the polynucleotide (expressed from the polynucleotide), or effective for the animal to generate *T. parva*-antigen-specific CD4+ helper and CD8+ CTL responses.

Another aspect of the description is a method for detecting a pathogenic protozoan infection in a subject, comprising contacting peripheral blood monocytes from the subject with peptide-antigen pulsed cytotoxic T lymphocytes, wherein the cytotoxic T lymphocytes are obtained from an animal to which has been administered a polypeptide of the invention, under conditions effective for the animal to generate *T. parva*-antigen-specific CTLs, or effective for the animal to generate *T. parva*-antigen-specific CD4+ helper and CD8+ cytotoxic T lymphocyte responses. Another aspect of the invention is a method for detecting *T. parva* in a sample suspected of containing *T. parva,* comprising detecting in the sample a polynucleotide of the invention. Any of the methods of the invention, including these detection methods, may be high throughput methods.

Another aspect of the invention is a method for identifying *T. parva* in a sample suspected of containing *T. parva,* comprising contacting the sample with an antibody of the invention, under conditions effective for the antibody to bind specifically to its cognate antigen, and detecting the presence of bound antibody. In embodiments of the invention, the detection is carried out by enzyme immunoassay, radioimmunoassay, fluorescence immunoassay, flocculation, particle agglutination, flow microfluorimetry, a competition assay, or *in situ* chromogenic assay; the antibody is either a monoclonal antibody or a polyclonal antibody; the assay is quantitative; or the assay is high throughput.

Another aspect of the description is a method for the identification of parasite antigens that are targets of cytotoxic T lymphocytes, comprising co-culturing immortalized fibroblast cell lines transfected with pooled cDNA harvested from a pathogen, with clones of lines of cytotoxic T cells, generated in an animal that has been immunized, by infection and treatment with the pathogen and assaying the supernatant from the co-culture for the presence of a soluble factor. In embodiments of this method, the soluble factor is a cytokine, *e.g.* a gamma interferon; the pathogen is a protozoan organism (*e.g.* an organism in the genus *Theileria,* such as *T. parva*); the fibroblast cell line is of bovine origin; or the fibroblast cell line of bovine origin displays bovine Class I, MHC antigens. The method may further comprise assaying the supernatent of co-cultured cells, for the presence of a soluble factor, secreted by the cytotoxic T cells.

Another aspect of the description is a method for a multi-matrix (e.g., a three-way matrix) resolution for identification of a single cDNA clone from a pool of cDNAs, in high throughput procedures, comprising:

(a) preparing a culture of transformed cells by transforming bacterial cells with DNA from a pool of about 25 to about 500 cDNAs, wherein said pool has tested positive in a routine assay;
(b) diluting the culture of transformed cells so as to yield a density of about 500-5000 growth colonies per 150 cm2, when plated on agar-containing plates;
(c) picking about 100 to 500 colonies from the growth cultures;
(d) placing about 5 to 60 pools of about 10-100 individual cultures grown from the colonies, into numbered tubes, in such a manner such that each individual bacterial culture is present in more than one of said pools, so that tubes are labeled with a unique number and positioned so that a matrix of tubes is created so as to accommodate a multichannel pipetting device;
(e) creating a corresponding matrix table by arraying the numbers on the corresponding tubes containing the pools into a matrix table;
(f) testing the DNA from each of the tubes in a screening assay; and
(g) identifying the individual positive colony by comparing the results with the matrix array.

In embodiments of this three way matrix resolution method, the screening assay causes the release of gamma interferon by CD8+ cytotoxic T cells; or the soluble factor is a cytokine, e.g. gammy interferon. The method may further comprise assaying the supernatant of co-cultured cells, for the presence of a soluble factor, secreted by the cytotoxic T cells.

## BRIEF DESCRIPTION OF THE DRAWINGS

FIG. 1 is a photograph of an autoradiograph of 32P-labellled double stranded cDNA copied from the poly(A+) *T. parva,* schizont RNA, used as starting material for the production of a cDNA expression library of *T. parva,* schizont stage material. The autoradiograph of electrophoresis gel of the 4 fractions (A, B, C and D) of $^{32}$-P-labelled ds-cDNA indicates the cDNA sizes in kilobases.

FIG. 2 is a graph, indicating positive cDNA pools, for expression of cloned *T. parva* antigens as measured by the Elispot technique where schizont cDNA pools, were co-cultured with cytotoxic T lymphocyte cell (CTL) lines, and then CTL gamma interferon secretion levels were used to identify those clones that elicited antigen-specific stimulation. Screening of schizont cDNA pools (B series) was performed with BV115 (4229 TpM) CD8 polyclonal CTL. COS-7 cells co-transfected with BoLA class I HD6 cDNA and schizont cDNA pools were cultured with CTL and recognition assessed by IFN-γELISpot. Responses are presented as mean spot forming cell (SFC)/well. Of the 200 cDNA pools tested, pools B42 and B 162 were selected as putative positives and subjected to resolution.

FIG. 3 is a graph of Elispot data, indicating the mean spot forming cells/well when COS-7 cells, co-transfected with a bovine MHC Class I gene and *T. parva* schizont cDNAs consisting of a pool of 10cDNAs were co-cultured with cytotoxic T lymphocyte cell (CTL) lines, as measured by CTL gamma interferon secretion, in a matrix comparison to reveal nucleotides encoding CTL-stimulating antigens. In this screening of resolved schizont cDNA pool B162 with BV115 (4229 TpM) CD8 polyclonal CTL. 48 pools of 10 cDNA derived were constructed in a 3 way matrix and co-transfected with HD6 into COS-7 cells. Recognition of transfectants by CTL was assessed by IFN-γELISpot. IFN-γ production is presented as mean number of spot forming cell (SFC)/well. Of the 48 cDNA pools, 8 were positive, the 3 way matrix was then decoded to reveal 5 single cDNA.

FIG. 4 is a graph that indicates individual single *T. parva* schizont cDNA clones, expressing stimulatory antigen. This screening of single schizont cDNA with BV115. (4229 TpM) CD8 polyclonal CTL following resolution of schizont cDNA pool B162. Five single cDNA decoded from the 3 way matrix were co-transfected with HD6 into COS-7 cells. Recognition of transfectants by CTL was assessed by IFN-γELISpot. IFN-γ production is presented as mean number of spot forming cells (SFC)/well. Of the 5 single cDNA, 3 were positive, the 3 had identical sequences and were different from the 2 negative cDNA.

FIG. 5 is a graph of Elispot data, indicating the mean spot forming cells/well when COS-7 cells, co-transfected with a bovine MHC Class I gene and an individual single *T. parva* schizont cDNA clone, Tp1, were co-cultured with cytotoxic T lymphocyte cell (CTL) lines, as measured by CTL gamma interferon secretion. Confirmation of BoLA class I HD6 restriction of Tp1 recognition. Tp1 cDNA was co-transfected into COS-7 cells with BoLA class I HD6 cDNA, BoLA class I KN104 cDNA or alone. Recognition of transfectants by BV115 (4229 TpM) CD8+ polyclonal CTL was assessed by IFN-γELISpot. IFN-γ production is presented as mean number of spot forming cells (SFC)/well. Only when Tp1 was co-transfected with HD6 cDNA did the CTL respond.

FIG. 6 is a graph of Elispot data comparing the ability of COS-7 co-transfected cells to present target antigen to CTLs, to the ability of immortalized skin fibroblast cells (iSF) to present target antigen to CTLs. Comparison of ability COS-7 and iSF to present target antigen containing schizont cDNA pools to CTL. COS-7 were co-transfected with HD6 cDNA and schizont cDNA pools, whilst BV115 iSF were transfected with the schizont cDNA pools alone. Recognition of transfectants by BV115 (4229) CD8+ polyclonal CTL was assessed by IFN-γELISpot. IFN-γ production is presented as mean number of spot forming cells (SFC)/well. Of the 7 pools tested only one, pool B42, gave a response above that of the untransfected cells. This pool was known to contain the Tp1 cDNA. The weaker response to transfected iSF is likely due to an inferior transfection efficiency compared to COS-7.

FIG. 7A is a graph of Elispot data (gamma interferon production); FIG. 7B is a graph of bioassay data (MHC Class II expression), for Tp1 transfected COS-7 cells. Both assays show the need for co-transfection of COS-7 cells with HD6 (MHC Class I)cDNA for the CTLS to be stimulated by Tp1 production. Comparison of detection of CTL IFN-□ release by ELISpot (A) and Bioassay (B) following recognition of Tp1 transfected COS-7 cells.

Single cDNA isolated following resolution of positive cDNA pool B42 were transfected into COS-7 cells with or without HD6 cDNA. Recognition of transfectants by BV115 (4229) CD8+ polyclonal CTL was assessed by release of IFN-γusing both an ELISpot and bioassay. IFN-γ production as detected by ELISpot is presented as mean number of spot forming cells (SFC)/well and IFN-γ bioactivity presented as % MHC class II expression. Of the 9 cDNA tested, both assays showed HD6 dependent recognition of 3 (#4, 178 and 309). All cDNA were sequenced and the positive cDNA identified as Tp1.

FIG. 8 is a graph of data that indicates the % lysis of COS-7 cells, co-transfected with a MHC Class I gene and a cDNA for Tp1 by CTLs. Note that lysis was abrogated when an anti-BoLA class I monoclonal antibody ("MHC I-block") was used to mask MHC Class I expression. Lysis of Tp1 transfected COS-7 cells (co-transfection with BoLA class I HD6 cDNA) and immortalised autologous skin fibroblasts (SF) by the schizont specific BV115 (4229 TpM) CD8+ polyclonal CTL line. HD6 expressing 4229 TpM line was included as a positive control and PIM transfected COS-7 cells and iSF included as negative controls. MHC class I restriction was assessed by pre-incubating Tp1 transfected COS cells with an anti-BoLA class I mAb (MHC I block).

FIG. 9 indicates the deduced amino acid sequence of Exonuclease III-deleted, Tp1 cDNA plasmids (SEQ ID NOS 1, 6, 7 & 43-46, respectively, in order of appearance). Clones were co-transfected with HD6 cDNA and recognition by CTL determined by IFN-γ ELISpot. Only the Tp1 orf and clones Tp1 Dell and Del2 were recognised implying that the HD6 restricted Tp1 epitope lay between amino acid position 141 and 241.

FIG. 10 indicates the deduced amino acid sequence of a series of deleted Tp1 clones (SEQ ID NOS 47-52, respectively, in order of appearance). The small m in bold represents the methionine added by the artificial ATG start codon by PCR. Constructs were co-transfected with HD6 cDNA and recognition by CTL determined by IFN-γ ELISpot. Clones Tp1.1, 1.4 and 1.6 were recognised by CTL thus narrowing down the epitope containing region to 66 amino acids.

FIG. 11 is a graph of Elispot data indicating mapping of a Tp1 epitope using a synthetic peptide library. This figure shows the fine mapping of the HD6 restricted Tp1 CTL epitope using synthetic peptide library. Twenty eight 12mer peptides overlapping by two amino acids encompassing the 66 amino acids encoded by the Tp1.2 insert were synthesised and used at a final concentration of 1□g/ml to pulse BV115 iSF. Recognition of peptide pulsed iSF by two polyclonal CTL lines derived from BV115, one maintained on 4229 TpM (4229 poly) the other on autologous BV115 TpM (BV 115 poly), was assessed by IFN-γ ELISpot. Significant responses were observed against peptides #10 and 11 that corresponded to the amino acid sequence RCVGYPKVKEEMLE (SEQ ID NO: 8).

FIG. 12 is a graph of Elispot data indicating mapping of the minimal length of the HD6 restricted epitope of Tp1 by the identification of the HD6 restricted Tp1 CTL epitope using synthetic peptides. Twenty seven peptides comprising of all the possible 9, 10 and 11mers from the sequence FLVGYPKVKEEMLEMA (SEQ ID NO: 32) were synthesised and used at a final concentration of 100pg/ml to pulse P815 cells stably expressing HD6 (P815/HD6). Recognition of peptide pulsed P815/HD6 by two HD6 restricted BV115 CTL clones, #94 and #122, was assessed by IFN-γ ELISpot. Significant responses were only observed against peptide #24, suggesting that the HD6 was the 11mer VGYPKVKEEML (SEQ ID NO: 9).

FIG. 13 is a comparison of the deduced amino acid sequences of Tp1 from two different *T. parva* strains, Muguga (SEQ ID NO: 1) and Marikebuni SEQ ID NO: 53). There was 95.8% identity in 542 aa overlap. Significantly there were two amino acid differences at positions 226 and 227, the C terminal end of the 11 amino acid HD6 restricted epitope.

FIG.14 is a graph of Elispot data that indicates the response of CD8+ T cells, harvested from an immune bull to the HD6 restricted epitope of Tp1. Measurement of the Tp1 specific CD8+ T cell responses of an immune bull following challenge with T. parva sporozoites are indicated. CD8+ T cells were purified from peripheral blood at various time-points and incubated with the HD6 restricted Tp1 11mer synthetic (epitope) or a Tp1 peptide which had not previously been shown to contain a CTL epitope (control) and responses were measured by IFN-Gamma ELISpot assay.

FIG. 15A is a photograph of a Coomassie stained gel and an anti-His-tag immunoblot (Western blot) of Tp1 expression, using a bacterial expression vector; FIG. 15B is a photograph of a Coomassie stained gel and an anti-His-tag immunoblot of Tp4 expression, using a bacterial expression vector; and, FIG. 15C is a photograph of a Coomassie stained gel and an anti-His-tag immunoblot of Tp5 expression, using a bacterial expression vector. The expression of CTL target antigens, Tp1 (A), Tp4 (B) and Tp5 (C) proteins was investigated. Recombinant proteins were isolated by Ni-NTA agarose and run on 12% SDS-PAGE gels followed by staining using coomassie blue or detection by Western blotting.

FIG.16A is a panel of histograms indicating numbers of infected Bovine lymphocytes, stained with the reporter molecules: control (a); mouse polyclonal antibody to Tp1 (b); mouse monoclonal antibody to Tp1 (c); mouse polyclonal antibody to Tp4 (d); and mouse polyclonal antibody to Tp5, (e. Monoclonal and polyclonal antibodies raised against the Tp1, Tp4 and Tp5 proteins were used for staining of *T. parva* infected cells. FIG. 16B shows a quantitation of the values shown in the histograms in FIG 16A.

FIG. 17. indicates Tp1 multiple sequence alignments (SEQ ID NOS 54-77, respectively, in order of appearance). Amino acid sequence comparison of a portion (containing the HD6 CTL epitope) of Tp1 generated from *T. parva* isolates from different regions. Domains with variations are underlined.

FIG. 18 indicates the level of CTL responses to several Tp antigens, following CP(Canary Pox mediated)/MVA (modified Vaccinia Virus Ankara-vector mediated) immunization protocols. The data indicates the production of *T. parva* antigen-specific CD8 (CTLs) in animals following immunization protocols with CanaryPox viral vectors, expressing *T. parva* antigen.

FIG. 19 indicates the level of CTL responses to several Tp antigens, following DNA/MVA immunization protocols. The data indicates the production of *T. parva* antigen-specific CD8 (CTLs) in animals following immunization protocols with modified Vaccinia (Ankara) viral vectors, expressing *T. parva* antigen.

FIG. 20 indicates control data for CP/MVA and DNA/MVA immunization protocols, where cattle were given phosphate buffered saline injections rather than Tp antigens. The control experimental data for measuring the development of CTLs following immunization indicates a lack of *T parva*, antigen-specific CD8+ cells in PBS immunized animals.

## DETAILED DESCRIPTION OF THE INVENTION

Class I MHC-restricted $CD8^+$ cytotoxic T lymphocytes (CTLs) are responsible for protecting cattle against a lethal challenge with Theileria parva (*T. parva*), sporozoites (Morrison, Taracha, and McKeever, 1995). These CTLs are directed at schizont-infected cells, which they recognize and lyse. Schizont antigens that are recognized by these $CD8^+$ CTLs are the prime candidates for inclusion into an effective sub-unit vaccine for the prevention of East Coast Fever disease (ECF).

The Examples herein show the identification of a variety of antigens which are suitable for use in subunit vaccines.

The present invention provides polynucleotides, and novel methods for their identification, which encode proteins and peptides that are targets of antigen-specific cytotoxic CD8+ T lymphocytes which have been shown to be protective against ECF infection in adoptive cell transfer experiments. Experimental immunization of cattle with these antigens or recombinant viral vector cell systems producing these antigens have stimulated the production of antigen-specific T cell responses in the immunized animals.

The proteins identified by the novel method can also be used to as antigens to stimulate the production of antibodies. Such antibodies are useful for the detection of the presence of a stimulating antigen, in animals tissues and fluids. Presence of such antigen would indicate infection of the animal by organisms producing or bearing, the antigen. For example, in one embodiment of the presents description infection of animal by *Theileria parva* (*T. parva*) can be detected by antibodies produced as a result of using peptides encoded by polynucleotide sequences as antigens to stimulate the production of such antibodies

In a particularly preferred embodiment of the subject description, the antigens encoded by polynucleotide sequences, cloned from expressed genes of *T. parva*, are identified by the use of immortalized cloned bovine skin fibroblast cell lines to stimulate the activation of cytotoxic CD8+ T lymphocytes by antigen when stimulation is measured by the release of soluble factors, more specifically gamma interferon. The subject description also relates to compositions isolated by methods including one or more of these particular steps and the use of these compositions: to stimulate or induce cytotoxic T cells, as diagnostic reagents for the detection of disease, or an immune response, in kits or high throughput "chip" methods for the detection of or expression of, identical or homologous nucleic acids. In another preferred embodiment of the subject description, the antigens identified by the novel method are useful for immunization of animals for the production of CTLs that recognize *T. parva.*

The present description identifies a group of polynucletide sequences that encode *T. parva* antigens useful for a variety of applications.

The nucleic acids of the invention may comprise recombinant nucleic acid. By the term "recombinant nucleic acid" herein is meant nucleic acid, originally formed in vitro or in a cell in culture, in general, by the manipulation of nucleic acid by endonucleases and/or exonucleases and/or polymerases and/or ligases and/or recombinases, to produce a nucleic acid not normally found in nature. Thus an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes of the invention.

Furthermore, as a result of the degeneracy of the genetic code, a multitude of nucleotide sequences may be produced which are based upon the sequences provided herein and corresponding peptides, polypeptides, or proteins. Some of these nucleotide sequences will bear only minimal homology to the sequences disclosed herein; however the subject description specifically contemplates each and every possible variation of nucleotide sequence that could be made by selecting combinations based on possible codon choices. These combinations are made in accordance with the standard triplet genetic code as applied to the nucleotide sequence of naturally occurring peptide, polypeptide, or protein, and all such variations are to be considered as being specifically disclosed herein. Recombinant nucleotide variants are alternate polynucleotides which encode a particular protein. They may be synthesized, for example, by making use of the "redundancy" in the genetic code. Various codon substitutions, such as the silent changes which produce specific restriction sites or codon usage-specific mutations, may be introduced to optimize cloning into a plasmid or viral vector or expression in a particular prokaryotic or eukaryotic host system, respectively.

It is possible to produce the polynucleotides of the subject invention, or portions thereof, entirely by synthetic chemistry. After synthesis, the nucleic acid sequence can be used alone or joined with a preexisting sequence and inserted into one of the many available DNA vectors and their respective host cells using techniques well known in the art. Moreover, synthetic chemistry may be used to introduce specific mutations into the nucleotide sequence. Alternatively, a portion of sequence in which a mutation is desired can be synthesized and recombined with a portion of an existing genomic or recombinant sequence.

Peptides and polypeptides of the invention can also be produced, entirely or in part, by synthetic chemistry, using conventional procedures.

Nucleotide sequences encoding a peptide, polypeptide, or protein may be joined to a variety of other nucleotide sequences by means of well established recombinant DNA techniques (Sambrook J. et al. (1989) Molecular Cloning: A Laboratory Manual, Cold Spring Harbor Laboratory, Cold Spring Harbor, N.Y.; or Ausubel F. M. et al. (1989) Current Protocols in Molecular Biology, John Wiley & Sons, New York City). Useful sequences include an assortment of cloning vectors such as plasmids, cosmids, lambda phage derivatives, phagemids, and the like. Vectors of interest include vectors for replication, expression, probe generation, sequencing, and the like. In general, vectors of interest may contain an origin of replication functional in at least one organism, convenient restriction endonuclease sensitive sites, and selectable markers for one or more host cell systems.

Another aspect of the subject description is to provide for hybridization probes which are capable of hybridizing with naturally occurring antigen sequences or nucleotide sequences encoding the disclosed peptide, polypeptide, or protein. The stringency of the hybridization conditions will determine whether the probe identifies only the native nucleotide sequence or sequences of closely related molecules. If degenerate nucleotide sequences of the subject description are used for the detection of related sequences, they should preferably contain at least 50% of the nucleotides of the sequences presented herein.

"Probes" are nucleic acid sequences of variable length, preferably between at least about 10 and as many as about 6,000 nucleotides, depending on use. They are used in the detection of identical, similar, or complementary nucleic acid sequences. Longer length probes are usually obtained from a natural or recombinant source, are highly specific and much slower to hybridize than oligomers. They may be single- or double-stranded and designed to have specificity in PCR, hybridization membrane-based, or ELISA-Like technologies.

Hybridization probes of the subject description may be derived from the nucleotide sequences of the attached List Sequences and the Sequences provided as SEQ ID NO: 18-22 (see, e.g., Figures 9,10, and 17), or from surrounding or included genomic sequences comprising untranslated regions such as promoters, enhancers and introns. Such hybridization probes may be labeled with appropriate reporter molecules. Means for producing specific hybridization probes include oligolabelling, nick translation, end-labeling or PCR amplification using a labeled nucleotide. Alternatively, the cDNA sequence may be cloned into a vector for the production of mRNA probe. Such vectors are known in the art, are commercially available, and may be used to synthesize RNA probes in vitro by addition of an appropriate RNA polymerase such as T7, T3 or SP6 and labelled nucleotides. A number of companies (such as Pharmacia Biotech, Piscataway, N.J.; Promega, Madison, Wis.; US Biochemical Corp, Cleveland, Ohio; etc.) supply commercial kits and protocols for these procedures.

The nucleotide sequences (shown as SEQ ID NO: 18-22) can be used to generate probes for mapping the native genomic sequence. The sequence may be mapped to a particular chromosome or to a specific region of the chromosome using well known techniques. These include in situ hybridization to chromosomal spreads, flow-sorted chromosomal preparations, or artificial chromosome constructions such as yeast artificial chromosomes (YACs), bacterial artificial chromosomes (BACs), bacterial P1 constructions or single chromosome cDNA libraries.

In situ hybridization of chromosomal preparations and physical mapping techniques such as linkage analysis using established chromosomal markers are invaluable in extending genetic maps in organisms, including intracellular parasites. The nucleotide sequences of the subject invention may also be used to detect differences in the chromosomal location of nucleotide sequences due to translocation, inversion, or recombination.

Other aspects of the description include use of the disclosed sequences or recombinant nucleic acids derived therefrom to produce purified peptides. The nucleotide sequences as disclosed herein may be used to produce an amino acid sequence using well known methods of recombinant DNA technology. Goeddel (Gene Expression Technology, Methods and Enzymology [1990] Vol 185, Academic Press, San Diego, Calif.) is one among many publications which teach expression of an isolated, purified nucleotide sequence. The amino acid or peptide may be expressed in a variety of host cells, either prokaryotic or eukaryotic. Some expression vectors are viral vectors such as vaccinia-based vectors and adeno-associated viral vectors, among others (Dunachie and Hill, 2003). Host cells may be from the same species from which the nucleotide sequence was derived or from a different species.

Still further aspects of the invention use these purified peptides to produce antibodies or other molecules able to bind to the peptides. These antibodies or binding agents can then be used for the screening of cells in order to localize the cellular distribution of the peptides or proteins. The antibodies are also useful for the affinity purification of recombinantly produced peptides or proteins. Such antibodies are also useful as diagnostic reagents for the detection of protozoan diseases or in antibody-mediated tests and assays.

Disclosed *T. parva* antigens can also be used as a diagnostic aid or in methods that measure the presence of T. parva cytotoxic lymphocytes in immunized or nfected animals by co-culturing mononuclear cells harvested from an animal suspected to have an infection due to *T. parva,* with a *T. parva* antigen-specifc cytotoxic T cell under standard culture conditions for mammalian cell cultures. Mononuclear cells would have been incubated in an culture medium containing an indicator that is released upon cell death, such as $^{51}Cr$. This is a method that is well-known in the art.

The disclosed nucleotide sequences can be used individually, or in panels, in tests or assays to detect levels of peptide, polypeptide, or protein expression. The form of such qualitative or quantitative methods may include northern analysis, dot blot or other membrane based technologies, dip stick, pin or chip technologies, PCR, ELISAs or other multiple sample format technologies.

An "oligonucleotide" or "oligomer" is a stretch of nucleotide residues which has a sufficient number of bases to be used in a polymerase chain reaction (PCR). These short sequences are based on (or designed from) genomic or cDNA sequences and are used to amplify, confirm, or reveal the presence of an identical, similar or complementary DNA or RNA in a particular cell or tissue. Oligonucleotides or oligomers comprise portions of a DNA sequence having at least about 10 nucleotides and as many as about 50 nucleotides, preferably about 15 to 30 nucleotides. They can be chemically synthesized and may be used as probes.

A "complementary" DNA or RNA is a sequence that is 100% identical to the strand to which it is complementary.

The polynucleotides of the subject invention can themselves be used as probes. Additional polynucleotide sequences can be added to the ends of (or internally in) the exemplified polynucleotide sequences so that polynucleotides that are longer than the exemplified polynucleotides can also be used as probes. Thus, isolated polynucleotides comprising one or more of the exemplified sequences are within the scope of the subject invention. Polynucleotides that have less nucleotides than the exemplified polynucleotides can also be used and are contemplated within the scope of the present description. For example, for some purposes, it might be useful to use a conserved sequence from an exemplified polynucleotide wherein the conserved sequence comprises a portion of an exemplified sequence. Thus, polynucleotides of the subject invention can be used to find additional, homologous (wholly or partially) genes.

Probes of the subject description may be composed of DNA, RNA, or PNA (peptide nucleic acid). The probe will normally have at least about 10 bases, more usually at least about 17 bases, and may have up to about 100 bases or more. Longer probes can readily be utilitzed, and such probes can be, for example, several kilobases in length. The probe sequence is designed to be at least substantially complementary to a portion of a gene encoding a protein of interest. The probe need not have perfect complementarity to the sequence to which it hybridizes. The probes may be labeled utilizing techniques that are well known to those skilled in this art.

One approach for the use of the subject description as probes entails first identifying DNA segments that are homologous with the disclosed nucleotide sequences using, for example, Southern blot analysis of a gene bank. Thus, it is possible, without the aid of biological analysis, to know in advance the probable activity of many new polynucleotides, and of the individual gene products expressed by a given polynucleotide. Such an analysis provides a rapid method for identifying commercially valuable compositions.

One hybridization procedure useful according to the subject description typically includes the initial steps of isolating the DNA sample of interest and purifying it chemically. Either lysed cells or total fractionated nucleic acid isolated from cells can be used. Cells can be treated using known techniques to liberate their DNA (and/or RNA). The DNA sample can be cut into pieces with an appropriate restriction enzyme. The pieces can be interest can be through electrophoresis in a gel, usually agarose or acrylamide. The pieces of interest can be transferred to an immobilizing membrane.

The particular hybridization technique is not essential to the subject description As improvements are made in hybridization techniques, they can be readily applied.

The probe and sample can then be combined in a hybridization buffer solution and held at an appropriate temperature until annealing occurs. Thereafter, the membrane is washed free of extraneous materials, leaving the sample and bound probe molecules typically detected and quantified by autoradiography and/or liquid scintillation counting. As is well known in the art, if the probe molecule and nucleic acid sample hybridize by forming a strong non-covalent bond between the two molecules, it can be reasonably assumed that the probe and sample are essentially identical or very similar. The probe’s detectable label provides a means for determining in a known manner whether hybridization has occurred.

In the use of the nucleotide segments as probes, the particular probe is labeled with any suitable label known to those skilled in the art, including radioactive and non-radioactive labels. Typical radioactive labels include $^{32}P$, $^{35}S$, or the like. Non-radioactive labels include, for example, ligands such as biotin or thyroxine, as well as enzymes such as hydrolases or peroxidases, or the various chemiluminescers such as luciferin, or fluorescent compounds like fluorescein and its derivatives. In addition, the probes can be made inherently fluorescent as described in International Application No. WO 93/16094.

Various degrees of stringency of hybridization can be employed. The more stringent the conditions, the greater the complementarity that is required for duplex formation. Stringency can be controlled by temperature, probe concentration, probe length, ionic strength, time, and the like. Preferably, hybridization is conducted under moderate to high stringency conditions by techniques well known in the art, as described, for example, in Keller, G. H., M. M. Manak (1987) DNA Probes, Stockton Press, New York, N.Y., pp. 169-170.

As used herein "moderate to high stringency" conditions for hybridization refers to conditions that achieve the same, or about the same, degree of specificity of hybridization as the conditions described herein. Examples of moderate to high stringency conditions are provided herein. Specifically, hybridization of immobilized DNA on Southern blots with $^{35}$P-labeled gene-specific probes is performed using standard methods (Maniatis et al.). In general, hybridization and subsequent washes are carried out under moderate to high stringency conditions that allow for detection of target sequences with homology to sequences exemplified herein. For double-stranded DNA gene probes, hybridization can be carried out overnight at 20-25° C. below the melting temperature (Tm) of the DNA hybrid in 6 x SSPE, 5 x Denhardt's solution, 0.1% SDS, 0.1 mg/ml denatured DNA. The melting temperature is described by the following formula from Beltz et al. (1983):

Tm=81.5° C.+16.6 Log[Na+]+0.41(% G+C)-0.61(% formamide)-600/length of duplex in base pairs.

Washes can typically be carried out as follows:

(1) Twice at room temperature for 15 minutes in 1.times. SSPE, 0.1% SDS (low stringency wash).
(2) Once at Tm-20° C. for 15 minutes in 0.2.times. SSPE, 0.1% SDS (moderate stringency wash).

For oligonucleotide probes, hybridization was carried out overnight at 10-20°C below the melting temperature (Tm) of the hybrid in 6xSSPE, 5xDenhardt's solution, 0.1% SDS, 0.1 mg/ml denatured DNA. Tm for oligonucleotide probes was determined by the following formula from Suggs et aL (1981):

Tm (°C.)=2(number T/A base pairs)+4(number G/C base pairs)

In general, salt and/or temperature can be altered to change stringency. With a labeled DNA fragment of greater than about 70 or so bases in length, the following conditions can be used:

1 Low: 1 or 2X SSPE, room temperature Low: 1 or 2X SSPE, 42 ° C. Moderate: 0.2X or 1X SSPE, 65° C. High: 0.1X SSPE, 65°C.

Duplex formation and stability depend on substantial complementarity between the two strands of a hybrid, and, as noted above, a certain degree of mismatch can be tolerated. Therefore, polynucleotide sequences of the subject description include mutations (both single and multiple), deletions, and insertions in the described sequences, and combinations thereof, wherein said mutations, insertions, and deletions permit formation of stable hybrids with a target polynucleotide of interest. Mutations, insertions, and deletions can be produced in a given polynucleotide sequence using standard methods known in the art. Other methods may become known in the future.

The mutational, insertional, and deletional variants of the polypeptide sequences of the description can be used in the same manner as the exemplified polynucleotide sequences so long as the variants have substantial secjuence similarity with the original sequence. As used herein, substantial sequence similarity refers to the extent of nucleotide similarity that is sufficient to enable the variant polynucleotide to function in the same capacity as the original sequence. Preferably, this similarity is greater than 50%; more preferably, this similarity is greater than 75%; and most preferably, this similarity is greater than 90%. The degree of similarity needed for the variant to function in its intended capacity will depend upon the intended use of the sequence. It is well within the skill of a person trained in this art to make mutational, insertional, and deletional mutations that are designed to improve the function of the sequence or otherwise provide a methodological advantage.

PCR technology. Polymerase Chain Reaction (PCR) is a repetitive, enzymatic, primed synthesis of a nucleic acid sequence. This procedure is well known and commonly used by those skilled in this art (see U.S. Pat. Nos. 4,683,195, 4,683,202, and 4,800,159; Saiki et al., 1985). PCR is based on the enzymatic amplification of a DNA fragment of interest that is flanked by two oligonucleotide primers that hybridize to opposite strands of the target sequence. The primers are oriented with the 3' ends pointing towards each other. Repeated cycles of heat denaturation of the template, annealing of the primers to their complementary sequences, and extension of the annealed primers with a DNA polymerase result in the amplification of the segment defined by the 5' ends of the PCR primers. Since the extension product of each primer can serve as a template for the other primer, each cycle essentially doubles the amount of DNA fragment produced in the previous cycle. This results in the exponential accumulation of the specific target fragment, up to several million-fold in a few hours. By using a thermostable DNA polymerase such as Taq polymerase, which is isolated from the thermophilic bacterium Thermus aquaticus, the amplification process can be completely automated. Other enzymes that can be used are known to those skilled in the art.

The polynucleotide sequences of the subject invention (and portions thereof such as conserved regions and portions that serve to distinguish these sequences from previously-known sequences) can be used as, and/or used in the design of, primers for PCR amplification. In performing PCR amplification, a certain degree of mismatch can be tolerated between primer and template. Therefore, mutations, deletions, and insertions (especially additions of nucleotides to the 5' end) of the exemplified polynucleotides can be used in this manner. Mutations, insertions and deletions can be produced in a given primer by methods known to an ordinarily skilled artisan.

Full length genes may be cloned utilizing partial nucleotide sequence and various methods known in the art. Gobinda et al. (1993; PCR Methods Applic 2:318-22) disclose "restriction-site PCR" as a direct method which uses universal primers to retrieve unknown sequence adjacent to a known locus. First, genomic DNA is amplified in the presence of primer to linker and a primer specific to the known region. The amplified sequences are subjected to a second round of PCR with the same linker primer and another specific primer internal to the first one. Products of each round of PCR are transcribed with an appropriate RNA polymerase and sequenced using reverse transcriptase.

Inverse PCR can be used to acquire unknown sequences starting with primers based on a known region (Triglia T. et al. (1988) Nucleic Acids Res 16:8186). The method uses several restriction enzymes to generate a suitable fragment in the known region of a gene. The fragment is then circularized by intramolecular ligation and used as a PCR template. Divergent primers are designed from the known region. The multiple rounds of restriction enzyme digestions and ligations that are necessary prior to PCR make the procedure slow and expensive (Gobinda et al. [1993] supra).

Capture PCR (Lagerstrom M. et al. (1991) PCR Methods Applic 1:111-19) is a method for PCR amplification of DNA fragments adjacent to a known sequence in eucaryotic and YAC DNA. As noted by Gobinda et al. (1993,supra), capture PCR also requires multiple restriction enzyme digestions and ligations to place an engineered double-stranded sequence into an unknown portion of the DNA molecule before PCR. Although the restriction and ligation reactions are carried out simultaneously, the requirements for extension, immobilization and two rounds of PCR and purification prior to sequencing render the method cumbersome and time consuming.

Parker J. D. et al. (Nucleic Acids Res [1991 119:3055-60), teach walking PCR, a method for targeted gene walking which permits retrieval of unknown sequences. PromoterFinder.TM. is a kit available from Clontech Laboratories, Inc. (Palo Alto, Calif.) which uses PCR and primers derived from p53 to walk in genomic DNA. Nested primers and special PromoterFinder.TM. libraries are used to detect upstream sequences such as promoters and regulatory elements. This process avoids the need to screen libraries and is useful in finding intron/exon junctions.

One PCR method replaces methods which use labeled probes to screen plasmid libraries and allow one researcher to process only about 3-5 genes in 14-40 days. In the first step, which can be performed in about two days, any two of a plurality of primers are designed and synthesized based on a known partial sequence. In step 2, which takes about six to eight hours, the sequence is extended by PCR amplification of a selected library. Steps 3 and 4, which take about one day, are purification of the amplified cDNA and its ligation into an appropriate vector. Step 5, which takes about one day, involves transforming and growing up host bacteria. In step 6, which takes approximately five hours, PCR is used to screen bacterial clones for extended sequence. The final steps, which take about one day, involve the preparation and sequencing of selected clones.

If the full length cDNA has not been obtained, the entire procedure is repeated using either the original library or some other preferred library. The preferred library may be one that has been size-selected to include only larger cDNAs or may consist of single or combined commercially available libraries, e.g., from Clontech Laboratories, Inc. (Palo Alto, Calif.). The cDNA library may have been prepared with oligo (dT) or random priming. Random primed libraries are preferred in that they will contain more sequences which contain 5' ends of genes. A randomly primed library may be particularly useful if an oligo (dT) library does not yield a complete gene. It must be noted that the larger and more complex the protein, the less likely it is that the complete gene will be found in a single plasmid.

CLONTECH PCR-Select.TM. cDNA Subtraction (Clontech Laboratories, Inc., Palo Alto, Calif.) is yet another means by which differentially expressed genes may be isolated. The procedure allows for the isolation of transcripts present in one mRNA population which is absent, or found in reduced numbers, in a second population of mRNA. Rare transcripts may be enriched 1000-fold.

Another method for analyzing either the size or the nucleotide sequence of PCR products is capillary electrophoresis. Systems for rapid sequencing are available from Perkin Elmer (Foster City Calif.), Beckman Instruments (Fullerton, Calif.), and other companies. Capillary sequencing employs flowable polymers for electrophoretic separation, four different fluorescent dyes (one for each nucleotide) which are laser activated, and detection of the emitted wavelengths by a charge coupled devise camera. Output/light intensity is converted to electrical signal using appropriate software (eg. Genotyper.TM. and Sequence Navigators.TM. from Perkin Elmer) and the entire process from loading of samples to computer analysis and electronic data display is computer controlled. Capillary electrophoresis provides greater resolution and is many times faster than standard gel based procedures. It is particularly suited to the sequencing of small pieces of DNA which might be present in limited amounts in a particular sample. The reproducible sequencing of up to 350 bp of M13 phage DNA in 30 min has been reported (Ruiz-Martinez M. C. et al. [1993] Anal Chem 65:2851-8).

Polynucleotides of the subject invention can be defined according to several parameters. One characteristic is the biological activity of the protein products as identified herein. The proteins and genes of the subject description can be further defined by their amino acid and nucleotide sequences. The sequences of the molecules can be defined in terms of homology to certain exemplified sequences as well as in terms of the ability to hybridize with, or be amplified by, certain exemplified probes and primers. Additional primers and probes can readily be constructed by those skilled in the art such that alternate polynucleotide sequences encoding the same amino acid sequences can be used to identify and/or characterize additional genes. The proteins of the subject invention can also be identified based on their immunoreactivity with certain antibodies.

The polynucleotides and proteins of the subject description include portions, fragments, variants, and mutants of the full-length sequences as well as fusions and chimerics, so long as the encoded protein retains the characteristic biological activity of the proteins identified herein. As used herein, the terms "variants" or "variations" of genes refer to nucleotide sequences that encode the same proteins or which encode equivalent proteins having equivalent biological activity. As used herein, the term "equivalent proteins" refers to proteins having the same or essentially the same biological activity as the exemplified proteins.

Variations of genes may be readily constructed using standard techniques such as site-directed mutagenesis and other methods of making point mutations and by DNA shuffling, for example. In addition, gene and protein fragments can be made using commercially available exonucleases, endonucleases, and proteases according to standard procedures. For example, enzymes such as Ba131 can be used to systematically cut off nucleotides from the ends of genes. Also, genes that encode fragments may be obtained using a variety of restriction enzymes. Proteases may be used to directly obtain active fragments of these proteins. Of course, molecular techniques for cloning polynucleotides and producing gene constructs of interest are also well known in the art. In vitro evaluation techniques, such as MAXYGEN's "Molecular Breeding" can also be applied to practice the subject invention.

A "selectable marker" is a gene whose expression allows one to identify cells that have been transformed or transfected with a vector containing the marker gene.

"Reporter" molecules are chemical moieties used for labeling a nucleic or amino acid sequence. They include, but are not limited to, radionuclides, enzymes, fluorescent, chemi-luminescent, or chromogenic agents. Reporter molecules associate with, establish the presence of, and may allow quantification of a particular nucleic or amino acid sequence.

A "portion" or "fragment" of a polynucleotide or nucleic acid comprises all or any part of the nucleotide sequence having fewer nucleotides than about 6 kb, preferably fewer than about 1 kb which can be used as a probe. Such probes may be labeled with reporter molecules using nick translation, Klenow fill-in reaction, PCR or other methods well known in the art. After pretesting to optimize reaction conditions and to eliminate false positives, nucleic acid probes may be used in Southern, northern or in situ hybridizations to determine whether target DNA or RNA is present in a biological sample, cell type, tissue, organ or organism.

A "polypeptide" comprises a protein, oligopeptide or peptide fragments thereof.

A "mutant, variant, or modified polypeptide" means any polypeptide encoded by a nucleotide sequence that has been mutated through insertions, deletions, substitutions, or the like.

"Chimeric" molecules are polynucleotides or polypeptides which are created by combining one or more nucleotide or peptide sequences (or their parts). In the case of nucleotide sequences, such combined sequences may be introduced into an appropriate vector and expressed to give rise to a chimeric polypeptide which may be expected to be different from the native molecule in one or more of the following characteristics: cellular location, distribution, ligand-binding affinities, interchain affinities, degradation/turnover rate, signaling, etc.

"Active" is that state which is capable of being useful or of carrying out some role. It specifically refers to those forms, fragments, or domains of an amino acid sequence which display the biologic and/or immunogenic activity characteristic of the naturally occurring peptide, polypeptide, or protein. For example, the present description relates to active fragments of polypeptides (*e.g.*, represented by SEQ ID NO: 1-5). Each of these active fragments retains at least one epitope of the larger polypeptide.

"Naturally occurring" refers to a polypeptide produced by cells which have not been genetically engineered or which have been genetically engineered to produce the same sequence as that naturally produced.

"derivative" refers to those polypeptides which have been chemically modified by such techniques as ubiquitination, labeling, pegylation (derivatization with polyethylene glycol), and chemical insertion or substitution of amino acids such as ornithine which do not normally occur in proteins.

"Recombinant polypeptide variant" refers to any polypeptide which differs from naturally occurring peptide, polypeptide, or protein by amino acid insertions, deletions and/or substitutions.

Amino acid "substitutions" are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid "insertions" or "deletions" are changes to or within an amino acid sequence. They typically fall in the range of about 1 to 5 amino acids. The variation allowed in a particular amino acid sequence may be experimentally determined by producing the peptide synthetically or by systematically making insertions, deletions, or substitutions of nucleotides in the sequence using recombinant DNA techniques.

An "oligopeptide" is a short stretch of amino acid residues and may be expressed from an oligonucleotide. Such sequences comprise a stretch of amino acid residues of at least about 5 amino acids and often about 17 or more amino acids, typically at least about 9 to 13 amino acids, and of sufficient length to display biologic and/or immunogenic activity.

A "standard" is a quantitative or qualitative measurement for comparison. Preferably, it is based on a statistically appropriate number of samples and is created to use as a basis of comparison when performing diagnostic assays, running clinical trials, or following patient treatment profiles. The samples of a particular standard may be normal or similarly abnormal.

An antibody or "specific binding parts" means any fragment of an antibody molecule that will bind antigen or other ligands such as lectins or other molecules. "Specific binding parts" is meant to include, but not be limited to antibody fragments such as Fab fragments, Fab'(2) fragments, Fc region fragments, Complimentarity determing regions (CDRs), Fv fragments, single chain Fv (scFv) fragments, and antigen binding site fragments.

An "antigen" is a macromolecule that is recognized by antibodies or immune cells and can trigger an immune response. Usually, an antigen is a protein or a polysaccharide, but it can be any type of molecule, even small molecules if coupled to a large carrier.

An "antigen specific" cytotoxic T cell is a T lymphocyte that can recognize and kill another cell that is expressing an antigen, usually in conjunction with a type or class of molecules referred to by those familiar with the art, as Major Histocompatiblity Complex (MHC) Class I molecules. Such Cytotoxic T cells are also referred to as "$CD8^+$" (CD8 positive) or CTLs, by those familiar with the art.

An epitope or antigenic determinant is a region or section of an antigen that is approximately the minimal length of antigenic sequence that will elicit an immune response, as measured by the development of an antibody response, the development of T antigen-specific T cells, or other measureable immune cell response. For example, an epitope could be a site on an antigen recognized by antibody.

A T cell "epitope" is an antigenic determinant recognized and bound by the T-cell receptor. Epitopes recognized by the T-cell receptor are often located in the inner, unexposed side of the antigen, and become accessible to the T-cell receptors after proteolytic processing of the antigen.

An "effective" immunization protocol is one in which an animal is protected against infection, at least to a measurable degree, when exposed to the specific infectious agent for which it was immunized.

An "ELISpot Assay" is a shorted name for "Enzyme-linked ImmunoSpot Assay", which refers to an antibody based method to detect secretion of soluble factors released by cells. Originally developed as a method to detect antibody-secreting B-cells, later the method was adapted to determine T-cell reaction to a specific antigen, often represented as number of activated cells per million.

"Effective conditions" for culturing cells or cell lines means those cuture conditions that allow the cells to respond in a manner than mimics or is the same as the response would be if the cells were responding in vivo.

"Harvesting" cells or other materials means to separate and collect those cells from a mixture of from one source for use in another protocol.

A Bovine MHC class II bioassay, means an assay that measures the presence of a T cell-derived soluble factor, such as interferon (IFN-$\gamma$) release from T cells responding to specific stimulation through the ability of IFN-$\gamma$ to induce and up-regulate expression of class II molecules on bovine endothelial cells. Bovine endothelial cells do not constutively express class II molecules unless triggered by external signals such as IFN-$\gamma$.

Since the list of technical and scientific terms cannot be all encompassing, any undefined terms shall be construed to have the same meaning as is commonly understood by one of skill in the art to which this invention belongs. Furthermore, the singular forms "a", "an" and "the" include plural referents unless the context clearly dictates otherwise.

The description is not to be limited only to the particular sequences, variants, formulations or methods described. The sequences, variants, formulations and methodologies may vary, and the terminology used herein is for the purpose of describing particular embodiments. The terminology and definitions are not intended to be limiting.

Because of the redundancy of the genetic code, a variety of different DNA sequences can encode the amino acid sequences encoded by the polynucleotide sequences disclosed herein. It is well within the skill of a person trained in the art to create these alternative DNA sequences encoding proteins having the same, or essentially the same, amino acid sequence. These variant DNA sequences are within the scope of the subject description. As used herein, reference to "essentially the same" sequence refers to sequences that have amino acid substitutions, deletions, additions, or insertions that do not materially affect biological activity. Fragments retaining the characteristic biological activity are also included in this definition.

The subject description comprises variant or equivalent proteins (and nucleotide sequences coding for equivalent proteins) having the same or similar biological activity of proteins encoded by the exemplified polynucleotides. Equivalent proteins will have amino acid similarity with an exemplified protein (or peptide). The amino acid identity will typically be greater than 60%. Preferably, the amino acid identity will be greater than 75%. More preferably, the amino acid identity will be greater than 80%, and even more preferably greater than 90%. Most preferably, amino acid identity will be greater than 95%. (Likewise, the polynucleotides that encode the subject polypeptides will also have corresponding identities in these preferred ranges.) These identities are as determined using standard alignment techniques for determining amino acid identity. The amino acid identity/similarity/homology will be highest in critical regions of the protein including those regions that account for biological activity or that are involved in the determination of three-dimensional configuration that is ultimately responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject description so long as the substitution does not materially alter the biological activity of the compound. Below is a list of examples of amino acids belonging to various classes:

Nonpolar - Ala, Val, Leu, Ile, Pro, Met, Phe, Trp, uncharged polar Gly, Ser, Thr, Cys, Tyr, Asn, Gln
Acidic - Asp, Glu Basic Lys, His

In some instances, non-conservative substitutions can also be made.

As used herein, reference to "isolated" polynucleotides and/or "purified" proteins refers to these molecules when they are not associated with the other molecules with which they would be found in nature. Thus, reference to "isolated" and/or "purified" signifies the involvement of the "hand of man" as described herein. Reference to "heterologous" proteins, genes, and gene constructs, also signifies the involvement of the "hand of man."

The invention also provides vectors containing the nucleic acid molecules described herein. The term "vector" refers to a vehicle, preferably a nucleic acid molecule, which can transport the nucleic acid molecules. When the vector is a nucleic acid molecule, the nucleic acid molecules are covalently linked to the vector nucleic acid. With this aspect of the invention, the vector includes a plasmid, single or double stranded phage, a single or double stranded RNA or DNA viral vector, or artificial chromosome, such as a BAC, PAC, YAC, OR MAC.

A vector can be maintained in the host cell as an extrachromosomal element where it replicates and produces additional copies of the nucleic acid molecules. Alternatively, the vector may integrate into the host cell genome and produce additional copies of the nucleic acid molecules when the host cell replicates.

The invention provides vectors for the maintenance (cloning vectors) or vectors for expression (expression vectors) of the nucleic acid molecules. The vectors can function in prokaryotic or eukaryotic cells or in both (shuttle vectors).

Expression vectors contain cis-acting regulatory regions that are operably linked in the vector to the nucleic acid molecules such that transcription of the nucleic acid molecules is allowed in a host cell. The nucleic acid molecules can be introduced into the host cell with a separate nucleic acid molecule capable of affecting transcription. Thus, the second nucleic acid molecule may provide a trans-acting factor interacting with the cis-regulatory control region to allow transcription of the nucleic acid molecules from the vector. Alternatively, a trans-acting factor may be supplied by the host cell. Finally, a trans-acting factor can be produced from the vector itself. It is understood, however, that in some embodiments, transcription and/or translation of the nucleic acid molecules can occur in a cell-free system.

The regulatory sequence to which the nucleic acid molecules described herein can be operably linked include promoters for directing mRNA transcription. These include, but are not limited to, the left promoter from bacteriophage .lambda., the lac, TRP, and TAC promoters from E. Coli, the early and late promoters from SV40, the CMV immediate early promoter, the adenovirus early and late promoters, and retrovirus long-terminal repeats.

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination and, in the transcribed region a ribosome binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook et al., Molecular Cloning: A Laboratory Manual. 2nd. ed., Cold Spring Harbor Laboratory Press, Cold Spring Harbor, N.Y., (1989).

A variety of expression vectors can be used to express a nucleic acid molecule. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses including the modified vaccinia virus Ankara strain (MVA), adenoviruses, poxviruses including fowlpox virus (FP9), pseudorabies viruses, and retroviruses. Vectors may also be derived from combinations of these sources such as those derived from plasmid and bacteriophage genetic elements, e.g. cosmids and phagemids. Appropriate cloning and expression vectors for prokaryotic and eukaryotic hosts are described in Sambrook et al., Molecular Cloning: A Laboratory Manual. 2nd. ed., Cold Spring Harbor Laboratory Press, Cold Spring Harbor, N.Y., (1989).

The regulatory sequence may provide constitutive expression in one or more host cells (i.e. tissue specific) or may provide for inducible expression in one or more cell types such as by temperature, nutrient additive, or exogenous factor such as a hormone or other ligand. A variety of vectors providing for constitutive and inducible expression in prokaryotic and eukaryotic hosts are well known to those of ordinary skill in the art.

The nucleic acid molecules can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are well known to those of ordinary skill in the art.

The vector containing the appropriate nucleic acid molecule can be introduced into an appropriate host cell for propagation or expression using well-known techniques. Bacterial cells include, but are not limited to, E. coli, Streptomyces, and Salmonella typhimurium. Eukaryotic cells include, but are not limited to, yeast, insect cells such as Drosophila, animal cells such as COS and CHO cells, and plant cells.

As described herein, it may be desirable to express the peptide as a fusion protein. Accordingly, the description provides fusion vectors that allow for the production of the peptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired peptide can ultimately be separated from the fusion moiety. Proteolytic enzymes include, but are not limited to, factor Xa, thrombin, and enteroenzyme. Typical fusion expression vectors include pGEX (Smith et al., Gene 67:31-40 (1988)), pMAL (New England Biolabs, Beverly, Mass.) and pRIT5 (Pharmacia, Piscataway, N.J.) which fuse glutathione S-transferase (GST), maltose E binding protein, or protein A, respectively, to the target recombinant protein. Examples of suitable inducible non-fusion E. coli expression vectors include pTrc (Amann et al, Gene 69:301-315 (1988)) and pET 11d (Studier et al, Gene Expression Technology: Methods in Enzymology 185:60-89 (1990)).

Recombinant protein expression can be maximized in host bacteria by providing a genetic background wherein the host cell has an impaired capacity to proteolytically cleave the recombinant protein. (Gottesman, S., Gene Expression Technology: Methods in Enzymology 185, Academic Press, San Diego, Calif. (1990) 119-128). Alternatively, the sequence of the nucleic acid molecule of interest can be altered to provide preferential codon usage for a specific host cell, for example E. coli. (Wada et al., Nucleic Acids Res. 20:2111-2118 (1992)).

The nucleic acid molecules can also be expressed by expression vectors that are operative in yeast. Examples of vectors for expression in yeast e.g., S. cerevisiae include pYepSec1 (Baldari, et al, EMBO J. 6:229-234 (1987)), pMFa (Kujan et al, Cell 30:933-943(1982)), pJRY88 (Schultz et al, Gene 54:113-123 (1987)), and pYES2 (Invitrogen Corporation, San Diego, Calf.).

The nucleic acid molecules can also be expressed in insect cells using, for example, baculovirus expression vectors. Baculovirus vectors available for expression of proteins in cultured insect cells (e.g., Sf9 cells) include the pAc series (Smith et al, Mol. Cell Biol. 3:2156-2165 (1983)) and the pVL series (Lucklow et al, Virology 170:31-39 (1989)).

In certain embodiments of the description the nucleic acid molecules described herein are expressed in mammalian cells using mammalian expression vectors. Examples of mammalian expression vectors include pCDM8 (Seed, B. Nature 329:840(1987)) and pMT2PC (Kaufinan et al., EMBO J. 6:187-195 (1987)).

The expression vectors listed herein are provided by way of example only of the well-known vectors available to those of ordinary skill in the art that would be useful to express the nucleic acid molecules. The person of ordinary skill in the art would be aware of other vectors suitable for maintenance propagation or expression of the nucleic acid molecules described herein. These are found for example in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, N.Y., 1989.

The description also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the nucleic acid molecule sequences described herein, including both coding and non-coding regions. Expression of this antisense RNA is subject to each of the parameters described above in relation to expression of the sense RNA (regulatory sequences, constitutive or inducible expression, tissue-specific expression).

The invention also relates to recombinant host cells containing the vectors described herein. Host cells therefore include prokaryotic cells, lower eukaryotic cells such as yeast, other eukaryotic cells such as insect cells, and higher eukaryotic cells such as mammalian cells.

The recombinant host cells are prepared by introducing the vector constructs described herein into the cells by techniques readily available to the person of ordinary skill in the art. These include, but are not limited to, calcium phosphate transfection, DEAE-dextran-mediated transfection, cationic lipid-mediated transfection, electroporation, transduction, infection, lipofection, and other techniques such as those found in Sambrook, et al. (Molecular Cloning: A Laboratory Manual. 2nd, ed., Cold Spring Harbor Laboratory, Cold Spring Harbor Laboratory Press, Cold Spring Harbor, N.Y., 1989).

Host cells can contain more than one vector. Thus, different nucleotide sequences can be introduced on different vectors of the same cell. Similarly, the nucleic acid molecules can be introduced either alone or with other nucleic acid molecules that are not related to the nucleic acid molecules such as those providing trans-acting factors for expression vectors. When more than one vector is introduced into a cell, the vectors can be introduced independently, co-introduced or joined to the nucleic acid molecule vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the nucleic acid molecules described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the mature proteins can be produced in bacteria, yeast, mammalian cells, and other cells under the control of the appropriate regulatory sequences, cell- free transcription and translation systems can also be used to produce these proteins using RNA derived from the DNA constructs described herein.

Where secretion of the peptide is desired, which is difficult to achieve with multi-transmembrane domain containing proteins such as MHC Class I-binding peptides, appropriate secretion signals are incorporated into the vector. The signal sequence can be endogenous to the peptides or heterologous to these peptides.

The expressed protein can be isolated from the host cell by standard disruption procedures, including freeze thaw, sonication, mechanical disruption, use of lysing agents and the like. The peptide can then be recovered and purified by well-known purification methods including ammonium sulfate precipitation, acid extraction, anion or cationic exchange chromatography, phosphocellulose chromatography, hydrophobic-interaction chromatography, affinity chromatography, hydroxylapatite chromatography, lectin chromatography, or high performance liquid chromatography.

It is also understood that depending upon the host cell in recombinant production of the peptides described herein, the peptides can have various glycosylation patterns, depending upon the cell, or maybe non-glycosylated as when produced in bacteria. In addition, the peptides may include an initial modified methionine in some cases as a result of a host-mediated process.

There are many methods for introducing a heterologous gene or polynucleotide into a host cell or cells under conditions that allow for stable maintenance and expression of the gene or polynucleotide. These methods are well known to those skilled in the art. Synthetic genes, such as, for example, those genes modified to enhance expression in a heterologous host (such as by preferred codon usage or by the use of adjoining, downstream, or upstream enhancers) that are functionally equivalent to the genes (and which encode equivalent proteins) can also be used to transfect hosts. Methods for the production of synthetic genes are known in the art. Recombinant hosts can be used for the expression or propagation of genes and polynucleotides of the present invention. The genes and polynucleotides within the scope of the present description can be introduced into a wide variety of microbial or plant hosts, such as bacterial cells, yeast, insect cells, plant cell cultures or plants, mammalian cells. For example, T. parva nucleic acids can be expressed in a recombinant baculovirus (BV) possessing an optimized promoter and translation initiation region operably linked to the T. parva nucleic acid. In a preferred embodiment, an optimized promoter and translation initiation region are operably linked to the T. parva nucleic acid. In one embodiment, insect host cells can be transformed with baculovirus expressing T. parva nucleic acids. In still another embodiment, Tn5 (Trichoplusia ni or High Five.TM.) host cells can be transformed with baculovirus expressing T. parva nucleic acids.

*T. parva* "recombinant protein", is a protein made using recombinant techniques, i.e. through the expression of a recombinant nucleic acid as depicted above. A recombinant protein is distinguished from naturally occurring protein by at least one or more characteristics. For example, the protein may be isolated or purified away from some or all of the proteins and compounds with which it is normally associated in its wild type host, and thus may be substantially pure. For example, an isolated protein is unaccompanied by at least some of the material with which it is normally associated in its natural state, preferably constituting at least about 0.5%, more preferably at least about 5% by weight of the total protein in a given sample. A substantially pure protein comprises at least about 75% by weight of the total protein, with at least about 80% being preferred, and at least about 90% being particularly preferred. The definition includes the production of a protein from one organism in a different organism or host cell. Alternatively, the protein may be made at a significantly higher concentration than is normally seen, through the use of an inducible promoter or high expression promoter, such that the protein is made at increased concentration levels. Alternatively, the protein may be in a form not normally found in nature, as in the addition of an epitope tag or amino acid substitutions, insertions and/or deletions, as discussed below.

Included in the definition of *T. parva* antigen polypeptides are *T. parva* polypeptide variants. These variants fall into one or more of three classes: substitutional, insertional or deletional variants. These variants ordinarily are prepared by site specific mutagenesis of nucleotides in the DNA encoding a *T. parva* antigen polypeptide, using cassette or PCR mutagenesis, scanning mutagenesis, gene shuffling or other techniques well known in the art, to produce DNA encoding the variant, and thereafter expressing the DNA in recombinant cell culture as outlined above. However, variant *T. parva* polypeptide fragments having up to about 100-150 residues may be prepared by in vitro synthesis using established techniques. Amino acid sequence variants are characterized by the predetermined nature of the variation, a feature that sets them apart from naturally occurring allelic or interspecies variation of the *T. parva* antigen polypeptide amino acid sequence.

While the site or region for introducing an amino acid sequence variation is predetermined, the mutation per se need not be predetermined. For example, in order to optimize the performance of a mutation at a given site, random mutagenesis may be conducted at the target codon or region and the expressed T. parva antigen polypeptide variants can be screened for the optimal combination of desired activity. Techniques for making mutations at predetermined sites in DNA having a known sequence are well known. For example, the variations can be made using uligonucleotide-mediated site-directed mutagenesis [Carter et al., Nucl. Acids Res., 13:4331 (1986); Zoller et al., Nucl. Acids Res., 10: 6487 (1987)], cassette mutagenesis [Wells et al., Gene, 34:315 (1985)], restriction selection mutagenesis [Wells et al., Philos. Trans. R. Soc. London SerA, 317:415 (1986)], PCR mutagenesis, or other known techniques can be performed on the cloned DNA to produce *T. parva* antigen polypeptide variant DNA. Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunmingham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol.,150:1 (1976)].

If alanine substitution does not yield adequate amounts of variant, an isoteric amino acid can be used. Screening of the mutants or variants is done using Elispot and/or bioassays of T. parva antigen polypeptide activities and/or properties as described herein.

The present: description further provides fragments of the antigen peptides, in addition to proteins and peptides that comprise and consist of such fragments, particularly those comprising the residues identified in SEQ ID NO:9-17. The fragments to which the description pertains, however, are not to be construed as encompassing fragments that may be disclosed publicly prior to the present invention.

As used herein, a fragment comprises at least about 8, 10, 12, 14, 16, or more contiguous amino acid residues from an antigen peptide. Such fragments can be chosen based on the ability to retain one or more of the biological activities of the antigen peptide or could be chosen for the ability to perform a function, e.g. bind a substrate or act as an immunogen. Particularly important fragments are biologically active fragments, peptides that are, for example, about 8 or more amino acids in length. Such fragments will typically comprise a domain or motif of the antigen peptide, e.g., active site, a transmembrane domain or a substrate-binding domain. Further, possible fragments include, but are not limited to, domain or motif containing fragments, soluble peptide fragments, and fragments containing immunogenic structures. Predicted domains and functional sites are readily identifiable by computer programs well known and readily available to those of skill in the art (e.g., PROSITE analysis).

Polypeptides often contain amino acids other than the 20 amino acids commonly referred to as the 20 naturally occurring amino acids. Further, many amino acids, including the terminal amino acids, may be modified by natural processes, such as processing and other post-translational modifications, or by chemical modification techniques well known in the art. Common modifications that occur naturally in antigen peptides are described in basic texts, detailed monographs, and the research literature, and they are well known to those of skill in the art.

Known modifications include, but are not limited to, acetylation, acylation, ADP-ribosylation, animation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well known to those of skill in the art and have been described in great detail in the scientific literature. Several particularly common modifications, glycosylation, lipid attachment, sulfation, gamma-carboxylation of glutamic acid residues, hydroxylation and ADP-ribosylation, for instance, are described in most basic texts, such as Proteins-Structure and Molecular Properties, 2.sup.nd Ed., T. E. Creighton, W. H. Freeman and Company, New York (1993). Many detailed reviews are available on this subject, such as by Wold, F., Posttranslational Covalent Modification of Proteins, B. C. Johnson, Ed., Academic Press, New York 1-12 (1983); Seifter et al. (Meth Enzymol. 182: 626-646 (1990)) and Rattan et al. (Ann. N.Y Acad. Sci. 653:48-62 (1992)).

Accordingly, the antigen peptides of the present description also encompass derivatives or analogs in which a substituted amino acid residue is not one encoded by the genetic code, in which a substituent group is included, in which the mature antigen peptide is fused with another compound, such as a compound to increase the half-life of the antigen peptide (for example, polyethylene glycol), or in which the additional amino acids are fused to the mature antigen peptide, such as a leader or secretory sequence or a sequence for purification of the mature antigen. peptide or a pro-protein sequence.

Although an amino acid sequence or oligopeptide used for antibody induction does not require biological activity, it must be immunogenic. A peptide, polypeptide, or protein used to induce specific antibodies may have an amino acid sequence consisting of at least five amino acids and preferably at least 10 amino acids. Short stretches of amino acid sequence may be genetically or chemically fused with those of another protein such as keyhole limpet hemocyanin, and the chimeric peptide used for antibody production. Alternatively, the oligopeptide may be of sufficient length to contain an entire domain.

Antibodies specific for peptides, polypeptides, or proteins may be produced by inoculation of an appropriate animal with an antigenic fragment of the peptide, polypeptide, or protein. Antibody production includes not only the stimulation of an immune response by injection into animals, but also analogous processes such as the production of synthetic antibodies, the screening of recombinant immunoglobulin libraries for specific-binding molecules (Orlandi R et al. [1989] PNAS 86:3833-3837, or Huse W. D. et al. [1989] Science 256:1275-1281), or the in vitro stimulation of lymphocyte populations. Current technology (Winter G. and Milstein C. [1991] Nature 349:293-299) provides for a number of highly specific binding reagents based on the principles of antibody formation. These techniques may be adapted to produce molecules which specifically bind antigen peptides. Antibodies or other appropriate molecules generated against a specific immunogenic peptide fragment or oligopeptide can be used in Western analysis, enzyme-linked immunosorbent assays (ELISA) or similar tests to establish the presence of or to quantitate amounts of peptide, polypeptide, or protein in normal, diseased, or transformed cells, tissues, organs, or organisms as well as liquid suspensions containing said peptide, polypeptide, or protein.

The description also provides antibodies that selectively bind to one of the peptides of the present invention, a protein comprising such a peptide, as well as variants and fragments thereof. As used herein, an antibody selectively binds a target peptide when it binds the target peptide and does not significantly bind to unrelated proteins. An antibody is still considered to selectively bind a peptide even if it also binds to other proteins that are not substantially homologous with the target peptide so long as such proteins share homology with a fragment or domain of the peptide target of the antibody. In this case, it would be understood that antibody binding to the peptide is still selective despite some degree of cross-reactivity.

As used herein, an antibody is defined in terms consistent with that recognized within the art: they are multi-subunit proteins produced by a mammalian organism in response to an antigen challenge. The antibodies of the present description include polyclonal antibodies and monoclonal antibodies, as well as fragments of such antibodies, including, but not limited to, Fab or$F(ab')_2$, and Fv fragments.

Many methods are known for generating and/or identifying antibodies to a given target peptide. Several such methods are described by Harlow, Antibodies, Cold Spring Harbor Press, (1989).

In general, to generate antibodies, an isolated peptide is used as an immunogen and is administered to a mammalian organism, such as a rat, rabbit or mouse. The full-length protein, an antigenic peptide fragment or a fusion protein can be used. Particularly important fragments are those covering functional domains, such as the domains identified in the figures, and domain of sequence homology or divergence amongst the family, such as those that can readily be identified using protein alignment methods and as presented in the Figures.

Antibodies are preferably prepared from regions or discrete fragments of the antigen proteins. Antibodies can be prepared from any region of the peptide as described herein. However, preferred regions will include those involved in function/activity and/or antigen/binding partner interaction.

An antigenic fragment will typically comprise at least 8 contiguous amino acid residues. The antigenic peptide can comprise, however, at least 10, 12, 14, 16 or more amino acid residues. Such fragments can be selected on a physical property, such as fragments correspond to regions that are located on the surface of the protein, e. g., hydrophilic regions or can be selected based on sequence uniqueness.

Detection on an antibody of the present description can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, .beta.-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotnazinylamme fluorescein, dansyl chloride or phycoerythrin; an example of a luminescent material includes luminol; examples of bioluminescent materials include luciferase, luciferin, and acquorin, and examples of suitable radioactive material include $^{125}I$, $^{131}I$, $^{35}S$ or $^{3}H$.

The antibodies can be used to isolate one of the proteins of the present invention by standard techniques, such as affinity chromatography or immunoprecipitation. The antibodies can facilitate the purification of the natural protein from cells and recombinantly produced protein expressed in host cells. In addition, such antibodies are useful to detect the presence of one of the proteins of the present invention in cells or tissues to determine the pattern of expression of the protein among various tissues in an organism and over the course of schizont development. Such antibodies can be used to detect protein in situ, in vitro, or in a cell lysate or supernatant in order to evaluate the abundance and pattern of expression. Also, such antibodies can be used to assess abnormal tissue distribution or abnormal expression during development or progression of a protozoan infection. Antibody detection of circulating fragments of the full length protein can be used to identify turnover.

Further, the antibodies can be used to assess expression in disease states such as in active stages of the disease or in lymphocytes harvested from infected animals. Experimental data as provided in FIG. 16 indicates expression in bovine lymphocytes, infected with *T. parva.* If a disease is characterized by a specific mutation in the protein, antibodies specific for this mutant protein can be used to assay for the presence of the specific mutant protein.

The diagnostic uses can be applied, not only in genetic testing, but also in monitoring a treatment modality. Accordingly, where treatment is ultimately aimed at preventing or controlling infection, antibodies directed against the protein or relevant fragments can be used to monitor therapeutic efficacy.

The antibodies are also useful for inhibiting protein function, for example, blocking the binding of the antigenic peptide to a binding partner such as a substrate. These uses can also be applied in a therapeutic context in which treatment involves inhibiting the protein's function. An antibody can be used, for example, to block binding, thus modulating (agonizing or antagonizing) the peptides activity. Antibodies can be prepared against specific fragments containing sites required for function or against intact protein that is associated with a cell or cell membrane. See SEQ ID NO: 1-7 and 9-17 and Fig. 9 and 10, for structural information relating to the proteins of the present description.

The description also encompasses kits for using antibodies to detect the presence of a protein in a biological sample. The kit can comprise antibodies such as a labeled or labelable antibody and a compound or agent for detecting protein in a biological sample; means for determining the amount of protein in the sample; means for comparing the amount of protein in the sample with a standard; and instructions for use. Such a kit can be supplied to detect a single protein or epitope or can be configured to detect one of a multitude of epitopes, such as in an antibody detection array. Arrays are described in detail below for nuleic acid arrays and similar methods have been developed for antibody arrays.

The proteins of the present invention can be used in assays related to the functional information provided in the Figures; for example, to stimulate CD8+ cytotoxic T cell lines, to raise antibodies or to elicit another immune response; as a reagent (including the labeled reagent) in assays designed to quantitatively determine levels of the protein (or its binding partner or ligand) in biological fluids; and as markers for tissues in which the corresponding protein is preferentially expressed (either constitutively or at a particular stage of tissue differentiation or development or in a disease state). Where the protein binds or potentially binds to another protein or ligand (such as, for example, in a enzyme-effector protein interaction or enzyme-ligand interaction), the protein can be used to identify the binding partner/ligand so as to develop a system to identify inhibitors of the binding interaction. Any or all of these uses are capable of being developed into reagent grade or kit format for commercialization as commercial products.

Methods for performing the uses listed above are well known to those skilled in the art. References disclosing such methods include "Molecular Cloning: A Laboratory Manual", 2d ed., Cold Spring Harbor Laboratory Press, Sambrook, J., E. F. Fritsch and T. Maniatis eds., 1989, and "Methods in Enzymology: Guide to Molecular Cloning Techniques", Academic Press, Berger, S. L. and A. R. Kimmel eds., 1987.

The potential uses of the peptides of the present invention are based primarily on the source of the protein as well as the class/action of the protein. For example, antigens isolated from T. parva and their protozoan orthologs serve as targets for identifying agents for use in mammalian therapeutic applications, e.g. an animal drug, particularly in modulating a biological or pathological response in a cell, tissue, or protozoan, that expresses an immunogenic antigen. Experimental data as provided in FIG. 16 indicates that the antigens of the present description are expressed in infected host lymphocytes, as indicated by flow cytometric analysis. Such uses can readily be determined using the information provided herein, that which is known in the art, and routine experimentation.

To perform assays of the description such as astsays to detect a pathogen, *e.g.*, by detecting the presence and/or expression of a polypeptide of the invention in the pathogen, it is sometimes desirable to immobilize either the antigen protein, or fragment, or its target molecule to facilitate separation of complexes from uncomplexed forms of one or both of the proteins, as well as to accommodate automation of the assay.

Techniques for immobilizing proteins on matrices can be used in assays of the description. In one embodiment, a fusion protein can be provided which adds a domain that allows the protein to be bound to a matrix. For example, glutathione-S-transferase fusion proteins can be adsorbed onto glutathione sepharose beads (Sigma Chemical, St. Louis, Mo.) or glutathione derivabed microtitre plates, which are then combined with the cell lysates (e.g., $^{35}$S-labeled) and the candidate compound, and the mixture incubated under conditions conducive to complex formation (e.g., at physiological conditions for salt and pH). Following incubation, the beads are washed to remove any unbound label, and the matrix immobilized and radiolabel determined directly, or in the supernatant after the complexes are dissociated. Alternatively, the complexes can be dissociated from the matrix, separated by SDS-PAGE, and the level of antigen-binding protein found in the bead fraction quantitated from the gel using standard electrophoretic techniques. For example, either the polypeptide or its target molecule can be immobilized utilizing conjugation of biotin and streptavidin using techniques well known in the art. Alternatively, antibodies reactive with the protein but which do not interfere with binding of the protein to its target molecule can be derivatized to the wells of the plate, and the protein trapped in the wells by antibody conjugation. Preparations of an antigen-binding protein and a candidate compound are incubated in the antigen protein-presenting wells and the amount of complex trapped in the well can be quantitated. Methods for detecting such complexes, in addition to those described above for the GST-immobilized complexes, include immunodetection of complexes using antibodies reactive with the antigen protein target molecule, or which are reactive with antigen protein and compete with the target molecule, as well as enzyme-linked assays which rely on detecting an enzymatic activity associated with the presence of the target molecule.

The antigen proteins of the present invention are also useful to provide a target for diagnosing a disease or a disease mediated by the peptide. Accordingly, the description provides methods for detecting the presence, or levels of, the protein (or encoding RNA) in a cell, tissue, or organism. Experimental data as provided in FIG. 16 indicates expression in T. parva-infected bovine lymphocytes. The method involves contacting a biological sample with a compound capable of interacting with the antigen protein such that the interaction can be detected. Such an assay can be provided in a single detection format or a multi-detection format such as an antibody chip array.

One agent for detecting a protein in a sample is an antibody capable of selectively binding to protein. A biological sample includes tissues, cells and biological fluids isolated from a subject, as well as tissues, cells and fluids present within a subject.

The peptides of the present invention also provide targets for diagnosing active parasite activity or disease, in an animal having a variant peptide, particularly activities and conditions that are known for other members of the family of proteins to which the present one belongs. Thus, the peptide can be isolated from a biological sample and assayed for the presence of a genetic mutation that results in aberrant peptide. This includes amino acid substitution, deletion, insertion, rearrangement, (as the result of aberrant splicing events), and inappropriate post-translational modification. Analytic methods include altered electrophoretic mobility, altered tryptic peptide digest, altered enzyme activity in cell-based or cell-free assay, alteration in substrate or antibody-binding pattern, altered isoelectric point, direct amino acid sequencing, and any other of the known assay techniques useful for detecting mutations in a protein. Such an assay can be provided in a single detection format or a multi-detection format such as an antibody chip array.

In vitro techniques for detection of peptide include enzyme linked immunosorbent assays (ELISAs), Western blots, immunoprecipitations and immunofluorescence using a detection reagent, such as an antibody or protein binding agent, as well as the methods that represent embodiments of the present invention. Alternatively, the peptide can be detected in vivo in a subject by introducing into the subject a labeled anti-peptide antibody or other types of detection agent. For example, the antibody can be labeled with a radioactive marker whose presence and location in a subject can be detected by standard imaging techniques. Such methods can be used to detect the allelic variant of a peptide expressed in an infected subject and methods which detect fragments of a peptide in a sample.

The diagnostic uses can be applied, not only in genetic testing, but also in monitoring a treatment modality. Accordingly, where treatment is ultimately aimed at preventing or controlling infection, antibodies directed against the protein or relevant fragments can be used to monitor therapeutic efficacy.

The description also encompasses kits for using antibodies to detect the presence of a protein in a biological sample. The kit can comprise antibodies such as a labeled or labelable antibody and a compound or agent for detecting protein in a biological sample; means for determining the amount of protein in the sample; means for comparing the amount of protein in the sample with a standard; and instructions for use. Such a kit can be supplied to detect a single protein or epitope or can be configured to detect one of a multitude of epitopes, such as in an antibody detection array. Arrays are described in detail below for nucleic acid arrays and similar methods have been developed for antibody arrays.

The present invention further provides isolated nucleic acid molecules that encode a T. parva antigen peptide or protein of the present invention (cDNA, transcript and genomic sequences). Such nucleic acid molecules will consist of, consist essentially of, or comprise a nucleotide sequence that encodes one of the enzyme peptides of the present description, an allelic variant thereof, or an ortholog or paralog thereof.

As used herein, an "isolated" nucleic acid molecule is one that is separated from other nucleic acid present in the natural source of the nucleic acid. Preferably, an "isolated" nucleic acid is free of sequences which naturally flank the nucleic acid (i.e., sequences located at the 5' and 3' ends of the nucleic acid) in the genomic DNA of the organism from which the nucleic acid is derived. However, there can be some flanking nucleotide sequences, for example up to about 5KB, 4KB, 3KB, 2KB, or 1KB or less, particularly contiguous peptide encoding sequences and peptide encoding sequences within the same gene but separated by introns in the genomic sequence. The important point is that the nucleic acid is isolated from remote and unimportant flanking sequences such that it can be subjected to the specific manipulations described herein such as recombinant expression, preparation of probes and primers, and other uses specific to the nucleic acid sequences.

Moreover, an "isolated" nucleic acid molecule, such as a transcript/cDNA molecule, can be substantially free of other cellular material, or culture medium when produced by recombinant techniques, or chemical precursors or other chemicals when chemically synthesized. However, the nucleic acid molecule can be fused to other coding or regulatory sequences and still be considered isolated.

For example, recombinant DNA molecules contained in a vector are considered isolated. Further examples of isolated DNA molecules include recombinant DNA molecules maintained in heterologous host cells or purified (partially or substantially) DNA molecules in solution. Isolated RNA molecules include in vivo or in vitro RNA transcripts of the isolated DNA molecules of the present invention. Isolated nucleic acid molecules according to the present invention further include such molecules produced synthetically.

The nucleic acid may be double stranded, single stranded, or contain portions of both double stranded or single stranded sequence. As will be appreciated by those in the art, the depiction of a single strand ("Watson") also defines the sequence of the other strand ("Crick"); thus the sequences depicted in the figures also include the complement of the sequence.

As disclosed herein, 100% sequence identity between the RNA and the target gene is not required to practice the present invention. Thus the invention has the advantage of being able to tolerate sequence variations that might be expected due to genetic mutation, strain polymorphism, or evolutionary divergence. RNAi molecules of the subject description are not limited to those that are targeted to the full-length polynucleotide or gene. Gene product can be inhibited with a RNAi molecule that is targeted to a portion or fragment of the exemplified polynucleotides; high homology (90-95%) or greater identity is also preferred, but not necessarily essential, for such applications.

Accordingly, the present description provides nucleic acid molecules that consist of, consist essentially of, or comprise, the nucleotide sequence shown in SEQ ID NO: 18-22 or any nucleic acid molecule that encodes a protein encoded by SEQ ID NO: 18-22 (as shown, e.g., in Figures 9, 10, and 17).

The term "consisting essentially of," when used in the context ofbiopolymers, refers to a sequence which is intermediate between the number of residues (amino acids or, in the present case, nucleotides) encompassed by the term "consisting of" and the longer length encompassed by the term "comprising." Residues in addition to the residues encompassed by "consisting of" language do not affect the basic and novel characteristics (*e.g.*, in the case of a polynucleotide, the ability to encode a functional peptide, or to bind specifically to a nucleic acid of interest) of the molecule encompassed by the "consisting of" language.

The description further provides nucleic acid molecules that encode fragments of the peptides of the present description as well as nucleic acid molecules that encode obvious variants of the enzyme proteins of the present description that are described above. Such nucleic acid molecules may be naturally occurring, such as allelic variants (same locus), paralogs (different locus), and orthologs (different organism), or may be constructed by recombinant DNA methods or by chemical synthesis. Such non-naturally occurring variants may be made by mutagenesis techniques, including those applied to nucleic acid molecules, cells, or organisms. Accordingly, as discussed above, the variants can contain nucleotide substitutions, deletions, inversions and insertions. Variation can occur in either or both the coding and non-coding regions. The variations can produce both conservative and non- conservative amino acid substitutions.

The present description further provides epitope fragments of the antigen proteins and the nucleic acid molecules encoding the antigens, provided in the description of epitope-mapping experiments described below. An epitope coding region comprises a contiguous nucleotide sequence greater than 12 or more nucleotides. Further, a fragment could at least 30, 40, 50, 100, 250 or 500 nucleotides in length. The length of the fragment will be based on its intended use. For example, the fragment can encode epitope bearing regions of the peptide, or can be useful as DNA probes and primers. Such fragments can be isolated using the known nucleotide sequence to synthesize an oligonucleotide probe. A labeled probe can then be used to screen a cDNA library, genomic DNA library, or mRNA to isolate nucleic acid corresponding to the coding region. Further, primers can be used in PCR reactions to clone specific regions of gene.

A probe/primer typically comprises substantially a purified oligonucleotide or oligonucleotide pair. The oligonucleotide typically comprises a region of nucleotide sequence that hybridizes under stringent conditions to at least about 12, 20, 25, 40, 50 or more consecutive nucleotides.

Orthologs, homologs, and allelic variants can be identified using methods well known in the art. As described in the Peptide Section, these variants comprise a nucleotide sequence encoding a peptide that is typically 60-70%, 70-80%, 80-90%, and more typically at least about 90-95% or more homologous to the nucleotide sequence shown in the Figure sheets or a fragment of this sequence. Such nucleic acid molecules can readily be identified as being able to hybridize under moderate to stringent conditions, to the nucleotide sequence shown in the Figure sheets or a fragment of the sequence. Allelic variants can readily be determined by genetic locus of the encoding gene. The gene encoding the novel enzyme of the present description is located on a genome component that has been mapped to the *T. parva* chromosomel and 2 which is supported by multiple lines of evidence, such as STS and BAC map data.

The nucleic acid molecules of the present invention are useful as probes, primers, chemical intermediates, and in biological assays. The nucleic acid molecules are useful as a hybridization probe for messenger RNA, transcript/ cDNA and genomic DNA to isolate full-length cDNA and genomic clones encoding the peptide described in SEQ ID NO: 9-17 and to isolate cDNA and genomic clones that correspond to variants (alleles, orthologs, etc.) producing the same or related peptides shown in SEQ ID NO:1-7 and 9-17. As illustrated in FIG. 10, deletions were identified at 2 different nucleotide positions.

The probe can correspond to any sequence along the entire length of the nucleic acid molecules provided in the SEQ ID information. Accordingly, it could be derived from 5' noncoding regions, the coding region, and 3' noncoding regions. However, as discussed, fragments are not to be construed as encompassing fragments disclosed prior to the present invention.

The nucleic acid molecules are also useful as primers for PCR to amplify any given region of a nucleic acid molecule and are useful to synthesize antisense molecules of desired length and sequence.

The nucleic acid molecules are also useful for constructing recombinant vectors. Such vectors include expression vectors that express a portion of, or all of, the peptide sequences. Vectors also include insertion vectors, used to integrate into another nucleic acid molecule sequence, such as into the cellular genome, to alter in situ expression of a gene and/or gene product. For example, an endogenous coding sequence can be replaced via homologous recombination with all or part of the coding region containing one or more specifically introduced mutations.

The nucleic acid molecules are also useful for expressing antigenic portions of the proteins.

The nucleic acid molecules are also useful as probes for determining the chromosomal positions of the nucleic acid molecules by means of in situ hybridization methods. The genes encoding the novel antigens of the present description are located on a genome component that has been mapped to *T. parva* chromosomes 1 (Tp1 & Tp4) and 2 (Tp5, Tp7 & Tp8).

The nucleic acid molecules are also useful in making vectors containing the gene regulatory regions of the nucleic acid molecules of the present description.

The nucleic acid molecules are also useful for designing antigens corresponding to all, or a part, of the mRNA produced from the nucleic acid molecules described herein.

The nucleic acid molecules are also useful for making vectors that express part, or all, of the peptides.

The nucleic acid molecules are also useful for constructing host cells expressing a part, or all, of the nucleic acid molecules and peptides.

The nucleic acid molecules are also useful as hybridization probes for determining the presence, level, form and distribution of nucleic acid expression. Such probes could be used to detect the presence of, or to determine levels of, a specific nucleic acid molecule in cells, tissues, and in organisms. The nucleic acid whose level is determined can be DNA or RNA. Accordingly, probes corresponding to the peptides described herein can be used to assess expression and/or gene copy number in a given cell, tissue, or organism. These uses are relevant for diagnosis of disorders involving an increase or decrease in antigen protein expression relative to normal results.

In vitro techniques for detection of mRNA include Northern hybridizations and in situ hybridizations. In vitro techniques for detecting DNA includes Southern hybridizations and in situ hybridization.

Probes can be used as a part of a diagnostic test kit for identifying cells or tissues that express an T. parva antigen protein, such as by measuring a level of an antigen-encoding nucleic acid in a sample of cells from a subject e.g., mRNA or genomic DNA, or determining if an antigen gene has been mutated.

The : description encompasses kits for detecting the presence of an antigen nucleic acid in a biological sample. For example, the kit can comprise reagents such as a labeled or labelable nucleic acid or agent capable of detecting antigen nucleic acid in a biological sample; means for determining the amount of antigen nucleic acid in the sample; and means for comparing the amount of antigen nucleic acid in the sample with a standard. The compound or agent can be packaged in a suitable container. The kit can further comprise instructions for using the kit to detect antigen protein mRNA or DNA.

The present description further provides nucleic acid detection kits, such as arrays or microarrays of nucleic acid molecules that are based on the sequence information provided in SEQ ID NO: 18-31.

As used herein "Arrays" or "Microarrays" refers to an array of distinct polynucleotides or oligonucleotides synthesized on a substrate, such as paper, nylon or other type of membrane, filter, chip, glass slide, or any other suitable solid support. In one embodiment, the microarray is prepared and used according to the methods described in U.S. Pat. No. 5,837,832, Chee et aL, PCT application W095/11995 (Chee et al.), Lockhart, D. J. et al. (1996; Nat. Biotech. 1.4: 1675-1680) and Schena, M. et al. (1996; Proc. Natl. Acad. Sci. 93: 10614-10619). In other embodiments, such arrays are produced by the methods described by Brown et al., U.S. Pat. No. 5,807,522.

The microarray or detection kit is preferably composed of a large number of unique, single-stranded nucleic acid sequences, usually either synthetic antisense oligonucleotides or fragments of cDNAs, fixed to a solid support. The oligonucleotides are preferably about 6-60 nucleotides in length, more preferably 15-30 nucleotides in length, and most preferably about 20-25 nucleotides in length. For a certain type of microarray or detection kit, it may be preferable to use oligonucleotides that are only 7-20 nucleotides in length. The microarray or detection kit may contain oligonucleotides that cover the known 5', or 3', sequence, sequential oligonucleotides which cover the full length sequence; or unique oligonucleotides selected from particular areas along the length of the sequence. Polynucleotides used in the microarray or detection kit may be oligonucleotides that are specific to a gene or genes of interest.

In order to produce oligonucleotides to a known sequence for a microarray or detection kit, the gene(s) of interest (or an ORF identified in the present description) is typically examined using a computer algorithm which starts at the 5' or at the 3' end of the nucleotide sequence. Typical algorithms will then identify oligomers of defined length that are unique to the gene, have a GC content within a range suitable for hybridization, and lack predicted secondary structure that may interfere with hybridization. In certain situations it may be appropriate to use pairs of oligonucleotides on a microarray or detection kit. The "pairs" will be identical, except for one nucleotide that preferably is located in the center of the sequence. The second oligonucleotide in the pair (mismatched by one) serves as a control. The number of oligonucleotide pairs may range from two to one million. The oligomers are synthesized at designated areas on a substrate using a light-directed chemical process. The substrate may be paper, nylon or other type of membrane, filter, chip, glass slide or any other suitable solid support.

In another aspect, an oligonucleotide may be synthesized on the surface of the substrate by using a chemical coupling procedure and an ink jet application apparatus, as described in PCT application W095/251116 (Baldeschweiler et al.).

In another aspect, a "gridded" array analogous to a dot (or slot) blot may be used to arrange and link cDNA fragments or oligonucleotides to the surface of a substrate using a vacuum system, thermal, UV, mechanical or chemical bonding procedures. An array, such as those described above, may be produced by hand or by using available devices (slot blot or dot blot apparatus), materials (any suitable solid support), and machines (including robotic instruments), and may contain 8, 24, 96,384,1536, 6144 or more oligonucleotides, or any other number between two and one million which lends itself to the efficient use of commercially available instrumentation.

In order to conduct sample analysis using a microarray or detection kit, the RNA or DNA from a biological sample is made into hybridization probes. The mRNA is isolated, and cDNA is produced and used as a template to make antisense RNA (aRNA). The aRNA is amplified in the presence of fluorescent nucleotide, and labeled probes are incubated with the microarray or detection kit so that the probe sequences hybridize to complementary oligonucleotides of the microarray or detection kit. Incubation conditions are adjusted so that hybridization occurs with precise complementary matches or with various degrees of less complementarity. After removal of nonhybridized probes, a scanner is used to determine the levels and patterns of fluorescence. The scanned images are examined to determine degree of complementarity and the relative abundance of each oligonucleotide sequence on the microarray or detection kit. The biological samples may be obtained from any bodily fluids (such as blood, urine, saliva, phlegm, gastric juices, etc.), cultured cells, biopsies, or other tissue preparations. A detection system may be used to measure the absence, presence, and amount of hybridization for all of the distinct sequences simultaneously. This data may be used for large-scale correlation studies on the sequences, expression patterns, mutations, variants, or polymorphisms among samples.

Using such arrays, the present description provides methods to identify the presence or expression of the antigen proteins/peptides of the present invention. In detail, such methods comprise incubating a test sample with one or more nucleic acid molecules and assaying for binding of the nucleic acid molecule with components within the test sample. Such assays will typically involve arrays comprising many genes, at least one of which is a gene of the present description and or alleles of the enzyme gene of the present description. The figures and associated information below provide information on epitope sequence and micro-variation in strains of *T. parva,* that have been found in the gene encoding the antigen of the present description.

Conditions for incubating a nucleic acid molecule with a test sample vary. Incubation conditions depend on the format employed in the assay, the detection methods employed, and the type and nature of the nucleic acid molecule used in the assay. One skilled in the art will recognize that any one of the commonly available hybridization, amplification or array assay .formats can readily be adapted to employ the novel fragments of the T. parva genome disclosed herein. Examples of such assays can be found in Chard, T, An Introduction to Radioimmunoassay and Related Techniques, Elsevier Science Publishers, Amsterdam, The Netherlands (1986); Bullock, G. R. et al., Techniques in Immunocytochemistry, Academic Press, Orlando, Fla. Vol. 1 (1982), Vol. 2 (1983), Vol. 3 (1985); Tijssen, P., Practice and Theory of Enzyme Immunoassays: Laboratory Techniques in Biochemistry and Molecular Biology, Elsevier Science Publishers, Amsterdam, The Netherlands (1985).

The test samples of the present description include cells, protein or membrane extracts of cells. The test sample used in the above-described method will vary based on the assay format, nature of the detection method and the tissues, cells or extracts used as the sample to be assayed. Methods for preparing nucleic acid extracts or of cells are well known in the art and can be readily be adapted in order to obtain a sample that is compatible with the system utilized.

In another embodiment of the present description, kits are provided which contain the necessary reagents to carry out the assays of the present description.

Specifically, the description provides a compartmentalized kit to receive, in close confinement, one or more containers which comprises: (a) a first container comprising one of the nucleic acid molecules that can bind to a fragment of the *T. parva* genome disclosed herein; and (b) one or more other containers comprising one or more of the following: wash reagents, reagents capable of detecting presence of a bound nucleic acid.

In detail, a compartmentalized kit includes any kit in which reagents are contained in separate containers. Such containers include small glass containers, plastic containers, strips of plastic, glass or paper, or arraying material such as silica. Such containers allows one to efficiently transfer reagents from one compartment to another compartment such that the samples and reagents are not cross-contaminated, and the agents or solutions of each container can be added in a quantitative fashion from one compartment to another. Such containers will include a container which will accept the test sample, a container which contains the nucleic acid probe, containers which contain wash reagents (such as phosphate buffered saline, Tris-buffers, etc.), and containers which contain the reagents used to detect the bound probe. One skilled in the art will readily recognize that the previously unidentified enzyme gene of the present description can be routinely identified using the sequence information disclosed herein can be readily incorporated into one of the established kit formats which are well known in the art, particularly expression arrays.

The *T. parva* antigens of the present description have particular potential for induction of in vivo, antigen-specific CD8+ cytotoxic T cells for prophylactic immunization of cattle for the prevention of East Coast Fever disease. In addition, CTLs specific to the following metazoan parasites may also be induced by compositions identified in accordance with the methods of the present description: *Plasmodium falciparum* (which causes malaria), Schistosoma mansoni (which causes schistosomiasis), and Trypanosoma cruzi (which causes Chagas' disease), Giardia lamblia, Entoemeba histolytica, Cryptospiridium spp., Leishmania spp., Brugia spp., Wuchereria spp., Onchocerca spp., Strongyloides spp., Coccidia, Haemanchus spp., Ostertagia spp., Trichomonas spp., Dirofilaria spp., Toxocara spp., Naegleria spp., Pneumocystis carinii, Ascaris spp., other Trypanosoma spp., other Schistosome spp., other Plasmodium spp., Babesia spp., Theileria spp., including but not limited to T. parva, T. lawrencei, T. annulata, T. hirci, T. ovis, T. lastoguardi, T. orientalis, T. buffeli and T. taurotragi, Babesia spp., including, but not limited to B. bigemina, B. divergens, B. major, B. bovis, B. motasi, B. ovis, B. cabelli, B. equii, B. traumani, B. canis, B. gibsoni, B. felis, and B. microfti, Adelina app., including, but not limited to A. delina, A. castana, A. picei, A. palori, and A. triboli, Anisakis and Isospora beli.

Following are examples which illustrate procedures for practicing the invention. These examples should not be construed as limiting. All percentages are by weight and all solvent mixture proportions are by volume unless otherwise noted. As used herein and further defined below, "nucleic acid" may refer to either DNA or RNA, or molecules which contain both deoxy and ribonucleotides. The nucleic acids include genomic DNA, cDNA, and oligonucleotides including sense and anti-sense nucleic acids. Such nucleic acids may also contain modifications in the ribose-phosphate backbone to increase stability and half life of such molecules in physiological environments.

## EXAMPLES

### Generation of a unidirectional cDNA expression library of *Theileria parva* schizont stage

Generation of *Theileria parva* Muguga-infected cell lines (TpM) --Peripheral blood mononuclear cells (PBMC) were isolated from venous blood and infected with *T. parva* (Muguga) sporozoites in accordance with published protocols (Goddeeris & Morrison, 1988). Infected cell lines were maintained in RPMI-1640 supplemented with 10% Fetaclone II (Hyclone, UK; tested for BVDV & *Mycoplamsa spp.),* 100iU/ml Penicillin, 100$\mu$g/ml Streptomycin, 50$\mu$g/ml Gentamycin, 5x10$^{-5}$M 2-mercaptoethanol and 2mM L-Glutamine and passaged 1/5 three times a week.

### Purification of schizonts

TpM in log growth phase, were harvested and placed in 50 ml Falcon tubes and spun down at 1500 rpm for 10 minutes at room temperature. The cell pellet was resuspended in 50 ml of plain culture medium (RPMI 1640 with 25mM HEPES) and a cell count was [erformed. The suspensions were spun down again to pellet the cells. The cell pellet was resuspended in the residual medium. The cell suspension was mixed with diluted antibody (anti-class I; IL-A19; dilution 1:500 or IL-A88, dilution 1:100), that had been diluted in plain culture medium to yield a final concentration of 1x10$^{8}$ cells per ml. Cell suspensions were then incubated on ice for 30 minutes. Excess antibody was removed by washing that entailed adding plain culture medium to 50 ml and centrifuging the suspension at 1000 rpm for 10 minutes at room temperature.

The cell pellet was resuspended in 2-3 ml of plain culture medium, and freshly thawed rabbit serum (Complement, previously tested for toxicity) was added to a final dilution of 1:3 to 1:5 and to a final concentration of $5x10^7$ or $1x10^8$ cells per ml. The suspension was Incubated at 37 °C for 30-90 minutes either in a water bath with shaking at 15 minutes interval until lysis was indicated to be complete (lysis checked by trypan blue differential staining using microscope). The suspension were spun at low speed (800 rpm) for 3 minutes to remove large cell debris. Then the schizont-rich supernatant was transferred into a fresh tube and centrifuged at 2,500 rpm for 10 minutes at 4 °C. The supernatant was discarded and the schizont-rich pellet was resuspended in 6 ml of plain culture medium. Schizonts from host cell debris and nuclei were separated by Percoll gradient centrifugation as described by Baumgartner et al. (Baumgartner *et al.*, 1999).

**Isolation of mRNA-poly($A^+$) from schizonts**

Poly($A^+$)RNA was isolated from frozen pellets containing approximately $3x10^9$ schizonts (from F100 TpM) using FastTrack 2.0 kit (Invitrogen). Frozen schizont pellets were thawed rapidly at room temperature then resuspended and lysed in 15 ml FastTrack 2.0 lysis buffer. The purification of poly($A^+$)RNA was done following the recommendations of the manufacturer (Invitrogen). The total amount of poly($A^+$)RNA isolated and dissolved in 20 μl of sterile distilled water, as estimated by ethidium-bromide-stained agarose gel, was 2 μg. For subsequent analyses, the 20 μl poly($A^+$)RNA in a microcentrifuge tube was precipitated on dry-ice for 20 minutes after addition of 2 μl of 3 M sodium acetate buffer at pH 5.2, 50 μl of absolute ethanol. The frozen solution was thawed and centrifuged at 14,000 rpm for 15 minutes at 4 °C. The ethanol was removed and the RNA precipitate was resuspended in 1 ml of 70% ethanol and re-centrifuged. After removing the ethanol, the RNA was air-dried at room temperature and re-dissolved in 6 μl of water. It was then used to construct a unidirectional cDNA library using Invitrogen cDNA synthesis kit (Cat. No. 11917-010).

**Synthesis of cDNA**

In a microcentrifuge tube, schizont poly($A^+$)RNA (2 μg in 6 μl water) was mixed with 2 μl (1 μg) of oligo(dT) *Not* I primer-adapter and incubated at 70 °C for 10 minutes. The mixture was spun down briefly then 4 μl 5x first strand buffer, 2 μl 0.1 M DTT, 1 μl 10 mM dNTPs mix and 1 μl [$\alpha$-$^{32}$P]dCTP (1 μCi/μl) were added, mixed and incubated at 37 °C for 2 minutes. After adding 4μl SuperScript II RT, the reaction was mixed gently and incubated at 37 °C for 1 hour. The reaction was placed on ice. One microlitre was removed and used for the calculation of the first strand cDNA yield. The remaining 19 μl was used for the second strand cDNA synthesis. To the remaining 19 μl first strand cDNA reaction placed on ice, 92 μl $H_2O$, 30 μl 5x second strand buffer, 3 μl 10 mM dNTP mix, 1 μl *E. coli* DNA ligase (10 U/μl), 4 μl *E. coli* DNA polymerase I (10 U/μl) and 1 μl *E. coli* RNAse H (2 U/μl) were added. The reaction was mixed gently, spun down briefly and incubated at 16 °C for 2 hours. Two μl (10 U) T4 DNA polymerase was added to the reaction, mixed and incubated for additional 5 minutes at 16 °C. The reaction was stopped by placing the tube on ice and adding 10 μl 0.5 M EDTA. One hundred and fifty microlitres of phenol-chloroform solution was added to the cDNA reaction, and then the mixture was vortexed thoroughly for 30 seconds and spun in a microcentrifuge at 14,000 rpm for 10 minutes at 4 °C. The top aqueous phase was transferred in a fresh tube and extracted with 150 μl chloroform as above. The top aqueous phase was transferred in a fresh tube to which 75 μl 7.5 M ammonium acetate and 450 μl absolute ethanol were added and mixed. The tube was immediately centrifuged at 14,000 rpm for 20 minutes at room temperature. The supernatant was carefully removed and the cDNA pellet was gently overlaid with 0.5 ml of 70% ethanol. The tube was centrifuged at 14,000 rpm for 2 minutes at room temperature. The supernatant was removed and the cDNA pellet was air-dried at room temperature and then redissolved in 18 μl $H_2O$.

**Ligation and fractionation of cDNA**

The ligation was done using Invitrogen kit. The ligation reaction was prepared by adding to the tube containing the 18 μl of ds-cDNA the following reagents: 10 μl 5x Adapter buffer, 10 μl BstX I adapter (at 1 μg/μl in $H_2O$) 7 μl 0.1M DTT and 5 μl T4 DNA ligase. The reaction was mix gently and incubated at 16 °C for 20 hours. The ligation reaction was stopped by incubating the tube at 70 °C for 10 minutes then chilling on ice. To the 50 μl of ligation reaction, the following reagents were added and the mixture incubated at 37 °C for 90 minutes: 30 μl $H_2O$, 10 μl 10x buffer 3 (15 U/μl, GIBCO). A Sepharose CL-2B gel filtration chromatography was prepared during Not I digestion reaction by washing 4 times with 0.8 ml TEN buffer (TEN buffer =10 mM Tris-Cl, pH 7.5, 0.1 mM EDTA, 25 mM NaCL). The column is very slow and each wash takes about approx. 20 minutes. Fifty microlitre of $H_2O$ was added to the Not I digestion (final volume: 100 μl) and the reaction was loaded onto the column at the center of the resin. All the flow through in a single 1.5 ml Eppendorf tube labeled tube 1. Then 100 μl of TEN was added onto the column and the flow through collected in a single 1.5 ml Eppendorf tube labeled tube 2. From this step, TEN was added to the resin in fractions of 100 μl and only one drop of the flow through was collected in each tube. Fraction collection was done up to tube 24. Each tubes containing cDNA fractions from chromatography was placed in a counting container (with no scintillation fluid) and counted using the tritium channel of a Beckman counter machine. Fractions representing the first 300 μl and fractions collected after the first 3000 μl were discarded. The remaining were grouped into 4 fractions represented by tubes 7 to 12 (fraction A), tubes 13 and 14 (fraction B), tubes 15 and 16 (fraction C) and tubes 17 and 18 (fraction D). The amount of cDNAs was as followed: fraction A, 13 ng; fraction B, 46 ng, fraction C, 95 ng; and fraction D, 82 ng. Five microlitres of each fraction was run on a 1% agarose gel in 0.5x TAE buffer at 7 V/m. The gel was then incubated in 10% TCA containing 1% NaPPi for 20 minutes. The gel was placed on a piece of Whatman 3M paper and overlaid with a pad of absorbent paper and a 500g weight for 10 minutes. The pad of absorbent paper and weight were removed and the gel was dried in a gel dryer under vacuum for 20 minutes. The dried gel was exposed to autoradiographic film at -80°C overnight (Figure 1).

**Cloning of cDNA into pcDNA3 plasmid vector.**

Each of the 4 cDNA fractions was adjusted to 150 μl by adding $H_2O$ and 1 μl (10 μg yeast t-RNA). Then 75 μl of 7.5 M NH4OAc, followed by 450 μl of ice-cold absolute ethanol were added. The mixture was vortexed thoroughly and immediately centrifuged at room temperature for 20 min at 14,000 rpm. The supernatant was removed carefully (and disposed properly), and then the pellet was overlaid with 0.5 ml of ice-cold 70% ethanol and centrifuged for 2 min at 14,000 rpm. The supernatant was removed carefully (and disposed properly). The cDNA was air-dried at room temperature and re-dissolved in $H_2O$ as follow: fraction A in 14 μl, fraction B in 28 μl, fraction in 70 μl and fraction D in 56 μl. Bacteria *E. coli* DH5α cells containing expression plasmid pcDNA3 were inoculated in 50 ml of 2x YT medium (tryptic soy broth 16 g/l; yeast extract, 10 g/l; NaCl, 5.0 g/l) containing 100 μg/ml ampicillin and grown overnight at 37°C with vigorous shaking. Bacteria culture was transferred in a 50 ml Falcon centrifuge tube and the cells were pelleted by centrifugation at 3,000 rpm in a minifuge (Heraeus Christ) for 10 minutes at room temperature. The supernatant was discarded and plasmid DNA was purified from bacteria pellet using the QIAprep miniprep (Qiagen)) DNA purification system. Plasmid DNA was eluted in $H_2O$. Twenty microgram of plasmid DNA (8 μl) was digested with 15 μl (10 U/μl) of the restriction enzyme Not I (Promega) in 300 μl reaction mixture containing 1x buffer D (Promega) at 37 °C for 3 hours. Complete digestion and linearization of the plasmid were confirmed by running 2 μg (30 μl) of the plasmid on a 0.7% agarose gel. The rest of the Not I reaction mix (270 μl) was mixed with 15 μl of BstX 1 (10 U/μl) and 15 μl of water and incubated at 55 °C for 3 hours. Then 15 μl of the reaction was checked on a 0.7% agarose gel. The rest of the Not I/BstX I double digested pcDNA3 vector (285 μl) was dephosphorylated with 3 μl (3 U) alkaline phosphatase at 37 °C for 2 hours. Digested and dephosphorylated plasmid vector was purified using CHROMA SPIN-1000 column (Clontech). The concentration of the vector was adjusted at 50 μg/ml in water. 1 μl (50 ng) of pcDNA3 was mixed with 1 μl (1 U) T4 DNA ligase (Promega), 1 μl 10x ligation buffer and 7 μl $H_2O$ and incubated at 16 °C for 16 hours. The reaction was stopped by heating at 70 °C for 10 minutes. Then 10 μl of $H_2O$ is added to give a final concentration of 2.5 ng/μl. 40 μl of electrocompetent bacteria DH5α previously prepared and tested for their efficiency were electroporated with 2.5 ng (1 μl) of pcDNA3 at 2.49 kV/25 μF using a cuvette of 0.2 cm electrode (BioRad) cold on ice. The electroporated bacteria were resuspended in 1 ml LB medium, incubated at 37 °C for 1 hour and then plated on LB agar plates and incubated overnight at 37 °C. Plasmid vector and ds-cDNAs were mixed in a ratio of 50 ng of vector for 10-20 ng of cDNAs in a 20-μl ligation reaction containing 4 units of T4 DNA ligase (Promega). Thus the final volume of ligation of different was as fellow: 20 μl for the 13 ng of fraction A, 40 μl for the 28 ng of fraction B, 100 μl for the 95 ng of fraction C, and 80 μl for the 82 ng of fraction D. Fraction E was a control ligation reaction of 40 μl containing 40 ng pcDNA3 and no ds-cDNAs. The ligation reactions were incubated at 4 °C for 16 hours. Water was added to each ligation mix to a final volume of 150 μl. Followed by 1 μl of 10 μg/μl yeast t-RNA. Each ligation was extracted with 150 μl phenol-chloroform by vortexing for 30 seconds and spinning at 14,000 rpm for 10 minutes at 4 °C. The top aqueous phase was reextracted with 150 μl chloroform. The top aqueous phase was transferred in a fresh tube and the DNA was precipitated by adding 0.1 volume of 3 M NaAc, pH 5.2, and 2.5 volumes (375 μl) of ice-cold absolute ethanol, followed by mixing briefly and incubating for15 minutes on dry-ice. The tubes were thawed at room temperature and centrifuged at 14,000 rpm for 10 minutes at 4 °C to pellet the DNA. The DNA pellet was washed with 1 ml of 70% ethanol, followed by centrifugation at 14,000 rpm for 5 minutes at 4 °C. The supernatant was removed and the DNA pellet was air-dried at Room temperature and re-dissolved in $H_2O$ as follow: fraction A, 5 μl; fraction B, 10 μl; fraction C, 25 μl; fraction D, 20 μl; and fraction E, 10 μl. Frozen electrocompetent DH5α *E. coli* were thawed on ice and maintained on ice with the cuvettes. In ice-cooled Eppendorf tubes, 2.5 μl of DNA was mixed with 40 μl of DH5a and transferred in 0.2 cm electrode cuvette for electroporation at 2.49 kV/25 μF. Each electroporation was recovered with 1 ml LB and transferred in 12 ml Sterilin tubes, followed by incubation at 37 °C for 1 hour on a shaker at 220 rpm. For fraction A, 2 electroporations were done; 4 for fraction B, 10 for fraction C, 8 for fraction D, and only 1 for the control fraction E. Electroporations from the same fraction were pooled together in one tube, mixed and plated. For plating, 20 μl of each pool was transferred in a fresh tube containing 100 μl of LB medium: dilution 1. 10 μl of dilution 1 was transferred in another fresh tube containing 100 μl of LB: dilution 2; 10 μl of dilution 2 was transferred in another fresh tube containing 100 μl of LB: dilution 3. Dilutions 1, 2 and 3 were plated onto LB agar plate containing ampicillin at 50 μg/ml for the titration of the library. The rest of each electroporation was plated at 2 ml per LB agar plate of 24x24 cm containing ampicillin at 50 μg/ml. Plates were incubated overnight at 37°C. Colonies from cultures plated out at low dilutions were counted and the total number of colonies in each fraction calculated a shown in

Table 1.

Table 1: Titration of cDNA library. The number of independent colonies in each fraction.

| Fractions | Number of independent colonies |
|---|---|
| A | 200 |
| B | 12,800 |
| C | 355,000 |
| D | 298,000 |
| **Total library** | **666,000** |

The content of a small tube (50 μl of bacteria) was resuspended in 950 μl of LB medium, mixed, diluted 10 fold several times and plated on agar plates containing ampicillin.

After incubation overnight at 37 °C, colonies formed on agar plates were counted and the total number of bacteria in each stabilates was determined.

The quality of the library was assessed as described in Table 2.

Table 2. Analysis of randomly selected cDNA from each fraction. Plasmid DNA was isolated from 20 cDNA clones from each of the 4 fractions, cut with restriction enzymes Hind III and Xba I and analysed for presence and size of inserts, whose sequences were blasted against non-redundant GenBank and TIGR T. *parva* genome databases (http://www.tigr.org/tdb/e2k1/tpa1/.) * An additional 120 cDNAs were sequenced and found to be of *T. parva* origin.

| | Fraction A | Fraction B | Fraction C | Fraction D |
|---|---|---|---|---|
| % of colonies containing inserts | 100 | 100 | 95 | 85 |
| Size of inserts (kb) | 1 - 3 | 0.4 - 3 | 0.6 - 2 | 0.2 - 1.5 |
| % of inserts of *T. parva* origin* | 100 | 100 | 100 | 100 |

**Preparation of cDNA pools for immunoscreening with CTL**

Pools of 50 as well as pools of 10 bacteria was prepared from fractions B and C followed by plasmid minipreps purification. Aliquots of frozen bacteria in small tubes containing 50 μul of bacteria were diluted and plated on agar plates to a density of 40 - 60 colonies per 4 - 6 $cm^2$ and grown overnight at 37°C.

Colonies were scraped from the agar plate in pools of about 50 or 10 colonies and resuspended in 3 - 4 ml LB medium with Ampicillin. Bacteria suspensions were cultivated at 37°C for 3 - 4 hrs with shaking. Then 0.8 ml of each culture was mixed with 0.8 ml LB medium containing 40% glycerol and frozen as a glycerol at -80°C while the remaining was used for plasmid purification. Plasmid DNA was eluted in 200 μl of water then transferred in a well of a 96 wells microwells plate and stored at -20°C until use. For the transfection each pool of cDNAs was adjusted at 100 ng/μul.

**Strategy for resolving positive pools to identify cDNA coding for CTL target antigen**

Competent *E. coli* DH5α (200 μul) were transformed with 1 μul (100 ng) of DNA from the positive pool. The transformation was diluted and plated on agar plates to a density of 2000 colonies per 150 $cm^2$ and grown overnight at 37°C.

For a positive pool of 10, only 48 individual colonies were picked and grown overnight in 2 ml of 2x YT medium containing ampicillin. Plasmid DNA was then isolated from each bacteria culture by minipreps. Plasmid DNA were eluted in 200 μl of water and then transferred in a well of a 96 wells microwells plate and stored at -20 °C. The working concentration was adjusted at 100 ng/μl and stored at -20 °C until use. For a positive pool of 50, a three-way matrix was used as follow: 256 individual colonies were picked and seeded in 200 μl of 2x YT medium containing ampicillin in 4 flat- bottom microwells plates. This gave 64 colonies per plate. Bacteria were grown at 37 °C overnight. From each plate containing 64 cultures, 12 pools of bacteria, each containing 16 individual bacteria cultures, were prepared by pooling 50 μl of each individual bacteria culture as shown in the table 3 below. In total, 48 pools of 16 individual bacteria were generated. Glycerol was added to 20% in the each of the remaining cultures in Table 3 and stored at -70 °C until the results of the screening allowed the picking of the positive colonies.

**Use of a 3-way matrix for rapid recovery of a positive cDNA clone**

Competent *E. coli* cells (200 μul) were transformed with 1 μl (100 ng) of DNA from the positive pool of 50 cDNAs. Transformed *E. coli* were diluted and plated on agar plates to a density of 2000 colonies per 150 $cm^2$ and grown overnight at 37°C. Two hundred fifty six (approximately 5x coverage of 50 cDNA per positive pool) individual colonies were picked and seeded in 200 μl of 2x YT medium containing ampicillin in 4 flat-bottom microwells plates to have 64 single bacteria colony cultures per plate arrayed in a 8 x 8 format and numbered from 1 to 64 as tabulated below (this is one of 4 plates containing 64 *E. coli* cultures).

| | | | | | | | | | |
|---|---|---|---|---|---|---|---|---|---|
| | | 1 | 9 | 17 | 25 | 33 | 41 | 49 | 57 | | |
| | | 2 | 10 | 18 | 26 | 34 | 42 | 50 | 58 | | |
| | | 3 | 11 | 19 | 27 | 35 | 43 | 51 | 59 | | |
| | | 4 | 12 | 20 | 28 | 36 | 44 | 52 | 60 | | |
| | | 5 | 13 | 21 | 29 | 37 | 45 | 53 | 61 | | |
| | | 6 | 14 | 22 | 30 | 38 | 46 | 54 | 62 | | |
| | | 7 | 15 | 23 | 31 | 38 | 47 | 55 | 63 | | |
| | | 8 | 16 | 24 | 32 | 40 | 48 | 56 | 64 | | |

Twelve pools of 16 individual cultures were generated from the 4 plates as shown in the table below. This format was high throughput in that use of a multi-channel pipetting device, accelerated the process. The 3-way matrix was such that each individual bacteria culture was present in 3 pools. Pools of bacteria cultures were grown at 37°C for 3 hours with shaking before plasmid DNA was extracted from pooled 16 aliquots of each pool. Glycerol was added to the remaining cultures in microplates and stored at -70°C. Plasmid DNA was adjusted at 100 ng/μul and used for transfection in screening assays. A positive cDNA clone was detected in 3 pools. For example, colony #25 contained the target cDNA clone, so pools #2, 5 and 11 were positive. Plasmid DNA was then prepared from the glycerol stock of bacteria colony #25 from the 3 pools individually and re-tested and sequenced.

| | Pool 1 | Pool 2 | Pool 3 | Pool 4 | Pool 5 | Pool 6 | Pool 7 | Pool 8 | Pool 9 | Pool 10 | Pool 11 | Pool 12 |
|---|---|---|---|---|---|---|---|---|---|---|---|---|
| Colony # | 1 | 17 | 33 | 49 | 1 | 2 | 3 | 4 | 1 | 5 | 9 | 13 |
| | 2 | 18 | 34 | 50 | 5 | 6 | 7 | 8 | 2 | 6 | 10 | 14 |
| | 3 | 19 | 35 | 51 | 9 | 10 | 11 | 12 | 3 | 7 | 11 | 15 |
| | 4 | 20 | 36 | 52 | 13 | 14 | 15 | 16 | 4 | 8 | 12 | 16 |
| | 5 | 21 | 37 | 53 | 17 | 18 | 19 | 20 | 17 | 21 | 25 | 29 |
| | 6 | 22 | 38 | 54 | 21 | 22 | 23 | 24 | 18 | 22 | 26 | 30 |
| | 7 | 23 | 39 | 55 | 25 | 26 | 27 | 28 | 19 | 23 | 27 | 31 |
| | 8 | 24 | 40 | 56 | 29 | 30 | 31 | 32 | 20 | 24 | 28 | 32 |
| | 9 | 25 | 41 | 57 | 33 | 34 | 35 | 36 | 33 | 37 | 41 | 45 |
| | 10 | 26 | 42 | 58 | 37 | 38 | 39 | 40 | 34 | 38 | 42 | 46 |
| | 11 | 27 | 43 | 59 | 41 | 42 | 43 | 44 | 35 | 39 | 43 | 47 |
| | 12 | 28 | 44 | 60 | 45 | 46 | 47 | 48 | 36 | 40 | 44 | 48 |
| | 13 | 29 | 45 | 61 | 49 | 50 | 51 | 52 | 49 | 53 | 57 | 61 |
| | 14 | 30 | 46 | 62 | 53 | 54 | 55 | 56 | 50 | 54 | 58 | 62 |
| | 15 | 31 | 47 | 63 | 57 | 58 | 59 | 60 | 51 | 55 | 59 | 63 |
| | 16 | 32 | 48 | 64 | 61 | 62 | 63 | 64 | 52 | 56 | 60 | 64 |

**Table 3: A 3-way matrix for rapid resolution of positive pools. In this format each colony is represented three times.**

Table 3: A 3-way matrix for rapid resolution of positive pools. In this format each colony is represented three times.

Pools of bacteria were grown at 37 °C for 3 hours with shaking before they were used for plasmid purification by minipreps. Plasmid DNA were eluted in 200 μl of water and then transferred in a well of a 96 wells microwells plate and stored at -20 °C. The working concentration was adjusted at 100 ng/μul and stored at-20 °C until use.

### Immunisation & details of cattle

The 7 cattle used in this Example were pure bred *Bos indicus* (Boran) pure bred *Bos taurus* (Friesian) or crossbreeds. Cattle were immunised by 'infection and treatment' against the Muguga stock of *T. parva* by simultaneous inoculation of sporozoites and long-acting oxytetracycline at 20mg/kg BW (Radley et al 1975). Cryopreserved sporozoites (Stabilate # 4133) were thawed and diluted 1/20 as previously described. Animals were given a subcutaneous injection of 1ml of diluted sporozoites 2cm above the right parotid lymph node. Animals were monitored daily for changes in rectal temperature and from day 5 post challenge lymph node biopsies were taken using a 21 G needle. Giemsa stained biopsy smears were examined for the presence of schizont infected cells and scored on a scale of 1-3. Animals suffering from moderate reactions were treated with Buparvaquone (Butalex, Mallinckrodt Veterinary Ltd, UK).

### Establishment of TpM

Prior to immunisation, venous blood was collected from the eleven animals, PBMC were purified and infected *in vitro* with *T. parva* (Muguga) sporozoites (Goddeeris & Morrison, 1988).

### Generation of *T. parva* specific CD8$^+$ CTL

Venous blood from immunised animals was collected from 4 weeks post-immunisation. PBMC were prepared as described previously (Goddeeris & Morrison, 1988). PBMC were adjusted to 4x10$^6$/ml in CTL medium (RPMI-1640 without HEPES supplemented with 10% FBS (HyClone; tested for BVDV & mycoplamsa spp.), L-glutamine, 2-mercaptoethanol and antibiotics as described above) and 1ml/well added to 24 well plates (Costar, Coming, NY, USA). PBMC were co-cultured with irradiated (50Gy) autologous *T. parva* infected cells (TpM) at 2 x 10 $^5$/well and incubated for 7 days at 37°C in a 5% $CO_2$ humidified atmosphere. Cells were harvested by aspiration and dead cells removed by centrifugation over Ficoll-Paque Plus (Amersham Pharmacia Biotech, Uppsala, Sweden). After washing in CTL medium, cells were added to 24 well plates (3x10$^6$/well) and co-cultured with irradiated PBMC (filler cells) at 1 x10$^6$/well and TpM at 2 x10$^5$/well for 7 days as before. Viable cells were harvested as described above, adjusted to 2 x10$^6$/well and stimulated with 4 x10$^5$/well irradiated TpM and 2 x10$^6$/well irradiated autologous PBMC as filler cells.

**$^{51}$Chromium Release Assay**: Autologous and allogeneic TpM in log phase of growth were resuspended at 2x10$^7$/ml cytotoxicity medium (RPMI-1640 medium with 5% Fetaclone II). 100μl of the target cells were mixed with 100μl (100μCi) of $^{51}$Cr-sodium chromate and incubated for 1 hour at 37°C. Cells were washed 3 times in 7ml of cytotoxicity medium by centrifugation at 1500 rpm for 7 min at RT and resuspended at 1X10$^6$/ml. Viable cells were harvested from TpM stimulated lines 7 days post-stimulation (effector cells) and resuspended in cytotoxicity medium at 2x10$^7$/ml. Two-fold doubling dilutions of effector cells were distributed in duplicate (100μl/well) to 96-well half area (A/2) flat-bottom culture plates (Costar, Coming, NY, USA) resulting in a range of effector cell concentrations of 4x10$^6$ to 2.5x10$^{5/}$well. Target cells were added to each well containing effector cells (50μl/well) resulting in target cell ratios ranging from 80: 1 to 5:1. In separate triplicate wells target cells were added to 100μl cytotoxicity medium or 1 % Tween20 to measure spontaneous and maximal release of the label respectively. Plates were incubated for 4hours at 37°C in 5% $CO_2$ humidified atmosphere. Cells were resuspended in wells by repeated pipetting and pelleted by centrifugation at 180Xg at room temperature. 75 μl of supernatant was transferred from each well into sample vials (Milian, Geneva, Switzerland) and gamma emissions counted in a gamma counter (Micromedic MEplus, TiterTek, Huntsville, AL, USA). Results were calculated and expressed as percent cytotoxicity (= 100 x (test release - spontaneous release) / (maximum release - spontaneous release).

Evidence for MHC class I restricted lysis was assessed by the capacity of monoclonal antibodies recognising bovine MHC class I to inhibit lysis. Cells were prepared for the cytotoxicity assay as described above except that target cells were resuspended at double the density (2x10 $^6$/ml) and 25μl added first to the plate. 25μl of either cytotoxicity medium or monoclonal antibody (mAb; IL-A88 diluted 1/15 in cytotoxicity medium was added to target cells and incubated for 30min at room temperature. Serial dilutions of effector cells were then added as described above.

Viable cells from TpM stimulated cultures were harvested day 7 post-infection and CD8$^+$ T cells were isolated either by positive selection using flow cytometry (FACStar Plus, BD Biosciences, San Jose, CA, USA) or negative selection using Dynabeads (Dynal Biotech, Bromborough, UK).

Positive selection: Cells were adjusted to 2x10$^7$/ml in sterile monoclonal antibody IL-A105 (specific for bovine CD8) diluted 1/100 and incubated for 30 minutes at 4°C. Cells were washed twice in cold culture medium and resuspended at 2x10$^7$/ml in sterile goat anti-mouse polyvalent immunoglobulins conjugated to FITC (Sigma). Cells were washed twice in cold medium before being run through a FACStar plus cell-sorter and CD8$^+$FITC$^+$ cells collected.

Negative selection: Cells were adjusted to 2x10$^7$/ml in sterile mAbs IL-A12 (specific for bovine CD4) and GB21A (specific for bovine γδ TCR) diluted 1/100 and incubated for 30 minutes at 4°C. Cells were washed twice in cold PBS and resuspended 1.4x10$^7$/ml in sterile PBS containing washed sheep anti-mouse IgG Dynabeads according to the manufacturers instructions Cells and beads were rotated for 30 min at 4°C and Dynabead rosetted cells were collected by placing the sample tube in a Dynal Magnetic Particle Concentrator (MPC) for 5 min, the supernatant was removed, transferred to another sample tube and residual Dynabead rosetted cells removed by another incubation in a Dynal MPC. The supernatant was removed and cells washed twice in complete medium.

Cloning: CD8+ T cells enriched by either of the methods described above were adjusted to 30, 10, and 3 cell/ml and distributed into 96-well, round bottom culture plates containing 2 x 10$^4$ irradiated autologous TpM, 5 x 10$^3$ irradiated autologous PBMC and 5U/ml recombinant human IL-2 (HuIL-2; Sigma, Poole, UK) in a final volume of 200μl/well. Plates were incubated at 37°C in humidified incubator containing 5% $CO_2$ in air. Wells showing significant cell growth were selected for analysis of lysis of autologous TpM in an ($^{111}$In) Indium oxine release assay. The $^{111}$In release assay was performed as described above for $^{51}$Cr release assay except that targets cells were labelled by addition of 5μCi $^{111}$In/ 1 x 10$^6$ cells and incubation for 15 minutes at 37°C. Cells were washed five times with cytotoxicity medium and resuspended at 1x10$^5$/ml and added 50μl/well to 96-well V-bottom 96 well plates. 100μl cells from wells showing significant growth were transferred to 96-well V-bottom culture plates (Greiner) and centrifuged (180xg) for 5 min. Cells were resuspended 100μl/well in cytotoxicity medium and transferred to wells containing labelled target cells. Plates were centrifuged as described above to pellet cells before being incubated for 4 hours at 37°C in 5% $CO_2$ humidified atmosphere.

CTL populations that exhibited lytic activity on autologous infected cells and originated from cell dilutions that gave rise to cell growth in less than 30% of the wells, as they have high probability of being clones, were selected for expansion. The remaining cells were harvested from each well and resuspend them in culture medium (without HEPES) at a concentration estimated to be between 500 and 5000 cells/ml. 100μl of cell suspension was distributed into 96 well, round bottom culture plates. 100μl of autologous irradiated TpM at 5 x10$^4$ autologous irradiated TpM in medium containing 5U/ml HuIL-2. Between day 14 and 21 post-stimulation clones and polyclonal CTL lines were subcultured in 96 well, round bottom culture plates by co-culturing 5000 CTL/well with 25,000 autologous irradiated TpM and 5U/ml HuIL-2 in a final volume of 200μl/well.

**Establishment and maintenance of bovine skin fibroblasts**

A skin biopsy was taken aseptically from the ears of cattle and placed in a 50ml falcon tube containing Alsever's solution. In the laboratory laminar flow hood, the biopsy was placed into sterile petri dishes (Sterilin) containing 1ml of 0.25% Trypsin-EDTA. Using a sterile scalpel blade, the sample was cut into small pieces and placed into a 50 ml falcon tube containing Trypsin-EDTA. The preparation was placed in a shaking incubator for 1-1.5 hr at 37°C with gentle continuous shaking. This facilitates detachment of cells. This digestion was stopped by addition of 1ml heat-inactivated FBS. The cell suspension was centrifuged for 10 min at 200xg and the cell pellet re-suspended in 6 ml of Dulbecco's minimum essential medium, DMEM (Gibco-BRL, Paisley, UK) supplemented with 10% heat-inactivated fetal bovine serum (FBS) (Hyclone, Logan, UT), 400 IU/ml penicillin, 300 μg/ml streptomycin and 2 mM L-glutamine. This cell suspension was seeded in 5ml amounts into 25-$cm^2$ flasks and incubated at 37°C in a 5% $CO_2$ humidified atmosphere. Cultures were examined microscopically after every 4 days for growth and half the medium was replaced with fresh one until growing colonies were evident. Once positive colonies were identified they were rinsed with $Ca^{2+}$- and $Mg^{2+}$-free PBS/EDTA (0.02% EDTA) and detached by 2 min incubation at 37°C with Trypsin-EDTA solution containing 2.5 mg/ml Trypsin and 0.2 mg/ml EDTA in HBSS (Sigma). Following a wash in complete DMEM containing 10% FBS the skin fibroblasts (SF) were passaged into 25-$cm^2$ tissue culture flasks (Costar). Cells were maintained in complete DMEM containing 10% FBS, 200 IU/ml penicillin, 100 μg/ml streptomycin and 2 mM L-glutamine and passaged every 3 days at a ratio of 1:3. Cells were expanded in 75-$cm^2$ tissue culture flasks (Costar) until confluent yielding 3-4$\times10^6$ cells/flask. For consistency a liquid nitrogen cell bank was prepared with 2$\times10^6$ cells/freezing vial in 10% DMSO in FBS.

**Establishment and maintenance of bovine testicular endothelial cells**

Bovine testicular vein or pulmonary artery EC lines were established as described by Byrom and Yunker (1990) with the modifications of Mwangi et al (1998). Briefly, the vein was placed in wash buffer consisting of $Ca^{2+}$- and $Mg^{2+}$-free PBS supplemented with 400 IU/ml penicillin, 400 μg/ml streptomycin and 5μg/ml fungizone. The vessel was then slit longitudinally, washed twice before being cut into 1-$cm^2$ pieces and then placed lumen side down on a drop of collagenase (1 mg/ml) and incubated for 1 h at 37°C. The cell suspension was centrifuged for 5 min at 200$\times$g and re-suspended in 24 ml 2$\times$ complete DMEM (Gibco-BRL, Paisley, UK) supplemented with 20% heat-inactivated fetal bovine serum (FBS) (Hyclone, Logan, UT), 400 IU/ml penicillin, 300 μg/ml streptomycin, 5 mg/ml fungizone, 300 μg/ml endothelial growth supplement (Sigma, St Louis, MO) and 2 mM L-glutamine. One ml of the cell suspension was seeded in each well of a 24-well tissue culture plate (Costar, Cambridge, MA, USA). Once confluent, monolayers in each well were rinsed with $Ca^{2+}$- and $Mg^{2+}$-free PBS/EDTA (0.02% EDTA) and detached by 2 min incubation at 37°C with 0.25% trypsin/EDTA solution containing 2.5mg/ml trypsin and 0.2 mg/ml EDTA in HBSS (Sigma). Following a wash in DMEM containing 10% FBS the cells were passaged into 25-$cm^2$ tissue culture flasks (Costar). Cells were maintained in complete DMEM containing 10% FBS, 200 IU/ml penicillin, 100 μg/ml streptomycin, 2.5 mg/ml fungizone, 2 mM L-glutamine and passaged every 3 days at a ratio of 1:3. Cells were expanded in 75-$cm^2$ tissue culture flasks (Costar) until confluent yielding between 3$\times10^6$ and 4$\times10^6$ cells per flask. For consistency a liquid nitrogen cell bank was prepared with 2x $10^6$ cells per freezing vial. Cells were raised from nitrogen into 75-$cm^2$ flasks and used between the fourth and tenth passages.

**Immortalization of bovine skin fibroblasts and endothelial cells**

Cells were immortalized with the SV40 early region gene by transfection of an expression plasmid psvNeo (ATCC code 37150). The product is supplied as a freeze dried to which 300ul of 2XYT with ampicillin was added and fully resuspended. The 300ul suspension was sub-cultured into 6mls and divided into 3mls each. The cultures were incubated overnight at 37°C and then subcultured again into 50mls of 2XYT with ampicillin overnight at 37°C. Maxiprep of psvNeo plasmid were made for use in immortalizations. Plasmid DNA for transfection was standardized to 2mg/ml.

The DNA mix was prepared by mixing 5μl DNA with 495μl of DMEM with antibiotics but no serum. A working dilution of Fugene 6 transfection reagent (Roche) was prepared by mixing 15μl of Fugene with 485μl of DMEM. Mixing was done using the 2ml sterile non-pyrogenic, DNAse and RNAse free cryopreservation tubes. The diluted Fugene was added to the diluted DNA drop wise with constant tapping at the end of the tube. The Fugene-DNA complex was allowed to form at room temperature for 30 min.

Cells were cultured in 6-well plates (Costar) at a density of 2 x $10^5$ cells per well and grown to confluence overnight at 37°C, 5% $CO_2$ in a humidified incubator. Culture medium was removed completely from the monolayers to be transfected. The Fugene-DNA complex was then added gently onto the monolayer. Plates were incubated at 37°C,

5% $CO_2$ in a humidified atmosphere for 3-4 hours. The transfection complex was removed; fresh DMEM medium containing 10% fetal calf serum was added and then cultured for a further 72h. Cells from each well were rinsed with PBS/EDTA, detached with Trypsin/EDTA, washed and re-suspended in complete DMEM and sub-cultured into one T-25 flask (Costar). After incubation for 2-3h, normal DMEM medium was removed and replaced with a selection DMEM medium containing 10% FCS and 0.5μg/ml of G418 (2.5μg/ml G418 for endothelial cells) and incubated further. Upon observation of high death rate of cells, half the medium was replaced with fresh selection medium until growing colonies were evident. This process took 3-4 weeks depending on the cell lines. Positive colonies were the sub-cultured into 24 well plates and then to T-25 flasks. Immortalization was confirmed by checking for expression of large T-antigen using an anti-SV40 antibody conjugated to HRP. Further expansion and maintenance of the cells was carried out using complete DMEM.

**Transfection of COS-7 cells and immortalised skin fibroblasts with schizont cDNA library**

COS-7 cells and iSF were maintained in T75 and T150 TC flasks with DMEM supplemented with 10% FCS, 2mM L-glutamine and antibiotics (TC medium) as described above. Cells are split 1:4 every 3 days. The day prior to transfection; cells were harvested by the removal of medium, washing in PBS and incubation in 0.25% Trypsin-EDTA for 5 min at 37°C. Once cells had detached TC medium was added and cells removed. Cells were washed by centrifugation at 1200 rpm for 10min and resuspended in TC medium. A viable cell count taken, density adjusted to $2.0x10^5$/ml, cells dispensed, 100μl/well, into 96 well flat-bottom TC plates and incubated for overnight at 37°C in a $CO_2$ (5%) humidified incubator.

DNA was prepared for either single transfections of SF or double transfections (co-transfection of schizont cDNA and BoLA class I cDNA) of COS cells. 6μl of schizont cDNA and 6μl of BoLA class I cDNA (for co-transfection) at 50ng/μl in $dH_2O$ were added to 150μl unsupplemented DMEM in wells of a 96-well round-bottom plate. FuGENE 6 transfection reagent was pre-warmed to 37°C. 0.9μl or 0.45μl FuGENE 6 was added to each well for double and single transfections respectively. The well contents mixed by shaking on a Dynatech Varishaker for 1 min and incubated at RT for 20 min. The medium from the 96 well plates containing adherent COS cells and SF was removed and each transfection complex added to triplicate wells (50μl/well). The cells were then incubated for 4 hours at 37°C in a $CO_2$ (5%) humidified incubator. The transfection complex was removed and replaced with 200μl/well DMEM supplemented as described above and incubate for 24 or 48 hours at 37°C in a $CO_2$ (5%) humidified incubator.

**Detection of CTL recognition of transfected SF and COS-7 cells by IFN-gammayELISpot**

24 hours post-transfection, medium was removed from wells containing transfected cells, cells were washed cells with PBS (200μl/well) and detached by the addition of 100μl/well Trypsin-EDTA as described above. Once the cells had detached the contents of each well were transferred to a 96 well round-bottom plates containing 100μl/well cold RPMI with no HEPES supplemented with 10% FCS (CTL medium). The cells were centrifuged at 1200 rpm for 3 min; supernatant removed and resuspended 50μl/well in CTL medium Schizont specific CTL, generated and maintained as described above, were harvested 7-14 days post-stimulation, transferred to polycarbonate tubes, pelleted at 1200 rpm for 10min and resuspended at $2x10^5$/ml in CTL medium supplemented with 5U/ml HuIL-2 (Sigma).

ELISpot plates (Millipore corporation, Bedford, MA, USA) were coated 50μl/well with 2μg/ml of murine anti-bovine IFN-γ mAb (CC302; Serotec, UK) and incubated overnight at 4°C. Wells were washed twice with unsupplemented RPMI-1640 and blocked 200μl/well with RPMI-1640 supplemented with 10% FBS by incubating at 37°C for 2 hours. The blocking medium was removed and replaced with 50μl/well CTL and 100μl/well transfected cells. As a positive control, irradiated TpM are serially diluted in COS cells or SF with each at a density of $4x10^5$/ml. and populations containing 32, 16, 8, 4, 2, 1 % TpM are added 50μl/well to wells containing CTL. Plates were incubated in a humidified incubator at 37°C for 20 hours. After incubation, the contents of wells were removed and wells washed four times with sterile distilled water supplemented with 0.05% Tween 20 per well and the plate shaken on a shaker for 30 seconds between washes. The process was repeated an additional four times, using PBS supplemented with Tween 20 (PBS-T). Wells were then incubated with 100μl/well rabbit anti-bovine IFN-γ antisera diluted 1/1500 in PBS-T supplemented with 0.2% BSA (PBS/BSA) for 1 hour at room temperature. Wells were washed 4 times with PBS-T before being incubated for 1 hour at room temperature with 100μl/well murine monoclonal anti-rabbit IgG conjugated to alkaline phosphatase (Sigma) diluted 1/2000 in PBS-TBSA. Sigma Fast BCIP/NBT buffered substrate (Sigma) was by dissolving 1 tablet/10ml $dH_2O$ and passing it through a 0.2μm filter. Plates were washed six times as described above with PBS-T, 100μl/well BCIP/NBT substrate added and plates incubated for 10minutes at room temperature in the dark. The substrate was then removed, wells washed with copious amounts of $H_2O$ and plates air-dried at room temperature in the dark. Plates were finally read on an automated ELISpot reader (AID Diagnostica, Strasberg, Germany).

**Bioassay for CTL activation based on rapid induction of class II MHC expression by constitutively negative bovine endothelial cells**

Endothelial cells were detached from confluent 75-$cm^2$ tissue culture flasks by treatment with trypsin/EDTA as described. $2.5\times10^4$ cells were seeded into each well of a 48-well plate in 1 ml of complete DMEM and incubated overnight. Cell-free test supernatants derived from either co-culture of *T. parva*-specific $CD8^+$ T cell lines and SF transfected with test genes or recombinant bovine IFN-γ (rBoIFN-γ, the kind gift of Ciba-Geigy, Basel, Switzerland) were dispensed in duplicate wells in a final volume of 160 μl per well. Following 48 h at 37oC, culture supernatants were discarded and the monolayers were washed 3X in FACS medium (RPMI 1640 supplemented with 2%-globulin-free horse serum and 0.1% sodium azide). Endothelial cells ere labelled on ice for 30 mini with 100 ul of an antibody cocktail comprising equal volumes of bovine class II MHC-specific monoclonal antibodies (mAbs) J11, R1, and IL-A21 (each at a 1/500 dilution of ascitic fluid). After 3 washed in FACS medium, 100 ul of FITC-conjugated goat anti-mouse Ig (Sigma) diluted 1/200 in FACS medium were added. After incubation at 4°C for 30 min and a further three washes in FACS medium, cells were detached as described previously and cell surface class II MHC expression determined by flow cytometry, using a FACScan (Becton-Dickinson, Sunnyvale, CA, USA). The percentage of class II MHC-expressing cells was determined by comparison with unstimulated EC labeled in an identical manner.

*T. parva*-specific $CD8^+$ CTL lines were generated and maintained using methods initially described. Test supernatants were collected from 96-well flat-bottomed microtitre plates (Costar) 48 hours after restimulation of resting T cell lines ($2\times10^4$ per well) with $4\times10^5$ COS-7 cells co-transfected with the KN104 gene and test gene(s) or confluent autologous SF transfected with the test gene(s) in a final volume of 200 μl for 24 h. These tests were set-up at least in duplicates. Where indicated, class I MHC was blocked using a specific antibody (IL-A88) to check for MHC class I restriction of the CTL lines. Additional negative control supernatants were derived from co-culture of CTL with untransfected immortalized SF or COS-7 cells. Positive control supernatants were obtained from co-culture of CTL with varying proportions of irradiated autologous TpM.

**Detection of CTL lysis of transfected iSF and COS-7 cells**

Autologous iSF or COS-7 cells were seeded in 6-well plates (Costar) at a density of $2.5\text{x}10^5$ /well and incubated for 2 hours at 37°C to allow cells to adhere. For single transfections of iSF, 2μg of test or control cDNA was added to 1ml unsupplemented DMEM containing 3μl FuGENE 6 transfection reagent and incubated for 40min. For COS-7 cells, 2μg of test or control cDNA-was added with 2μg of BoLA class I cDNA to 1ml DMEM containing 6μul FuGENE. 1ml of DNA/Fugene complex was added per well of adherent COS-7 or iSF. Plates were incubated for 4 hours at 37°C, the transfection complex was removed and replaced with 2ml/well of complete DMEM and the plates incubated for a further 20 hours at 37°C. Transfected cells were harvested by removal of medium, washing in PBS, detachment by Trypsin-EDTA and washing in complete DMEM. Transfected cells and TpM were labelled with $^{51}$Chromium and the ability of schizont-specific CTL lines (day 6-8 post-stimulation) to lyse these targets.was assessed as described above.

**Use of Exonuclease III digestion to map epitope-encoding regions from Tp1**

The recombinant Tp1 plasmid DNA was cut with restriction enzyme Apa I to generate Exonuclease III resistant 3'-protruding termini. The linearized plasmid DNA was then purified with phenol-chloroform before digestion with the restriction enzyme Xho I that will cut at the single and unique vector cloning site Xho I located between Not I and Apa I sites (NB: the Tp1 cDNA insert does not contain neither Apa I nor Xho I sites). Digestion of the DNA with Xho I generated an exonuclease III sensitive recessed 3'-terminus (only at the 3'side of the cDNA insert). The linearized plasmid DNA was purified as described previously, 100 μg of DNA was digested at 37 °C with 1 unit of Exonuclease III in 20 μul reactions at varying times (from 0 to 30 minutes). Reactions were stopped by 10 minutes incubation at 75 °C, DNA was ethanol-precipitated, washed with 70% ethanol, air-dried and re-dissolved in 20 μul of 1x Mung Bean nuclease. Five units of Mung Bean nuclease were added to each reaction, mixed and incubated at 30 °C for 1 hour to remove single-stranded extensions and create ligatable blunt ends. The DNA was again purified with phenol-chloroform, precipitated with ethanol, washed with 70% ethanol, air-dried and re-dissolved in 10μl of sterile distilled water. For re-ligation, 5 μul of DNA was mixed with 1μul of 10x T4 ligation buffer, 3 μl of water and 1 μl of T4 DNA ligase, and incubated at 16 °C overnight. Competent *E. coli* DH5α was transformed with 2 μl of the ligation and plated on agar plates as previously described. Plasmid DNA was isolated from single bacteria colonies as described and the inserts were excised by double digestion with BamH I and Bbs I (an isoschizomer of Bbv II). Plasmid clones with different cDNA inserts were selected for IFN-γ ELISpot screening. Oligonucleotide primers were generated and used in PCR to clone small overlapping fragments of a 600 bp region containing the CTGAMMA epitope, into the mammalian expression plasmid vector pTargeT (Promega). Clones generated were then analysed by IFN-GAMMAγ ELISpot.

**Identification of Tp1 CTL epitopes with synthetic peptide libraries**

Peptide libraries (Cleaved PepSets; Mimotopes, Clayton, Australia) were generated for the 66 amino acid portion of Tp1 shown to contain the HD6 restricted CTL epitope. The PepSet libraries contained every 12mer, 11mer, 10mer and 9mer offset by 2 amino acids from the protein sequences. However, the peptides were prepared by truncations of the 12mers at the N-terminus and were supplied lyophilised with each tube containing a nominal 12mer and the 9, 10, 11mer truncations with the same C-terminus. Peptides were dissolved in 400$\mu$ul 50% (v/v) DNA synthesis grade acetonitrile/water (Applied Biosystems, Warrington, UK). To aid the dissolution, tubes were held in a sonicator water bath for 2 x 10 min. Peptides were aliquoted into labelled cryopreservation tubes (Greiner) and stored at -20°C. For screening with CTL, peptides were prepared at 10$\mu$g/ml in complete RPMI-1640 and 10$\mu$ul added to triplicate wells of an ELISpot plate, coated, washed and blocked as described above. Autologous iSF or P815 cells stably expressing the BoLA class I HD6 (P815-HD6) or JSP-1 (P815-JSP-1) were adjusted to a density of 4x$10^5$/ml and 50$\mu$ul added to wells containing peptides. The plates were incubated at 37°C for 1 hour before CTL, prepared as described above for screening transfectants, were added 50$\mu$l/well. Plates were incubated for 20 hours at 37°C and then developed as described above. Based on the results of the screening with the Tp1 PepSet, individual 9, 10 and 11mer peptides were synthesised in order to define the CTL epitopes. Peptides were prepared and screened using the IFN-$\gamma$ ELISpot as described above.

Peptide-pulsed iSF and BoLA class I P815 transfectants were prepared as targets for $^{51}$Chromium release assays by incubating 2x$10^6$ iSF or P815 cells overnight in T25 tissue culture flasks (Costar) with Tp1 peptides diluted to 1$\mu$g/ml in complete DMEM. Cells were harvested, labelled and assayed as described above.

**Detection of Tp1 specific *ex vivo* CD8$^+$T cell responses from immune cattle after challenge with *T. parva* sporozoites**

Bull BV115, whose schizont specific CTL lines had been shown to recognise Tp1, was challenged with a lethal dose of *T. parva* (Muguga) sporozoites. Cryopreserved sporozoites (Stabilate # 4133) were thawed and diluted 1/20 as previously described. Animals were challenged by subcutaneous injection of 1ml of diluted sporozoites 2cm above the right parotid lymph node. Animals were monitored daily for changes in rectal temperature and from day 5 post challenge lymph node biopsies were taken using a 21 G needle. Giemsa stained biopsy smears were examined for the presence of schizont infected cells and scored on a scale of 1-3. Animals were bled on day 0 and daily from day 6 to 13 and PBMC were isolated as described above. CD8$^+$ T cells and CD14$^+$ monocytes were purified from PBMC by MACS magnetic cell sorting according to the manufacturers instructions (Miltenyi Biotec, Gergisch Gladbach, Germany). CD8$^+$ T cells were sorted indirectly using a monoclonal antibody specific for bovine CD8 (IL-A105) followed by incubation with goat anti-mouse IgG microbeads (Miltenyi Biotec). CD14 monocytes were sorted directly by incubation with CD14 microbeads (Miltenyi Biotec). PBMC and CD8$^+$ T cells were added to wells (2.5x$10^5$/well) of coated/blocked ELISpot plates and stimulated with autologous TpM (2.5x$10^4$/ well) or Tp1 peptides (1$\mu$g/ml final concentration). Purified monocytes were additionally added (2.5x$10^4$/well) to wells containing peptide and CD8$^+$ T cells. ELISpot plates were incubated and developed as described above.

In order to recall Tp1 peptide specific CTL responses, PBMC were stimulated with autologous TpM 14 days post-challenge as described above. Viable cells were harvested 7 days post-stimulation and lytic activity against TpM and Tp1 peptide pulsed uninfected T cell blasts assessed as described above.

**Results of screening for CTL target antigens**

**Optimisation of INF-gamma$\gamma$ ELISpot for the recognition of target antigens by schizont specific CTL**

The ability of the IFN-GAMMA-$\gamma$ ELISpot to detect the recognition of TpM by CTL was first assessed using a CD8$^+$ polyclonal CTL line from animal F100. Fourteen days post-stimulation, CTL were added (5000/well) to coated/blocked ELISpot wells containing 25,000 irradiated autologous schizont and the formation of IFN-GAMMA-$\gamma$ spots assessed after a 20-hour incubation. CO-culture of CTL with TpM resulted in significant release of IFN-GAMMA-gamma. Pre-incubating the CTL for 30 min with a mAb against BoLA class I completely inhibited the IFN-GAMMA-$\gamma$ response whilst mAbs against MHC class II or the irrelevant CD21 antigen had no effect. Significantly, there was almost no spontaneous release of IFN-GAMMA-$\gamma$ from CTL cultured without TpM. This TpM line did not constitutively express TpM and no IFN-GAMMA-$\gamma$ spots could be attributed to the TpM.

In an attempt to replicate the transient transfection situation, where CTL would be co-cultured with COS-7 or iSF of which only a small proportion of cells would be expressing the target antigen, TpM were titrated in COS-7 or iSF and co-cultured with CTL in IFN-GAMMA-$\gamma$ ELISpot plates. The stimulator population was fixed at an input of 40,000/well, with only the proportions of TpM and COS-7/SF varying. This cell input was adopted since it was thought to mirror the numbers of APC that would be co-cultured with CTL after transient transfection. Initially different CTL inputs were tried against TpM titrations with the aim of identifying the minimum CTL input required to detect significant responses to 1-3% TpM. A CTL input of 10,000/well was determined to be optimal since it could elicit significant responses to less than 1% TpM and it was practically feasible to raise such CTL numbers for screening experiments. Further titration experiments were performed with CTL clones from F100 to confirm that with these CTL and APC inputs the IFN-GAMMA-γ ELISpot was meeting or exceeding the desired sensitivity level. With all the clones tested the IFN-GAMMA-γthe ELISpot could still detect recognition of target cells when they constituted only 0.1 % of the total cell population. The ELISpot assay worked well with little background noise and met the sensitivity requirements when TpM were titrated in COS-7 cells but it was important to determine that the assay performed as well when the TpM were titrated in autologous SF. Neither primary nor iSF significantly affected the background levels or the sensitivity of the ELISpot assay.

In advance of the initiation of screening for CTL target antigens by the transient transfection of COS-7 cells and iSF, the efficiencies of COS-7 and iSF transfection in 96 well TC plates was assessed using GFP as a reporter gene. Whilst there was considerable variation in transfection efficiencies between cell lines and between experiments, COS-7 consistently transfected better than iSF with efficiencies varying from 5-50% whereas for iSF transfection efficiency ranged between 0.5 - 20%. The transfection efficiency of iSF was assessed to be good enough to allow the presentation and identification of transfected schizont cDNA.

**Evaluation of IFN-GAMMA-γ Bioassay as a complementary read-out system for CTL recognition of target antigens**

Bovine vascular EC were stained for surface class II MHC expression following culture for 48 h in the presence of media containing recombinant IFN-GAMMA-γ. The sensitivity to rBoIFN-gammaγ was determined and found to be between 100 and 10 pg/ml. Comparisons were made between IFN-GAMMA-γ bioassay and ELISpot by co-culturing *T.parva-specific* CTL with fixed number of autologous iSF containing varying proportions of target autologous TpM and skin fibroblasts. There was a good correlation between the two assays. Both assays detected production of IFN-GAMMA-γ in co-cultures, containing as low as 1% TpM.

**Identification of CTL target antigens**

**Tp1 Identification**

A $CD8^+$ polyclonal T cell line from Friesian bull BV115 was generated and maintained using an HD6 expressing 4229 TpM (4229 TpM). This CTL line, termed BV115 (4229 TpM) CD8+ polyclonal CTL line, lysed both autologous TpM and 4229 TpM showing that the line included schizont specific HD6 restricted CTL.

This CTL line was used to screen the 1000 pools of 50 schizont cDNA (B & C series) following co-transfection of COS-7 cells with schizont cDNA pools and a pcDNA3 construct expressing the full length cDNA for HD6. The CTL line responded to 10 cDNA pools and the results of screening pools B1 - B200 are shown in Figure 2. Six of the ten putative positive pools were subjected to resolution and by way of illustration the results of screening the 48 resolved pools from the putative positive pool B162 are shown in Figure 3. The 3-way matrix was decoded and the single cDNA were screened, the results from screening the single cDNA originating from pool B162 are shown in Figure 4. Of the five single cDNA only three were recognised by the CTL line, all five cDNA were sequenced and found that the positive cDNA were identical whilst the negative cDNA were unrelated. It is likely that the negative cDNA were contaminated with the positive cDNA during propagation of the *E. coli* in microtitre plates since they were always grown in wells adjacent to wells containing the positive cDNA.

The response to the other resloved cDNA pools were due to this same cDNA, which was termed Tp1. Specific PCR primers were designed and the remaining putative positive pools were found to be positive for Tp1 cDNA. HD6 restriction recognition of Tp1 was assessed by co-transfection of Tp1 with HD6 or another BoLA class I cDNA, KN104 or transfection of Tp1 alone, Figure 5 shows that only when COS-7 cells were co-transfected with HD6 and Tp1 did the CTL secrete IFN-γ. The ability of autologous immortalised skin fibroblasts to present Tp1 was assessed by transfection and compared to co-transfected COS-7 cells. Figure 6 shows that immortalised skin fibroblasts presented Tp1 to the CTL although the response was less than induced by the COS-7 cells, probably due to the reduced transfection efficiency of skin fibroblasts. Recognition of Tp1 transfected COS-7 cells and immortalised skin fibroblasts was also confirmed by the use of the IFN-γ bioassay (Figure 7).

The ability of BV115 (4229 TpM) CD8 polyclonal CTL line to lyse Tp1 transfected targets was then assessed using a $^{51}$Chromium release assay. COS-7 cells co-transfected with HD6 and Tp1, and Tp1 transfected autologous iSF were labelled and used as target cells. CTL efficiently lysed 4229 TpM and Tp1 transfected cells (Figure 8). The lysis of Tp1 expressing targets was inhibited by the presence of a blocking monoclonal antibody against BoLA class I. The CTL did not lyse cells transfected with the irrelevant *T. parva* antigen PIM.

**Mapping of the HD6 restricted Tp1, Tp4, Tp5, Tp7, and Tp8 CTL epitopes**

Following Exonuclease III digestion, six plasmids containing Tp1 inserts of differing sizes (Tp1 Del1 to De16; denoted as SEQ ID NO: 6, 7, 32,33, 34, and 35 in Figure 9.), were co-transfected with HD6 into COS-7 cells and recognition of plasmid clones by BV115 (4229 TpM) CD8 polyclonal CTL line assessed by IFN-GAMMA-γ ELISpot. Positive ELISpot responses were observed against clones Tp1 Del1 (SEQ ID. NO: 6) and Tp1 Del2 (SEQ ID NO:7). Both ELISpot positive and negative plasmid clones were sequenced and compared. Figure 9 shows a comparison of the predicted amino acid sequence of Tp1 (SEQ ID NO;1) with the deleted clones (SEQ ID NO: SEQ ID NO: 6, 7, 32, 33, 34,and 35). The sequence comparison located the HD6 restricted CTL epitope within a 101 amino acid fragment of Tp1.

Oligonucleotide primers were generated and used in PCR to clone a 600bp fragment of Tp1, overlapping the epitope-encoding region (Tp1.6; SEQ ID NO: 40). IFN-GAMMAγ ELISpot confirmed CTL recognition of this portion of Tp1 and small overlapping fragments (Tp1.1-Tp1.5; SEQ ID NO: 35 through SEQ ID NO:40) of the 600 bp region were cloned into the mammalian expression plasmid vector pTargeT. Clones were then analysed by ELISpot and the CTL epitope was further narrowed down to a 200 bp DNA fragment corresponding to 66 amino acids (Figure 10). A cleaved PepSet library of 28 overlapping peptides each were synthesised to encompass the 66 amino acids encoded by the Tp1.2 insert (Mimotopes). Recognition of Tp1 peptides was assessed by IFN-GAMMAγ ELISpot, using autologous immortalised skin fibroblasts or murine mastocytoma P815 cells (ATCC #TIB-64, ATCC, Manassas, VA USA) stably expressing HD6 as antigen-presenting cells. Figure 11 shows the results of screening the cleaved Tp1 Pepset with BV115 (4229 TpM) CD8 polyclonal CTL line and BV115 CTL clones. The recognition of peptides #10 and #11 suggested that the epitope fell within the region RCVGYPKVKEEMLE (extending from amino acid #212 to amino acid #225 of SEQ ID NO: 1). All possible 9, 10 and 11mers were then synthesised for this sequence and screened against the polyclonal line and clones. Figure 12 shows the responses of two BV115 CTL clones to individual Tp1 peptides. Both clones responded to peptide #24, the 11mer VGYPKVKEEML (SEQ ID NO:9 and corresponding nucleotide sequence SEQ ID NO:23), suggesting that this was the minimal length HD6 restricted epitope of Tp1. This data was supported by $^{51}$Chromium release assays that demonstrated significant CTL lysis of Tp1 peptide #24 pulsed iSF or P815-HD6.

Additional experiments, using a set of 4 overlapping peptides, including the 11-mer representing SEQ ID NO: 9; a 10-mer that was missing the last right-hand amino acid (amino acid number 224 of SEQ ID NO:1); a 10-mer, missing the first left-hand amino acid of SEQ ID NO:14 (amino acid number 214 of SEQ ID NO1); and an 11-mer that included the additional left-hand amino acid (amino acid number 213 of SEQ ID NO:1) but missing that last right-hand amino acid of SEQ ID NO:14 (amino acid number 224 of SEQ ID NO:1), indicated that the minimal length of the CTL-stimulatory epitope is represented by SEQ ID NO:14.

The same types of experiments were conducted with subsets of the antigens Tp4 (SEQ ID NO:2), Tp5 (SEQ ID NO:3), Tp7 (SEQ ID NO:4), and Tp8 (SEQ ID NO:5). Within Tp4, the minimum epitope is the sequence identified by the amino acid SEQ ID NO: 14. This was established by testing a set of 8 peptides that included 9-mer polypeptide, SEQ ID NO: 14 (amino acid numbers 338 to 346 in SEQ ID NO:2), an 8-mer polypeptide representing amino acids numbers 339 to 346 of SEQ ID NO:2, an 8-mer polypeptide representing the amino acid numbers 441 to 348 of SEQ ID NO:2;an 8-mer polypeptide representing the a peptide of amino acid numbers 338 to 345 of SEQ ID NO:2; a 9-mer polypeptide of amino acids representing amino acid numbers 339 to 348 of SEQ ID NO:2; a 10-mer polypeptide of amino acids representing amino acid numbers 338 to 347 of SEQ ID NO:2, a 9-mer polypeptide of amino acids representing amino acid numbers 339 to 347 of SEQ ID NO:2, and an 11-mer polypeptide of amino acids numbers 338 to 348 of SEQ ID NO:2. (The corresponding nucleotide sequence for SEQ ID NO:14 was determined to be the sequence represented in SEQ ID NO:28). Experiments determining the minimal length of the Tp5 that would stimulate CTLS were designed to test a set of 5 overlapping peptides in the region of SEQ ID NO: 15. These 5 peptides included the 11-mer polypeptide of SEQ ID NO:15 (amino acids numbers 8 to 95 of SEQ ID NO: 3); an 11-mer polypeptide of amino acids numbers 85 to 95 of SEQ ID NO:3 ; an 11-mer polypeptide of amino acid numbers 87 to 97 of SEQ ID NO:3; and an 8-mer polypeptide of amino acids numbers 88 to 95 of SEQ ID NO:3. The minimum Tp7 epitope is represented by the polypeptide of SEQ ID NO: 16 (and corresponding nucleotide sequence of SEQ ID NO:30). This was determined with an overlapping set of 7 polypeptides including SEQ ID NO:16 (amino acid numbers 206 to 214 in SEQ ID NO:4); an 11-mer polypeptide (amino acid numbers 204 to 214 of SEQ ID NO:4); a 10-mer polypeptide (amino acid numbers 205 to 213 of SEQ ID NO:4); a 9-mer polypeptide (amino acid number 204 to 212 of SEQ ID NO:4); an 8-mer polypeptide (amino acid numbers 204 to 211 of SEQ ID NO:4); and an 8-mer poly peptide (amino acid numbers 207 to 214 of SEQ ID NO;4). Within Tp8, the minimum epitope is represented by the polypeptide of SEQ ID NO: 17 (and corresponding nucleotide sequence of SEQ ID NO:30). This was determined by using a set of 4 overlapping polypeptides including SE ID NO:17 (amino acid numbers 269 to 277 with SEQ ID NO:5); a 9-mer polypeptide (amino acid numbers 270 to 278 of SEQ ID NO:5); a 10-mer polypeptide (amino acid numbers 268 to 277 of SEQ ID NO:5), and an 11-mer polypeptide (amino acid numbers 268 to 278 of SEQ ID NO:5).

Table 4

| *T. parva* **antigenic polypeptides** | | **CTL (antigen)epitopes** |
|---|---|---|
| | aa sequence | DNA sequence |
| Tp1 (SEQ ID NO: 1) | SEQ. ID. NO:9 VGYPKVKEEML | SEQ. ID. NO:23 GTAGGGTATCCAAAGGTTAAAGAAGAAATGCTA |
| Tp4 (SEQ ID NO: 2) | SEQ. ID. NO:14 TGASIQTTL | SEQ. ID. NO:28 ACTGGTGCTTCTATTCAAACCACTCTC |
| Tp5 (SEQ ID NO: 3) | SEQ. ID. NO:15 SKADVIAKY | SEQ. ID. NO:29 AGCAAGGCTGACGTGATCGCAAAGTAC |
| Tp7 (SEQ ID NO: 4) | SEQ. ID. NO:16 EFISFPISL | SEQ. ID. NO:30 GAGTTCATTTCATTCCCAATCTCGCTC |
| Tp8 (SEQ ID NO: 5) | SEQ. ID. NO:17 -CGAELNHFL | SEQ. ID. NO:31 TGCGGTGCTGAATTGAACCACTTCTTG |

Tp1 was cloned from the Marikebuni stock of *T. parva.* The deduced amino acid sequence was compared to that of Tp1 from the Muguga stock and found to be 95.8% identical (Figure 13). The ability of CTL to recognise the Marikabuni and Muguga Tp1 was assessed using the ELISpot. BV115 (4229 TpM) CD8 polyclonal CTL line only recognised the Muguga Tp1. This is explained by a double amino acid substitution at the C-terminus of the predicted HD6 restricted epitope.

**Evidence that Tp1 is recognised by $CD8^+$T cells from an immune resolving a challenge infection**

Bull BV115, whose CTL had recognised Tp1, was challenged with a lethal dose of *T. parva* (Muguga) sporozoites and the response of purified CD8+ T cells to the HD6 restricted Tp1 eptiope or control Tp1 peptide which did not contain any previously identified epitopes were measured longitudinally. The animal was solidly resistant to challenge with no fever or detectable parasitosis. From day 8 post-challenge, CD8+ T cells responded specifically to the Tp1 epitope and were sustained over the period of observation (Fig 14). The kinetics of this response is comparable to that previously described for schizont-specific CTL precursors in blood following challenge of immune cattle with *T. parva* sporozoites (McKeever *et al* 1994). Attempts were made to detect Tp1 and TpM specific lytic responses directly in peripheral blood post-challenge but these failed. An experiment was instigated to first expand schizont specific CTL numbers by a single *in vitro* stimulation with TpM and then to assess Tp1 specific lysis. Stimulated cells exhibited high cytotoxic activity against both TpM and T cell blasts pulsed with the Tp1 epitope. These data suggest that a component of the protective $CD8^+$ T cell response is Tp1-specific highlighting the potential of this antigen as a vaccine candidate.

**Identification of other CTL target antigens**

Four other CTL target antigens, namely Tp4, Tp5, Tp7 and Tp8, have been identified through screening and resolution of the schizont cDNA library using either the ELISpot assay or bioassay. These assays utilised CTLs generated from five cattle representing four class I MHC genotypes. Cells transfected with these four antigens were recognised and lysed specifically by CTLs. Further assays have been performed to define the CTL epitope in Tp5. Experiments have been carried out which demonstrate that Tp5 is recognised by *ex vivo* $CD8^+$ T cells obtained from an immune animal (BV050). These results are summarised in Table 5.

Table 5: A summary of results showing the identification of further CTL target antigens.

| **Antigen** | **CTL** | **Assay for ID** | **Lysis** | **Epitope** | **Ex vivo response** |
|---|---|---|---|---|---|
| Tp4 | BV057<br>BX063<br>BX065 | ELISpot Bioassay | ✓ | Not done | Not done |
| Tp5 | BV050 | ELISpot | ✓ | ✓ | ✓ |
| Tp7 | BW012 | ELISpot | ✓ | Not done | Not done |

(continued)

| Antigen | CTL | Assay for ID | Lysis | Epitope | Ex vivo response |
|---|---|---|---|---|---|
| Tp8 | BX063<br>BX065 | ELISpot | ✓ | Not done | Not done |

**Nucleotide and deduced amino acid sequences and putative identity of the 5 candidate antigens**

Following conformation of the candidates as CTL target antigens, the individual cDNA were sequenced and confirmed to be of *T. parva* origin by interrogating the *T. parva* genome sequence database. BLAST searches (Altschul *et al* 1990) of DNA and protein databases were performed and homoloques of some of the antigens identified. SignalP (Nielsen *et al* 1997) and Tmpred (http://www.ch.embnet.org/software/TMPRED_form.html) analyses were conducted to predict the presence of a signal peptide and transmembrane domain. SEQ ID NO: 17-22 indicate the DNA and SEQ ID NO: 1-5, the deduced protein sequences of the five candidate antigens.

**Protein Expression**

**Construction of Plasmids**

The reading frames of *T. parva* candidate antigen genes were amplified by PCR using Taq polymerase (Promega, Madison, WI USA 53711) from the original full-length genes in their respective plasmids in pcDNA3. Both the forward and reverse primers contained restriction enzyme sites (Table 6). The PCR products were digested with the respective restriction enzymes, and ligated into bacterial His-tag expression vectors, pQE30 (Qiagen, 28159 Avenue Stanford, Valencia, CA91355) or PET28 (Novagen, Madison, WI 53719 USA). The plasmids were transformed into *E. coli* strain DH5α (Life Technologies, Carlsbad, CA 92008, USA). All the plasmids were sequenced to ensure that they harbored no substitutions compared to the original genes. Purified plasmids were then used to transform competent BL21 DE3 bacterial cells.

Table 6: Primers, restriction sites and vectors used for the cloning of *T. parva* antigens for protein production.

**Expression of the proteins**

| Gene (Plasmid) | Forward Primer (Restriction Site) | Reverse Primer (Restriction Site) | Expression Vector | PCR Product (s) (kb) |
|---|---|---|---|---|
| Tp1 (pcDNA3) | GGATCCCCGGAAAAAGAAG AGGAACTC (*Bam*HI)<br>(SEQ ID NO: 33) | CTGCAGTTAAT TTTTGAGGTAAATTTTG (*Pst*I)<br>(SEQ ID NO: 37) | pQE30 | 1.5 |
| Tp4 (pcDNA3) | AATGTAGTTTTATCTAAATTGCC A (*Bam*HI)<br>(SEQ ID NO: 34) | GAGGAGATAAG TTGAGAGCAACATC (*Sal*I)<br>(SEQ ID NO: 38)<br>(SEQ ID NO: 38) | pQE30 | 0.4 |
| Tp5 (pcDNA3) | GGATCCGAAATGGCGAAAAATA AAGGCAAAGGA (*Bam*HI)<br>(SEQ ID NO: 35) | CTGCAGTTATAAATCAT CGATATCGAAATCT (*Pst*I)<br>(SEQ ID NO: 39) | pQE30 | 0.6 |
| Tp7 (pcDNA3) | GCCAAGAATTCGATGACATCAA AGGACGAG (gene internal *Bam*HI site)<br>(SEQ ID NO: 36) | GGCGCGGCCGCGTCAA CTTCCTCCATTTTG (*Not*I, *Xho*I)<br>(SEQ ID NO: 40) | pET28b | 2.0, 1.1 |

Single BL21 DE3 bacterial colonies bearing the recombinant plasmids were isolated and cultured in 2XYT (formula) at 37°C to an $OD_{600}$ of 0.6, and protein expression induced by addition of IPTG to a final concentration of 1mM, and further cultured for 4 h. The cells were harvested by centrifugation at 4000 g for 20 min. Recombinant proteins were isolated by either the native or denaturing nickel-nitrilotriacetic (Ni-NTA) agarose according to the manufacturer's protocol (Qiagen, 28159 Avenue Stanford, Valencia, CA91355), dialyzed against PBS and stored at -20 °C. Purified proteins were checked on 12% SDS-PAGE gels and Western blot. Protein concentration was determined by the BCA Protein Assay reagent (PIERCE, Rockford, IL 61105, USA). Bacterial cells or purified proteins were applied to a 12% SDS-PAGE gel under denaturing conditions. Proteins were electroblotted onto nitrocellulose sheets (Schleicher and Schull, Dassel, Germany). Mouse His-tag antibody (SIGMA) was used as the primary antibody, while anti mouse horseraddish peroxidase conjugate (SIGMA) was used as the secondary antibody followed detection with 3,3'-Diamnobenzidine and hydrogen peroxide.

The reading frame of the segments Tp1, Tp4 and Tp5 amplified and cloned into the bacterial expression vectors harbored no substitutions compared to the original gene sequences. Recombinant protein containing His-tag were produced from all the constructs (Figure 15), and all can be determined by immunoblotting using His-tag antibody.

**Polymorphism in CTL target antigens**

Specific Tp1 forward (IL #12588: 5'-ATG GCC ACT TCA ATT GCA TTT GCC-3' (SEQ ID NO: 41) and reverse (IL #12589: 5'-TTA AAT GAA ATA TTT ATG AGC TTC-3') (SEQ ID NO: 42) primers were designed and used in PCR to amplify a 430 bp region containing the BV115 CTL epitope from genomic DNA of *T. parva* (Kakuzi521, Kilifi KL2, Kilifi KL1, Kilifi BR305, Nyairo IL02, Nyairo IL17, D409 Tp Mariakani, Buffalo7344c1, Zambia 2 and Uganda) isolated from infected animals in different regions. PCR products were sequenced and their deduced amino acid sequences were compared with Tp1 (Figure 17). The result showed variations among the Tp1 studied. This strongly indicated that Tp1 is polymorphic.

**Vaccination strategy**

Cattle trials are performed to assess the vaccine potential of identified CTL target antigens utilizing a recombinant canarypox virus (patented by Merial Ltd) as an antigen delivery method. The first of these trials is testing one of the candidate antigens and involves use of 16 cattle. Animals are inoculated intramuscularly with 1 ml of vaccine ($1x10^8$ pfu of virus) and boosted similarly after 4 weeks. Following a further 4 weeks, cattle are subjected to an $LD_{100}$ challenge with *T.parva* sporozoites by administering subcutaneously 1 ml of diluted stabilated infective material. Animals are monitored parasitologically and clinically over a period of 2-3 weeks to determine whether the vaccine has protected. It is expected that the vaccines are protective.

Immunological assays are performed following immunization and challenge to evaluate antigen-specific CTL responses and relate these to the outcome to challenge.

## Claims

1. An isolated polypeptide, comprising a sequence shown by one of SEQ ID NO: 1, SEQ ID NO: 6, SEQ ID NO: 7 or SEQ ID NO: 9.

2. A pharmaceutical composition, comprising one or more polypeptides of claim 1 and a pharmaceutically acceptable carrier.

3. An immunogenic composition, comprising one or more polypeptides of claim 1 and, optionally, an adjuvant.

4. The immunogenic composition of claim 3, which stimulates cytotoxic T cells specific to the polypeptide.

5. The immunogenic composition of claim 3, which comprises an epitope that stimulates *Theileria parva* (*T. parva*-) specific cytotoxic T cells.

6. The polypeptide of claim 1, which is present in detectable amounts in isolates of *T. parva.*

7. The polypeptide of claim 1, comprising a *T. parva* antigen.

8. An isolated polynucleotide comprising:

(a) a sequence shown by SEQ ID NO: 18, or SEQ ID NO: 23;
(b) a sequence which is at least about 90% identical to the sequence of (a);
(c) a sequence which encodes a polypeptide shown by SEQ ID NO: 1, SEQ ID NO: 6, SEQ ID NO: 7; or SEQ ID NO: 9; or
(d) a complement of any of (a), (b), or (c).

9. A pharmaceutical composition comprising the polynucleotide of claim 8 and a pharmaceutically acceptable carrier or excipient.

10. A recombinant construct, comprising a polynucleotide of claim 8, operably linked to an expression control sequence.

11. A vector comprising the recombinant construct of claim 10.

12. The vector of claim 11, which further comprises one or more sequences encoding a selectable marker.

13. The vector of claim 11 or 12, which comprises a plasmid, a bacteriophage, a minichromosome or a eukaryotic virus vector.

14. A host cell comprising a vector of any one of claims 11 to 13.

15. The host cell of claim 14, which is prokaryotic.

16. The host cell of claim 14, which is eukaryotic.

17. A method for producing a polypeptide which stimulates a T. parva-antigen specific cytotoxic lymphocyte (CTL), comprising culturing a host cell of any one of claims 14 to 16 under conditions effective for producing a polypeptide encoded by the polynucleotide, and harvesting the polypeptide.

18. An antibody specific for the polypeptide of claim 1.

19. The antibody of claim 18, which is a polyclonal antibody.

20. The antibody of claim 18, which is a monoclonal antibody.

21. The antibody of any one of claims 18 to 20, which is coupled to a carrier and/or a label.

22. A kit for detecting the presence of *T. parva* in a sample suspected of containing T. *parva,* or for purifying *T. parva* from a sample containing *T. parva,* comprising an antibody of any one of claims 18 to 21.

23. The kit of claim 22, which further comprises means for performing an enzyme-linked or Western blot assay to detect the presence of *T. parva.*

24. The kit of claim 22 or 23, which further comprises means for binding the antibody to *T. parva* in the sample, and for releasing the organism from the antibody.

25. An *in vitro* method for detecting a pathogenic protozoan infection in a subject, comprising contacting peripheral blood monocytes from the subject with peptide-antigen pulsed cytotoxic T lymphocytes, wherein the cytotoxic T lymphocytes are obtained from an animal to which has been administered:

(i) a polypeptide of claim 1, under conditions effective for the animal to generate *T. parva*-antigen-specific CTLs; or
(ii) a host cell of claim 16, under conditions effective for the animal to generate *T. parva*-antigen-specific CD4+ helper and CD8+ Cytotoxic T lymphocyte responses.

26. A method for detecting *T. parva* in a sample suspected of containing *T. parva,* comprising detecting in the sample a polynucleotide of claim 8.

**27.** The method of claim 26, which is high throughput.

**28.** A method for preparing a polyclonal antibody, comprising immunizing an animal with one or more polypeptides of claim 1 or with a host cell of claim 16.

**29.** A method for preparing a monoclonal antibody, comprising:

(a) immunizing an animal with a polypeptide of claim 1, or with a host cell of claim 16,
(b) recovering cells from the animal which produce antibody that binds to the polypeptide,
(c) preparing a hybridoma with the cells isolated in (b), and
(d) recovering a monoclonal antibody from the hybridoma that binds to the polypeptide in (a).

**30.** A method for identifying *T. parva* in a sample suspected of containing *T. parva,* comprising contacting the sample with an antibody of any one of claims 18 to 21, under conditions effective for the antibody to bind specifically to its cognate antigen, and detecting the presence of bound antibody.

**31.** The method of claim 30, wherein the detection is carried out by enzyme immunoassay, radioimmunoassay, fluorescence immunoassay, flocculation, particle agglutination, flow microfluorimetry, a competition assay, or *in situ* chromogenic assay.

**32.** The method of claim 30 or 31, wherein the antibody is a polyclonal antibody.

**33.** The method of claim 30 or 31, wherein the antibody is a monoclonal antibody.

**34.** The method of claim 30 or 31, which is quantitative.

**35.** The method of claim 30 or 31, which is high throughput.

**Patentansprüche**

**1.** Isoliertes Polypeptid, umfassend eine Sequenz, die in einer von SEQ ID NO:1, SEQ ID NO:6, SEQ ID NO:7 oder SEQ ID NO:9 gezeigt ist.

**2.** Arzneimittel, umfassend ein oder mehrere Polypeptide nach Anspruch 1 und einen pharmazeutisch verträglichen Träger.

**3.** Immunogene Zusammensetzung, umfassend ein oder mehrere Polypeptide nach Anspruch 1 und, gegebenenfalls, ein Adjuvans.

**4.** Immunogene Zusammensetzung nach Anspruch 3, die cytotoxische T-Zellen stimuliert, die spezifisch für das Polypeptid sind.

**5.** Immunogene Zusammensetzung nach Anspruch 3, die ein Epitop umfasst, welches *Theileria parva* (*T. parva*-)-spezifische cytotoxische T-Zellen stimuliert.

**6.** Polypeptid nach Anspruch 1, das in nachweisbaren Mengen in Isolaten von *T. parva* vorhanden ist.

**7.** Polypeptid nach Anspruch 1, umfassend ein *T. parva*-Antigen.

**8.** Isoliertes Polynucleotid, umfassend:

(a) eine Sequenz, die in SEQ ID NO:18 oder SEQ ID NO:23 gezeigt ist;
(b) eine Sequenz, die zu mindestens 90 % identisch mit der Sequenz von (a) ist;
(c) eine Sequenz, die ein Polypeptid codiert, das in SEQ ID NO:1, SEQ ID NO:6, SEQ ID NO:7 oder SEQ ID NO:9 gezeigt ist; oder
(d) ein Komplement von einem Beliebigen von (a), (b) oder (c).

**9.** Arzneimittel, umfassend das Polynucleotid nach Anspruch 8 und einen pharmazeutisch verträglichen Träger oder Exzipienten.

**10.** Fiekombinantes Konstrukt, umfassend ein Polynucleotid nach Anspruch 8, das funktionell mit einer Expressionskontrollsequenz verknüpft ist.

**11.** Vektor, der das rekombinante Konstrukt nach Anspruch 10 umfasst.

**12.** Vektor nach Anspruch 11, der weiterhin eine oder mehrere Sequenzen, die einen selektierbaren Marker codieren, umfasst.

**13.** Vektor nach Anspruch 11 oder 12, der ein Plasmid, einen Bakteriophagen, ein Minichromosom oder einen eukaryotischen Virus-Vektor umfasst.

**14.** Wirtszelle, die einen Vektor nach einem der Ansprüche 11 bis 13 umfasst.

**15.** Wirtszelle nach Anspruch 14, die prokaryotisch ist.

**16.** Wirtszelle nach Anspruch 14, die eukaryotisch ist.

**17.** Verfahren zur Herstellung eines Polypeptids, das *T. parva*-Antigen-spezifische cytotoxische Lymphocyten (CTL) stimuliert, umfassend das Züchten einer Wirtszelle nach einem der Ansprüche 14 bis 16 unter Bedingungen, die zur Herstellung eines Polypeptids, das durch das Polynucleotid codiert wird, wirksam sind, und Ernten des Polypeptids.

**18.** Antikörper, der spezifisch für das Polypeptid nach Anspruch 1 ist.

**19.** Antikörper nach Anspruch 18, der ein polyclonaler Antikörper ist.

**20.** Antikörper nach Anspruch 18, der ein monoclonaler Antikörper ist.

**21.** Antikörper nach einem der Ansprüche 18 bis 20, der an einen Träger und/oder eine Markierung gekoppelt ist.

**22.** Kit zum Nachweis des Vorhandenseins von *T. parva* in einer Probe, die vermutlich *T. parva* enthält, oder zur Aufreinigung von *T. parva* aus einer Probe, die *T. parva* enthält, umfassend einen Antikörper nach einem der Ansprüche 18 bis 21.

**23.** Kit nach Anspruch 22, das weiterhin Mittel zum Durchführen eines enzymgekoppelten oder Western-Blot-Assays zum Nachweis des Vorhandenseins von *T. parva* umfasst.

**24.** Kit nach Anspruch 22 oder 23, das weiterhin Mittel zum Binden des Antikörpers an *T. parva* in der Probe und zum Freisetzen des Organismus von dem Antikörper umfasst.

**25.** *In-vitro*-Verfahren zum Nachweis einer pathogenen Protozoen-Infektion in einem Individuum, umfassend das Inkontaktbringen von Monozyten des peripheren Bluts des Individuums mit Peptidantigen-gepulsten cytotoxischen T-Lymphocyten, wobei die cytotoxischen T-Lymphocyten von einem Tier erhalten wurden, dem Folgendes verabreicht wurde:

(i) ein Polypeptid nach Anspruch 1, unter Bedingungen, die wirksam sind, dass das Tier *T. parva*-Antigen-spezifische CTLs erzeugt; oder
(ii) eine Wirtszelle nach Anspruch 16, unter Bedingungen, die wirksam sind, dass das Tier *T. parva*-Antigen-spezifische CD4+-Helfer- und cytotoxische CD8+-T-Lymphocytenantworten erzeugt.

**26.** Verfahren zum Nachweis von *T. parva* in einer Probe, die vermutlich *T. parva* enthält, umfassend das Nachweisen eines Polynucleotids nach Anspruch 8 in der Probe.

**27.** Verfahren nach Anspruch 26, das ein Hochdurchsatzverfahren ist.

**28.** Verfahren zur Herstellung eines polyclonalen Antikörpers, umfassend das Immunisieren eines Tiers mit einem oder mehreren Polypeptiden nach Anspruch 1 oder mit einer Wirtszelle nach Anspruch 16.

**29.** Verfahren zur Herstellung eines monoclonalen Antikörpers, umfassend:

(a) Immunisieren eines Tiers mit einem Polypeptid nach Anspruch 1 oder mit einer Wirtszelle nach Anspruch 16,
(b) Gewinnen von Zellen aus dem Tier, die Antikörper produzieren, die an das Polypeptid binden,
(c) Herstellen eines Hybridoms mit den Zellen, die in (b) isoliert wurden, und
(d) Gewinnen eines monoclonalen Antikörpers aus dem Hybridom, der an das Polypeptid in (a) bindet.

**30.** Verfahren zur Identifizierung von *T. parva* in einer Probe, die vermutlich *T. parva* enthält, umfassend das Inkontaktbringen der Probe mit einem Antikörper nach einem der Ansprüche 18 bis 21, unter Bedingungen, die wirksam sind, dass der Antikörper spezifisch an sein entsprechendes Antigen bindet, und Nachweisen des Vorhandenseins von gebundenem Antikörper.

**31.** Verfahren nach Anspruch 30, wobei der Nachweis mit einem Enzym-Immunassay, einem Radioimmunassay, einem Fluoreszenzimmunassay, Flockung, Partikelagglutination, Durchflussmikrofluorimetrie, einem kompetitiven Assay oder einem chromogenen *in-situ*-Assay durchgeführt wird.

**32.** Verfahren nach Anspruch 30 oder 31, wobei der Antikörper ein polyclonaler Antikörper ist.

**33.** Verfahren nach Anspruch 30 oder 31, wobei der Antikörper ein monoclonaler Antikörper ist.

**34.** Verfahren nach Anspruch 30 oder 31, das quantitativ ist.

**35.** Verfahren nach Anspruch 30 oder 31, das ein Hochdurchsatzverfahren ist.

## Revendications

**1.** Polypeptide isolé, comprenant une séquence représentée par l'une de SEQ ID NO : 1, SEQ ID NO : 6, SEQ ID NO : 7 ou SEQ ID NO : 9.

**2.** Composition pharmaceutique, comprenant un ou plusieurs polypeptides selon la revendication 1 et un support pharmaceutiquement acceptable.

**3.** Composition immunogène, comprenant un ou plusieurs polypeptides selon la revendication 1 et, éventuellement, un adjuvant.

**4.** Composition immunogène selon la revendication 3, qui stimule les cellules T cytotoxiques spécifiques du polypeptide.

**5.** Composition immunogène selon la revendication 3, qui comprend un épitope qui stimule les cellules T cytotoxiques spécifiques de *Theileria parva* (*T. parva*).

**6.** Polypeptide selon la revendication 1, qui est présent dans des quantités détectables dans des isolats de *T. parva.*

**7.** Polypeptide selon la revendication 1, comprenant un antigène de *T. parva.*

**8.** Polynucléotide isolé comprenant :

(a) une séquence représentée par SEQ ID NO : 18, ou SEQ ID NO : 23 ;
(b) une séquence qui est au moins identique à environ 90 % à la séquence de (a) ;
(c) une séquence qui code pour un polypeptide représenté par SEQ ID NO : 1, SEQ ID NO : 6, SEQ ID NO : 7 ; ou SEQ ID NO : 9 ; ou
(d) un complémentaire de l'une quelconque de (a), (b) ou (c).

**9.** Composition pharmaceutique comprenant le polynucléotide selon la revendication 8 et un support ou un excipient pharmaceutiquement acceptable.

**10.** Produit d'assemblage recombinant, comprenant un polynucléotide selon la revendication 8, lié de manière fonctionnelle à une séquence de contrôle de l'expression.

**11.** Vecteur comprenant le produit d'assemblage recombinant selon la revendication 10.

**12.** Vecteur selon la revendication 11, qui comprend en outre une ou plusieurs séquences codant pour un marqueur sélectionnable.

**13.** Vecteur selon la revendication 11 ou 12, qui comprend un plasmide, un bactériophage, un minichromosome ou un vecteur eucaryote à base de virus.

**14.** Cellule hôte comprenant un vecteur selon l'une quelconque des revendications 11 à 13.

**15.** Cellule hôte selon la revendication 14, qui est procaryote.

**16.** Cellule hôte selon la revendication 14, qui est eucaryote.

**17.** Procédé de production d'un polypeptide qui stimule des lymphocytes cytotoxiques (LCT) spécifiques d'un antigène de *T parva,* comprenant la culture d'une cellule hôte selon l'une quelconque des revendications 14 à 16 dans des conditions efficaces pour la production d'un polypeptide codé par le polynucléotide, et la récolte du polypeptide.

**18.** Anticorps spécifique du polypeptide selon la revendication 1.

**19.** Anticorps selon la revendication 18, qui est un anticorps polyclonal.

**20.** Anticorps selon la revendication 18, qui est un anticorps monoclonal.

**21.** Anticorps selon l'une quelconque des revendications 18 à 20, qui est couplé à un support et/ou un marqueur.

**22.** Kit permettant la détection de la présence de *T. parva* dans un échantillon suspecté de contenir *T. parva,* ou la purification de *T. parva* à partir d'un échantillon contenant *T. parva*, comprenant un anticorps selon l'une quelconque des revendications 18 à 21.

**23.** Kit selon la revendication 22, qui comprend en outre un moyen pour réaliser un test ELISA ou Western blot afin de détecter la présence de *T. parva.*

**24.** Kit selon la revendication 22 ou 23, qui comprend en outre un moyen pour lier l'anticorps dirigé contre *T. parva* dans l'échantillon, et pour libérer l'organisme de l'anticorps.

**25.** Procédé *in vitro* de détection d'une infection à protozoaire pathogène chez un sujet, comprenant la mise en contact des monocytes du sang périphérique provenant du sujet avec des lymphocytes T cytotoxiques pulsés par un antigène peptidique, dans lequel les lymphocytes T cytotoxiques sont obtenus à partir d'un animal auquel a été administré :

(i) un polypeptide selon la revendication 1, dans des conditions efficaces pour que l'animal génère des LTC spécifiques d'un antigène de *T. parva* ; ou
(ii) une cellule hôte selon la revendication 16, dans des conditions efficaces pour que l'animal génère des réponses de lymphocytes T auxiliaires CD4+ spécifiques d'un antigène de T. parva et des réponses de lymphocytes T cytotoxiques CD8+ spécifiques d'un antigène de *T. parva.*

**26.** Procédé de détection de *T. parva* dans un échantillon suspecté de contenir *T. parva*, comprenant la détection dans l'échantillon d'un polynucléotide selon la revendication 8.

**27.** Procédé selon la revendication 26, qui est à haut débit.

**28.** Procédé de préparation d'un anticorps polyclonal, comprenant l'immunisation d'un animal avec un ou plusieurs polypeptides selon la revendication 1 ou avec une cellule hôte selon la revendication 16.

**29.** Procédé de préparation d'un anticorps monoclonal, comprenant :

(a) l'immunisation d'un animal avec un polypeptide selon la revendication 1, ou avec une cellule hôte selon la revendication 16,
(b) la récupération à partir de l'animal des cellules qui produisent l'anticorps qui se lie au polypeptide,
(c) la préparation d'un hybridome avec les cellules isolées en (b), et
(d) la récupération d'un anticorps monoclonal à partir de l'hybridome qui se lie au polypeptide en (a).

**30.** Procédé d'identification de *T. parva* dans un échantillon suspecté de contenir *T. parva*, comprenant la mise en contact de l'échantillon avec un anticorps selon l'une quelconque des revendications 18 à 21, dans des conditions efficaces pour que l'anticorps se lie spécifiquement à son antigène apparenté, et la détection de la présence de l'anticorps lié.

**31.** Procédé selon la revendication 30, dans lequel la détection est réalisée par un test immunoenzymatique, un test radioimmunologique, un test d'immunofluorescence, une floculation, une agglutination de particules, une microfluorimétrie en flux, un test par compétition, ou un test chromogène *in situ*.

**32.** Procédé selon la revendication 30 ou 31, dans lequel l'anticorps est un anticorps polyclonal.

**33.** Procédé selon la revendication 30 ou 31, dans lequel l'anticorps est un anticorps monoclonal.

**34.** Procédé selon la revendication 30 ou 31, qui est quantitatif.

**35.** Procédé selon la revendication 30 ou 31, qui est à haut débit.

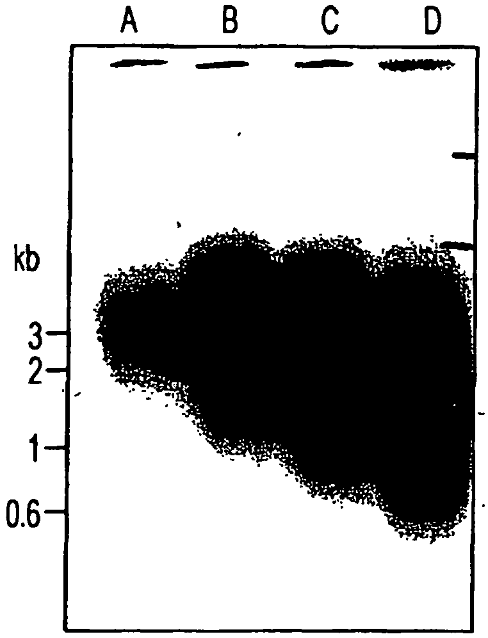


FIG. 1

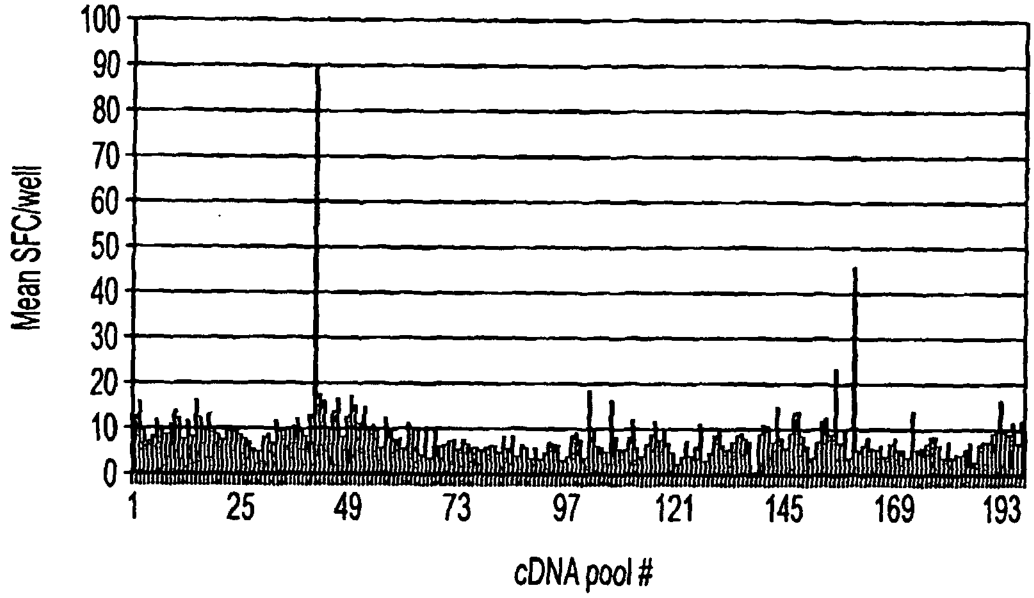

100
90
80
70
60
50
40
30
20
10
0
Mean SFC/well
1
25
49
73
97
121
145
169
193
cDNA pool #


FIG. 2

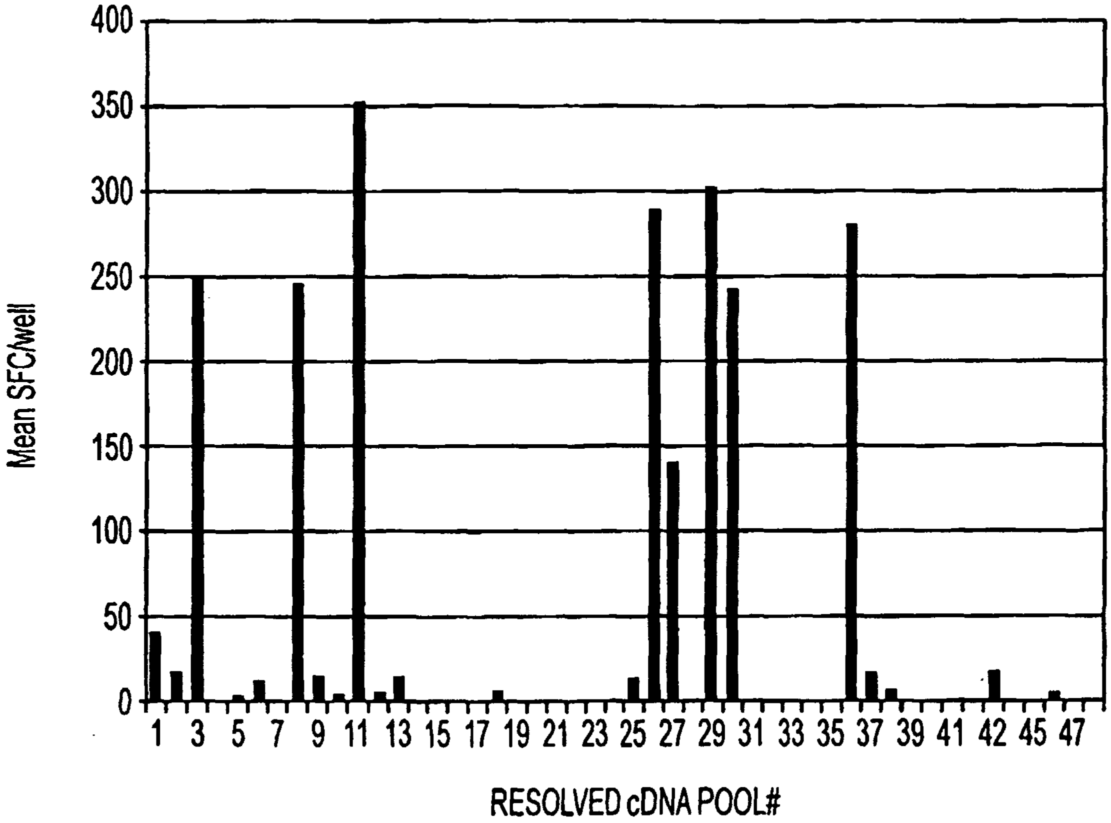
Mean SFC/well
400
350
300
250
200
150
100
50
0
1 3 5 7 9 11 13 15 17 19 21 23 25 27 29 31 33 35 37 39 41 42 45 47
RESOLVED cDNA POOL#

FIG. 3

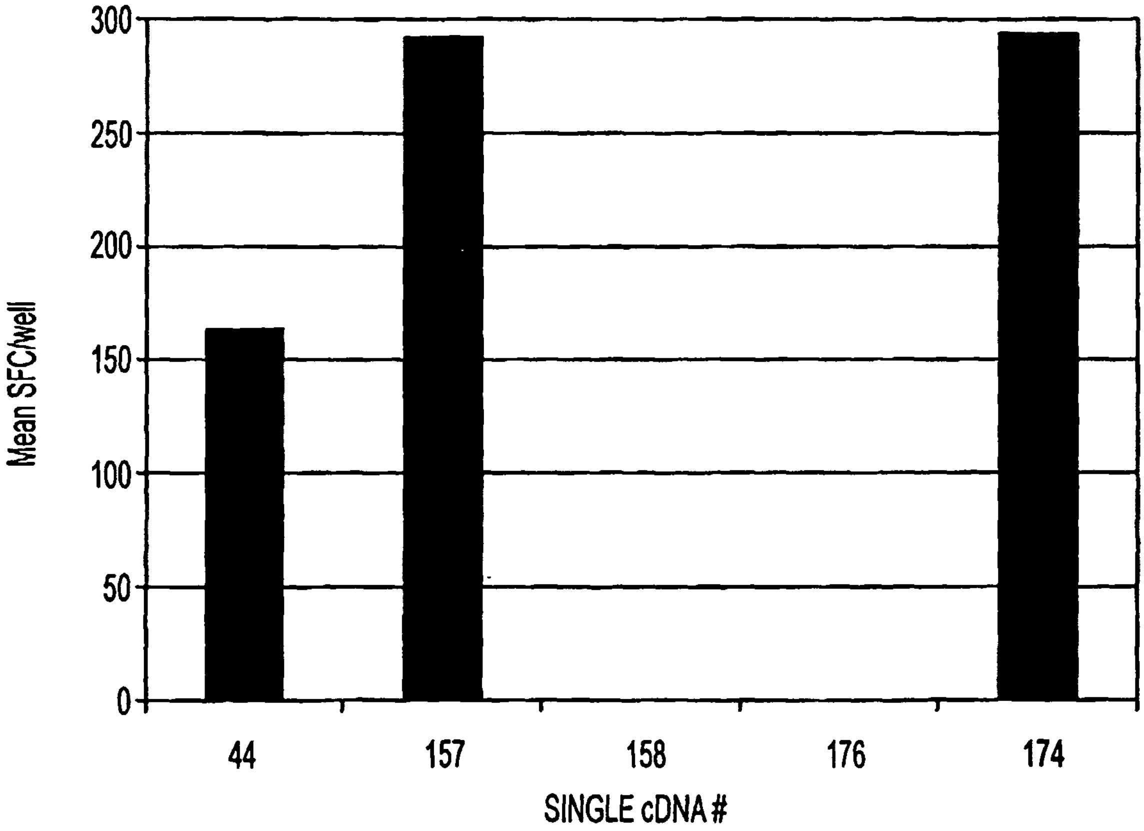
300
250
200
150
100
50
0
Mean SFC/well
44
157
158
176
174
SINGLE cDNA #

FIG. 4

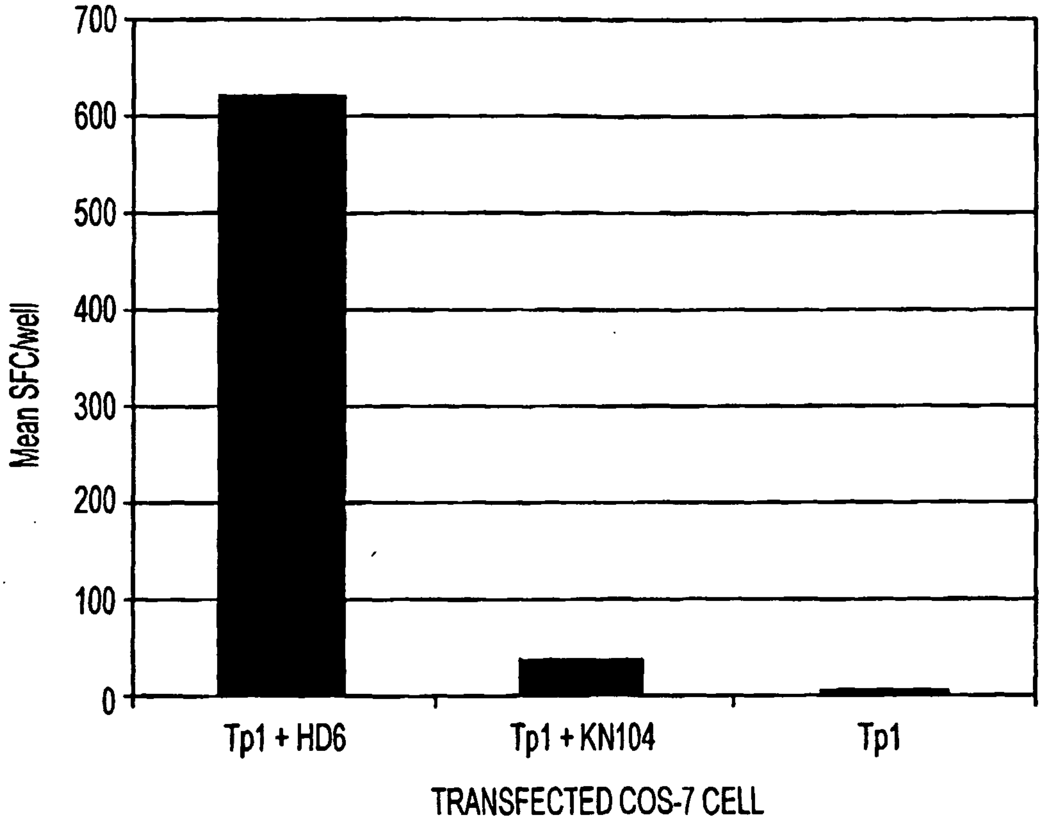
700
600
500
400
300
200
100
0
Mean SFC/well
Tp1 + HD6
Tp1 + KN104
Tp1
TRANSFECTED COS-7 CELL

FIG. 5

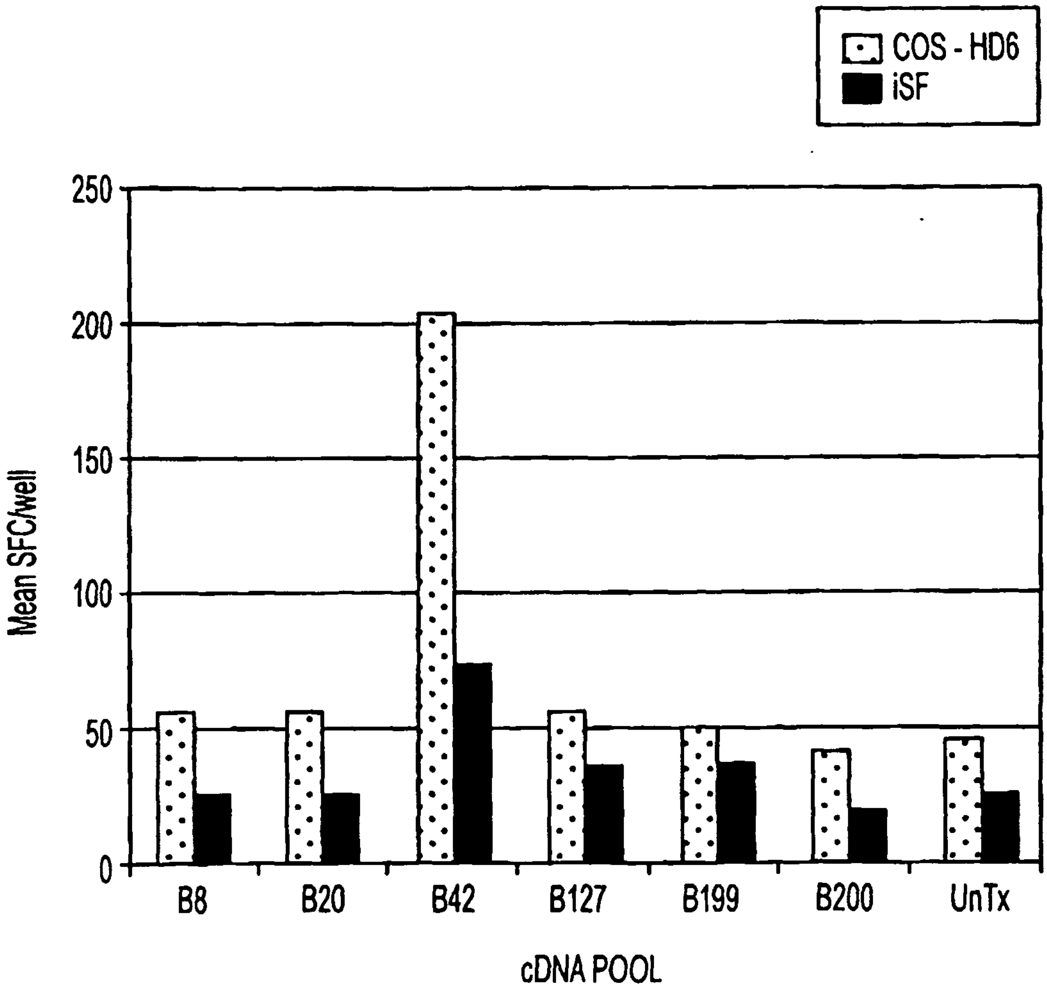
COS - HD6
iSF
250
200
150
100
50
0
Mean SFC/well
B8
B20
B42
B127
B199
B200
UnTx
cDNA POOL

FIG. 6

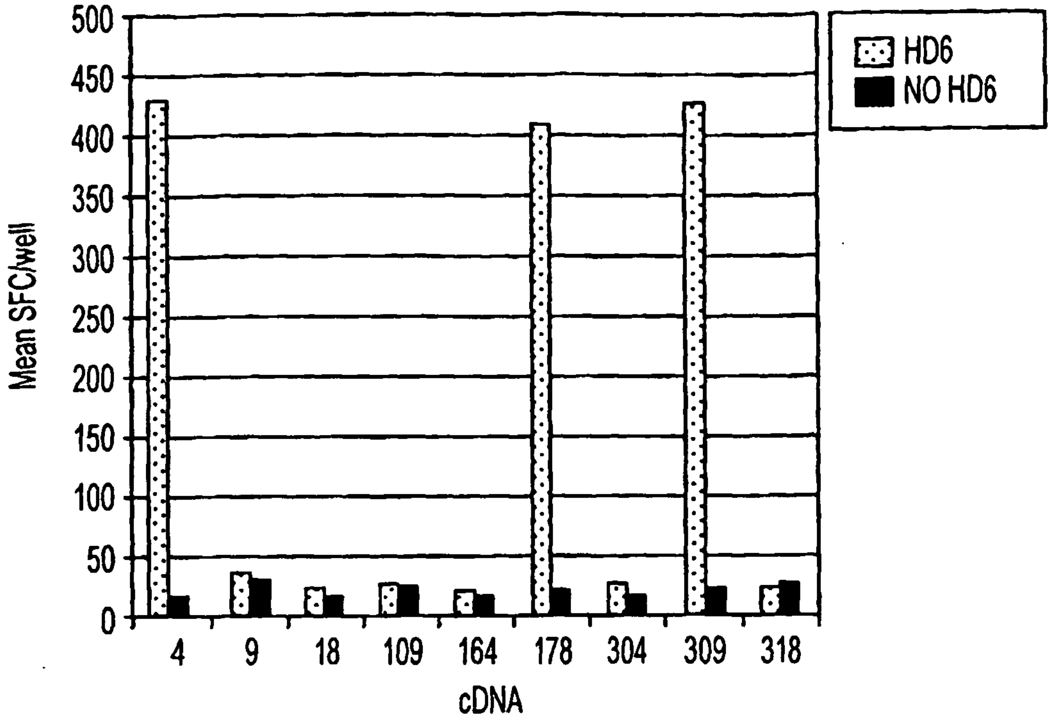
500
450
400
350
300
250
200
150
100
50
0
Mean SFC/well
4
9
18
109
164
178
304
309
318
cDNA
HD6
NO HD6

FIG. 7A

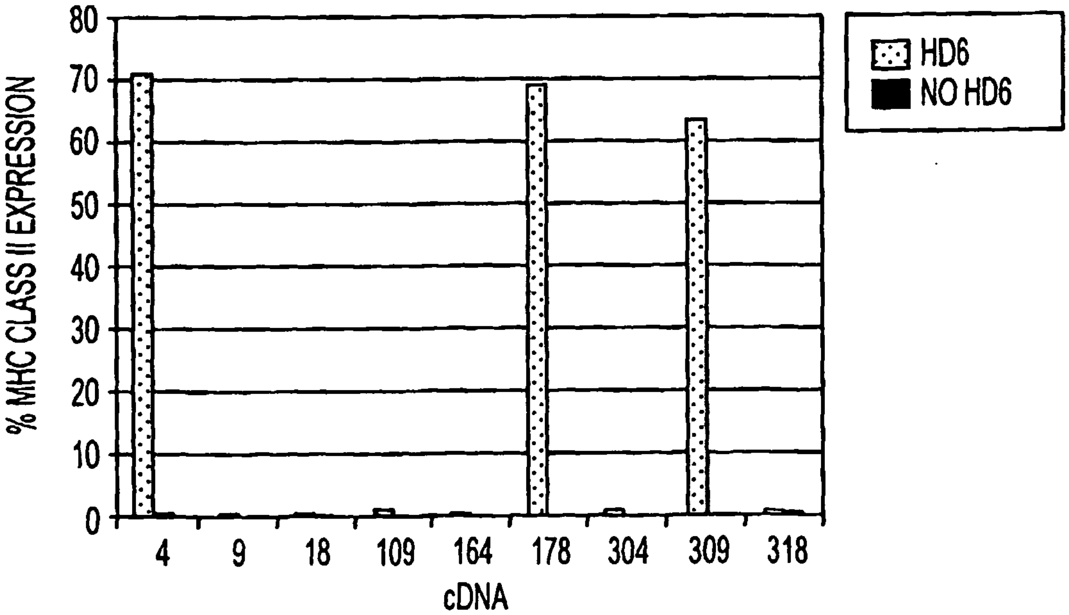
80
70
60
50
40
30
20
10
0
% MHC CLASS II EXPRESSION
4
9
18
109
164
178
304
309
318
cDNA
HD6
NO HD6

FIG. 7B

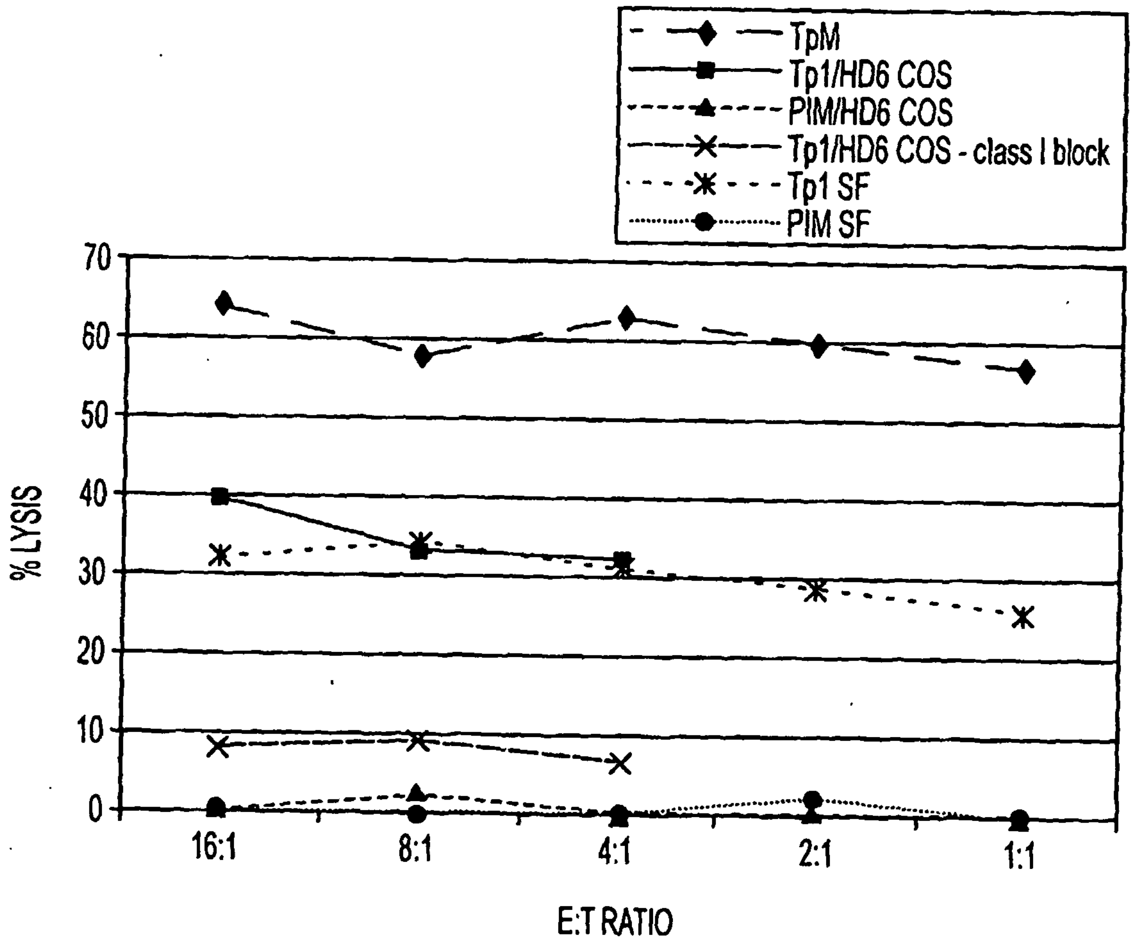
TpM
Tp1/HD6 COS
PIM/HD6 COS
Tp1/HD6 COS - class I block
Tp1 SF
PIM SF
70
60
50
40
30
20
10
0
% LYSIS
16:1
8:1
4:1
2:1
1:1
E:T RATIO

FIG. 8

```
                 10         20         30         40         50         60         70
80
Tp1 ORF   MRVKKVLLYT LPVVGILLAG SLIIFNFVRK RPEKEEELKP PSALEDELKK REEESRKRME EMQKEILEKK LREGKKALEE
Tp1 Del1  MRVKKVLLYT LPVVGILLAG SLIIFNFVRK RPEKEEELKP PSALEDELKK REEESRKRME EMQKEILEKK LREGKKALEE
Tp1 Del2  MRVKKVLLYT LPVVGILLAG SLIIFNFVRK RPEKEEELKP PSALEDELKK REEESRKRME EMQKEILEKK LREGKKALEE
Tp1 Del3  MRVKKVLLYT LPVVGILLAG SLIIFNFVRK RPEKEEELKP PSALEDELKK REEESRKRME EMQKEILEKK LREGKKALEE
Tp1 Del4  MRVKKVLLYT LPVVGILLAG SLIIFNFVRK RPEKEEELKP PSALEDELKK REEESRKRME EMQKEILEKK LREGKKALEE
Tp1 Del5  MRVKKVLLYT LPVVGILLAG SLIIFNFVRK RPEKEEELKP PSALEDELKK REEESRKRME EMQKEILEKK LREGKKALEE
Tp1 Del6  MRVKKVLLYT LPVVGILLAG SLIIFNFVRK RPEKEEE... .......... .......... .......... ..........
              90        100        110        120        130        140        150        160
Tp1 ORF   LEKREKEVVD EFAKHLKKPE ERLPKIILTL DSGFPTVDPI TYTSGVYMVA VSKTTFTSDS DLVDFTHTLL GIKFLVTGVQ
Tp1 Del1  LEKREKEVVD EFAKHLKKPE ERLPKIILTL DSGFPTVDPI TYTSGVYMVA VSKTTFTSDS DLVDFTHTLL GIKFLVTGVQ
Tp1 Del2  LEKREKEVVD EFAKHLKKPE ERLPKIILTL DSGFPTVDPI TYTSGVYMVA VSKTTFTSDS DLVDFTHTLL GIKFLVTGVQ
Tp1 Del3  LEKREKEVVD EFAKHLKKPE ERLPKII... DSGFPTVDPI TYTSGVYMVA VSKTTFTSDS DLVDFTHTLL GIKFLVTGVQ
Tp1 Del4  LEKREKEVVD EFAKHLKKPE ERLPKII... .......... .......... .......... .......... ..........
Tp1 Del5  LEKREKEVVD EFAKHLKKPE ERL....... .......... .......... .......... .......... ..........
Tp1 Del6  .......... .......... .......... .......... .......... .......... .......... ..........
                170        180        190        200        210        220        230        240
Tp1 ORF   FGGKTYTIKP IEATMATSIA FAADPGFCYF LLIPGPDSKP IFFKNDGDKF LRCVGYPKVK EEMLEMATKF NRLPKGVEIP
Tp1 Del1  FGGKTYTIKP IEATMATSIA FAADPGFCYF LLIPGPDSKP IFFKNDGDKF LRCVGYPKVK EEMLEMATKF NRLPKGVEIP
Tp1 Del2  FGGKTYTIKP IEATMATSIA FAADPGFCYF LLIPGPDSKP IFFKNDGDKF LRCVGYPKVK EEMLEMATKF NRLPKGVEIP
Tp1 Del3  .......... .......... .......... .......... .......... .......... .......... ..........
Tp1 Del4  .......... .......... .......... .......... .......... .......... .......... ..........
Tp1 Del5  .......... .......... .......... .......... .......... .......... .......... ..........
Tp1 Del6  .......... .......... .......... .......... .......... .......... .......... ..........
                250        260        270        280        290        300        310        320
Tp1 ORF   APPGVKPEAP TPTPTTITPS VPPTIPTPIT PSAPPTTPPT GLNFNLTVQN KFMIGSQEVK LNITHEYEGV YEAHKYFIER
Tp1 Del1  APPGVKPEAP TPTPTTITPS VPPTIPTPIT PSAPPTTPPT GLNFNLTVQN KFMIGSQEVK LNITHEYEGV YEAHKYFIER
Tp1 Del2  A......... .......... .......... .......... .......... .......... .......... ..........
Tp1 Del3  .......... .......... .......... .......... .......... .......... .......... ..........
Tp1 Del4  .......... .......... .......... .......... .......... .......... .......... ..........
Tp1 Del5  .......... .......... .......... .......... .......... .......... .......... ..........
Tp1 Del6  .......... .......... .......... .......... .......... .......... .......... ..........
                330        340        350        360        370        380        390        400
Tp1 ORF   GSFTPTSFSI GDLPQTGLPV NQTVDTIVVY FHRVTMGEPV GIPLIVLIFY KNQSRKYLNK GNGNWEESKA LLFREELDYL
Tp1 Del1  GSFTPTSFSI GDLPQTGLPV NQTVDTIVVY FHRVTMGEPV GIPLIVLIFY KNQSRKYLNK GNGNWEESKA LLFREELDYL
Tp1 Del2  .......... .......... .......... .......... .......... .......... .......... ..........
Tp1 Del3  .......... .......... .......... .......... .......... .......... .......... ..........
Tp1 Del4  .......... .......... .......... .......... .......... .......... .......... ..........
Tp1 Del5  .......... .......... .......... .......... .......... .......... .......... ..........
Tp1 Del6  .......... .......... .......... .......... .......... .......... .......... ..........
                410        420        430        440        450        460        470        480
Tp1 ORF   DSIFNDFVTV NLSRRSDYYR NGTGTSEIEQ TLDMNVYVEP DTPCAGWTTY IHKLEEGGEG GIEKPFQIRQ LWFSKQKFDI
Tp1 Del1  DSIFNDFVTV NLSRRSDYYR NGTGTSEIEQ TLDMNVYVEP DTPCAGWTTY IHKLEEGGEG GIEKPFQIRQ LWFSKQKFDI
Tp1 Del2  .......... .......... .......... .......... .......... .......... .......... ..........
Tp1 Del3  .......... .......... .......... .......... .......... .......... .......... ..........
Tp1 Del4  .......... .......... .......... .......... .......... .......... .......... ..........
Tp1 Del5  .......... .......... .......... .......... .......... .......... .......... ..........
Tp1 Del6  .......... .......... .......... .......... .......... .......... .......... ..........
                490        500        510        520        530        540        550
Tp1 ORF   FPMGKVSIVN VYGKNDEPLS YAPSIFSVIR EDGIQIFYVR AYSQYLLDSS VNPQNLPQKL NTL*......
Tp1 Del1  FPMGKVSIVN VYGKNDEPLS YAPSIFSVIR EDGIQIFYVR AYSQYLLDSS VNPQNLPQKL NTL*......
Tp1 Del2  .......... .......... .......... .......... .......... .......... ..........
Tp1 Del3  .......... .......... .......... .......... .......... .......... ..........
Tp1 Del4  .......... .......... .......... .......... .......... .......... ..........
Tp1 Del5  .......... .......... .......... .......... .......... .......... ..........
Tp1 Del6  .......... .......... .......... .......... .......... .......... ..........
```

FIG. 9

```
              200        210        220        230        240
Tp1.1    mPGPDSKP IFFKNDGDKF LRCVGYPKVK EEMLEMATKF NRLPKGVEIP
Tp1.2
Tp1.3
Tp1.4    mPGPDSKP IFFKNDGDKF LRCVGYPKVK EEMLEMATKF NRLPKGVEIP
Tp1.5
Tp1.6    mPGPDSKP IFFKNDGDKF LRCVGYPKVK EEMLEMATKF NRLPKGVEIP

              250        260        270        280        290
Tp1.1 APPGVKPEAP TPTPTTIT
Tp1.2         mP TPTTITPS VPPTIPTPIT PSAPPTTPPT GLNFNLTVQN
Tp1.3
Tp1.4 APPGVKPEAP TPTTITPSVP PTIPTPITPS APPTTPPTGL NFNLTVQNKF
Tp1.5         mP TPTTITPSVP PTIPTPITPS APPTTPPTGL NFNLTVQNKF
Tp1.6 APPGVKPEAP TPTTITPSVP PTIPTPITPS APPTTPPTGL NFNLTVQNKF

              300        310        320        330        340
Tp1.1
Tp1.2 KFMIGSQEVK LNITHEYEGV YEAHKYFI
Tp1.3                  mGV YEAHKYFIER GSFTPTSFSI GDLPQTGLPV
Tp1.4 KFMIGSQEVK LNITHEYEGV YEAHKYFIER GSFTPTSFSI GDLPQTGLPV
Tp1.5 KFMIGSQEVK LNITHEYEGV YEAHKYFIER GSFTPTSFSI GDLPQTGLPV
Tp1.6 KFMIGSQEVK LNITHEYEGV YEAHKYFIER GSFTPTSFSI GDLPQTGLPV

             350        360        369
Tp1.1
Tp1.2
Tp1.3 NQTVDTIVVY FHRVTMGEPV GIPLIVLIF
Tp1.4
Tp1.5 NQTVDTIVVY FHRVTMGEPV GIPLIVLIF
Tp1.6 NQTVDTIVVY FHRVTMGEPV GIPLIVLIF
```

FIG. 10

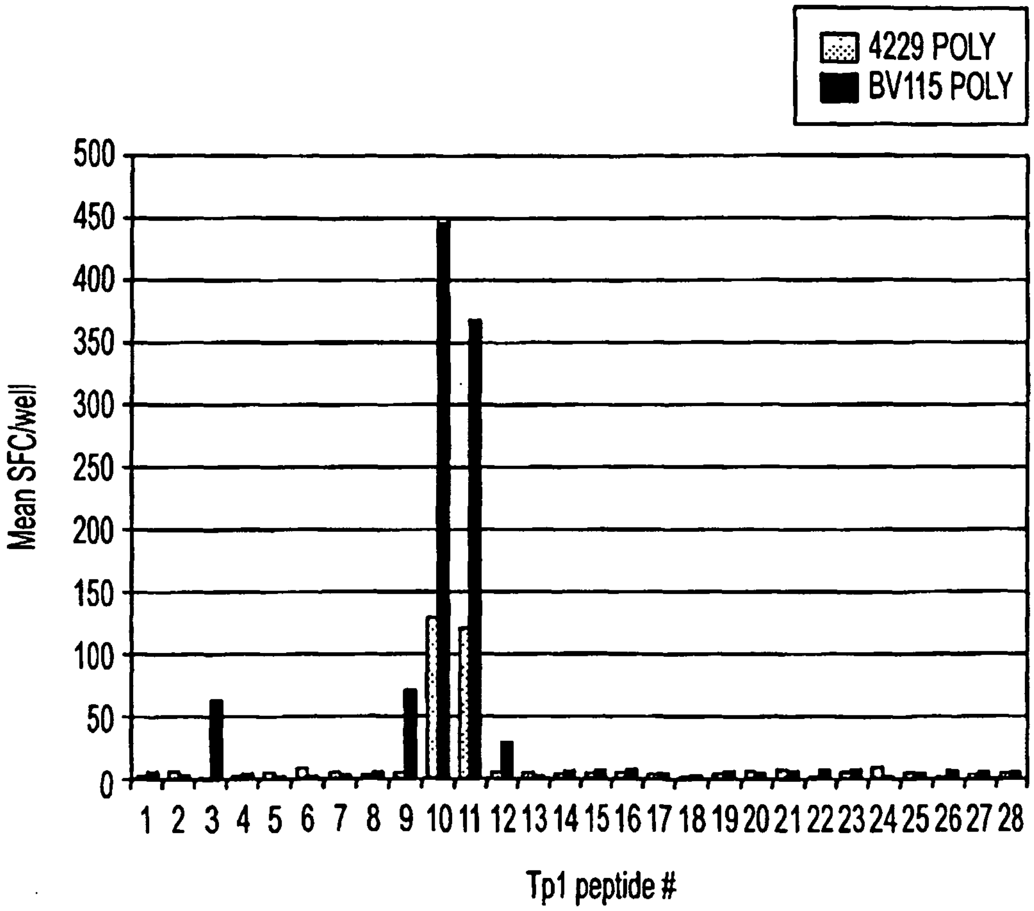
4229 POLY
BV115 POLY
500
450
400
350
300
250
200
150
100
50
0
Mean SFC/well
1 2 3 4 5 6 7 8 9 10 11 12 13 14 15 16 17 18 19 20 21 22 23 24 25 26 27 28
Tp1 peptide #

FIG. 11

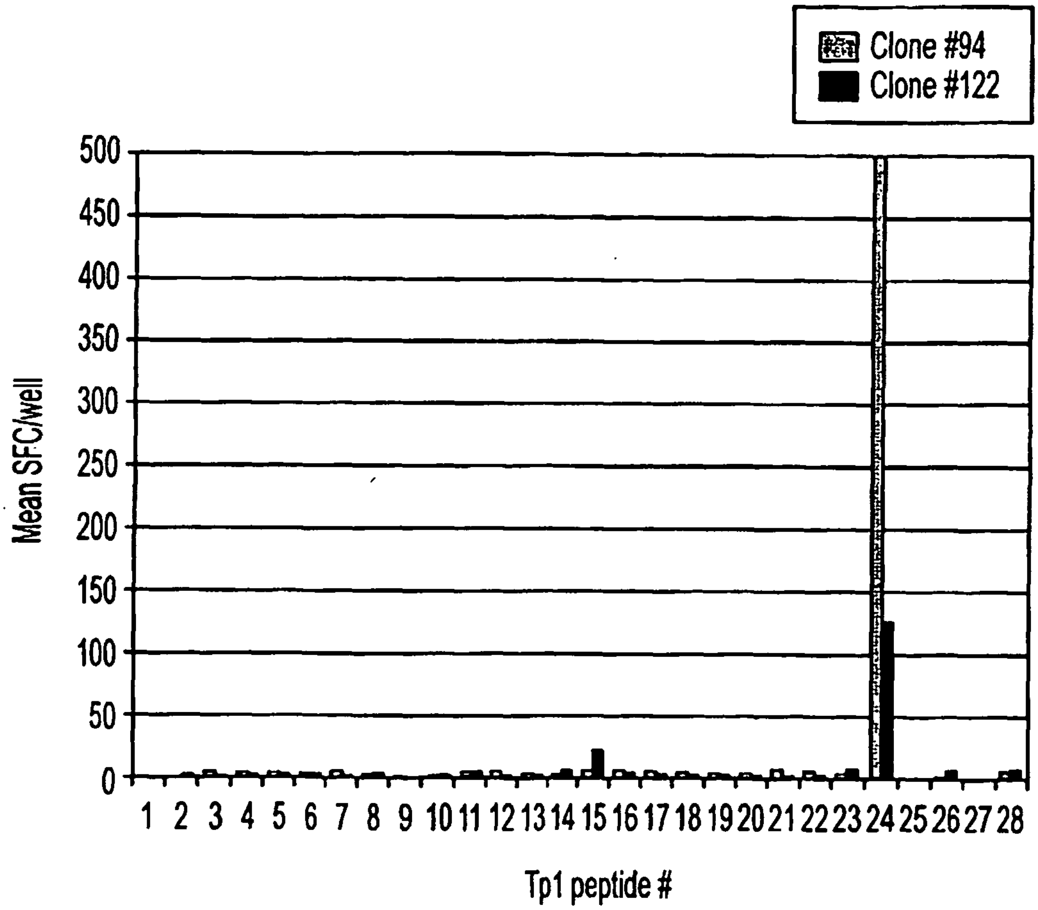
Clone #94
Clone #122
500
450
400
350
300
250
200
150
100
50
0
Mean SFC/well
1 2 3 4 5 6 7 8 9 10 11 12 13 14 15 16 17 18 19 20 21 22 23 24 25 26 27 28
Tp1 peptide #

FIG. 12

```
                       10        20        30        40        50        60
Tp1Muguga     MRVKKVLLYTLPVVGILLAGSLIIFNFVRKRPEKEEELKPPSALEDELKKREEESRKRME
              ::::::::::::::::::::::::::::::::::::::::::::::::::::::::::::
Tp1Marikebuni MRVKKVLLYTLPVVGILLAGSLIIFNFVRKRPEKEEELKPPSALEDELKKREEESRKRME
                      10        20        30        40        50        60
                      70        80        90       100       110       120
Tp1Muguga     EMQKEILEKKLREGKKALEELEKREKEVVDEFAKHLKKPEERLPKIILTLDSGFPTVDPI
              ::::::::::::::::::::::: :::.:::: :::::::::::::::: ::::::::::
Tp1Marikebuni EMQKEILEKKLREGKKALEELEKCEKEMVDEFEKHLKKPEERLPKIILILDSGFPTVDPI
                      70        80        90       100       110       120
                     130       140       150       160       170       180
Tp1Muguga     TYTSGVYMVAVSKTTFTSDSDLVDFTHTLLGIKFLVTGVQFGGKTYTIKPIEATMATSIA
              :::::::::::::::::::::::::::::::::::: :::::::::::::::::::::::
Tp1Marikebuni TYTSGVYMVAVSKTTFTSDSDLVDFTHTLLGIKFLVAGVQFGGKTYTIKPIEATMATSIA
                     130       140       150       160       170       180
                     190       200       210       220       230       240
Tp1Muguga     FAADPGFCYFLLIPGPDSKPIFFKNDGDKFLRCVGYPKVKEEMLEMATKFNRLPKGVEIP
              ::::::::::::::::::::::::::::::::::::::::::. ::::::::::::::::
Tp1Marikebuni FAADPGFCYFLLIPGPDSKPIFFKNDGDKFLRCVGYPKVKEEIIEMATKFNRLPKGVEIP
                     190       200       210       220       230       240
                     250       260       270       280       290       300
Tp1Muguga     APPGVKPEAPTPTPTTITPSVPPTIPTPITPSAPPTTPPTGLNFNLTVQNKFMIGSQEVK
              :::::::::::::::::::::::::::::::::::::::::::::::::::::.::::::
Tp1Marikebuni APPGVKPEAPTPTPTTITPSVPPTIPTPITPSAPPTTPPTGLNFNLTVQNKFMVGSQEVK
                     250       260       270       280       290       300
                     310       320       330       340       350       360
Tp1Muguga     LNITHEYEGVYEAHKYFIERGSFTPTSFSIGDLPQTGLPVNQTVDTIVVYFHRVTMGEPV
              ::::::: ::::::::::.:: ::::::::. :::::::::::::::::::::::::::
Tp1Marikebuni LNITHEYDGVYEAHKYFIEKGRFTPTSFSIGADPQTGLPVNQTVDTIVVYFHRVTMGEPV
                     310       320       330       340       350       360
                     370       380       390       400       410       420
Tp1Muguga     GIPLIVLIFYKNQSRKYLNKGNGNWEESKALLFREELDYLDSIFNDFVTVNLSRRSDYYR
              :::::::.:::::: ::::::::::::::::::::::.:::.:: .::::::::::::
Tp1Marikebuni GIPLIVLVFYKNQSTKYLNKGNGNWEESKALLFREELDFLDSMFNGYVTVNLSRRSDYYR
                     370       380       390       400       410       420
                     430       440       450       460       470       480
Tp1Muguga     NGTGTSEIEQTLDMNVYVEPDTPCAGWTTYIHKLEEGGEGGIEKPFQIRQLWFSKQKFDI
              :::::::::.:::::::::::::: :::::::::::::::::::::::::::::::::::
Tp1Marikebuni NGTGTSEIEKTLDMNVYVEPDTPCLGWTTYIHKLEEGGEGGIEKPFQIRQLWFSKQKFDI
                     430       440       450       460       470       480
                     490       500       510       520       530       540
Tp1Muguga     FPMGKVSIVNVYGKNDEPLSYAPSIFSVIREDGIQIFYVRAYSQYLLDSSVNPQNLPQKL
              ::::::::::::::::::::::::::::::::::::::::::::::::::::::::::::
Tp1Marikebuni FPMGKVSIVNVYGKNDEPLSYAPSIFSVIREDGIQIFYVRAYSQYLLDSSVNPQNLPQKL
                     490       500       510       520       530       540

Tp1Muguga     NTL
              ..
Tp1Marikebuni TAE
```

FIG. 13

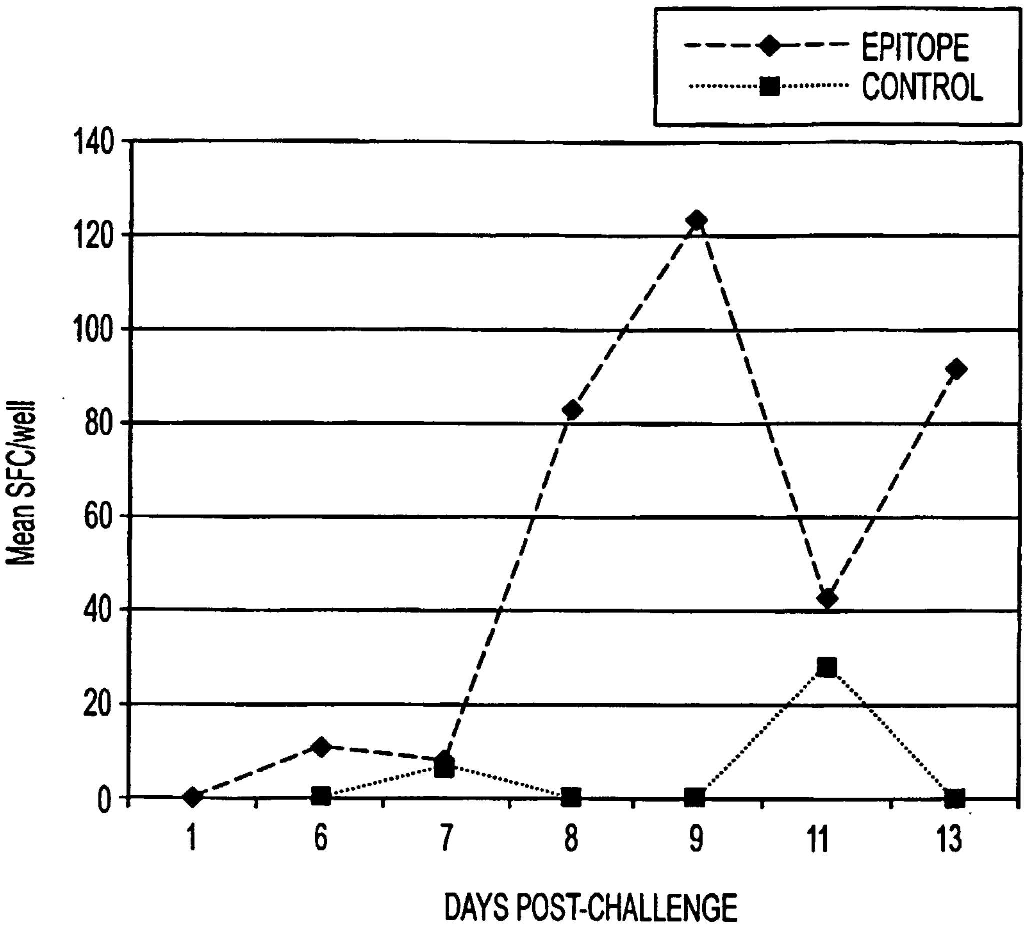
EPITOPE
CONTROL
140
120
100
80
60
40
20
0
Mean SFC/well
1
6
7
8
9
11
13
DAYS POST-CHALLENGE

FIG. 14

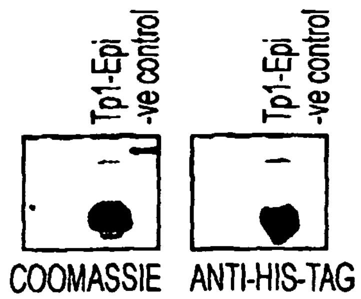


FIG. 15A

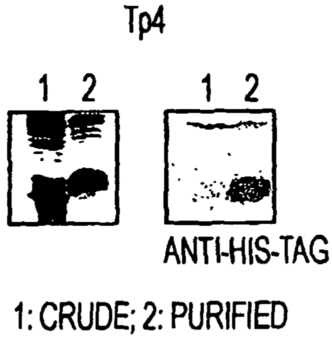


FIG. 15B

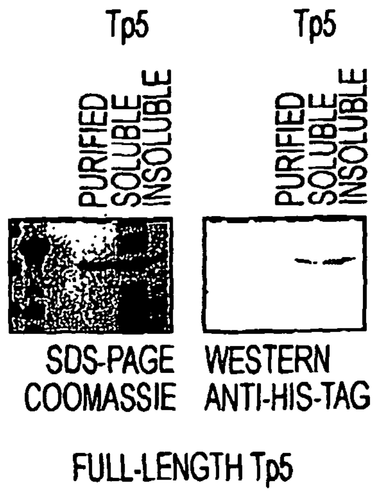


FIG. 15C

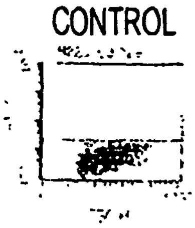


FIG. 16Aa

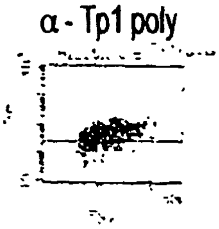


FIG. 16Ab

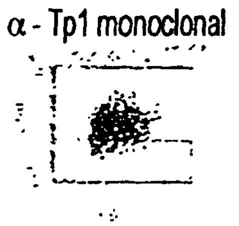


FIG. 16Ac

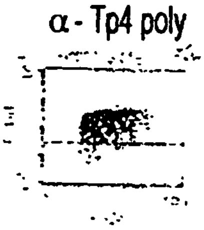


FIG. 16Ad

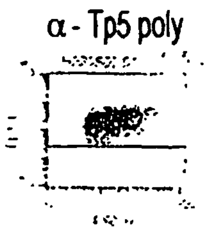


FIG. 16Ae

| ANTIBODY | % OF T. *PARVA* INFECTED CELLS STAINED |
|---|---|
| Anti-Tp1 polyclonal Ab | 78 |
| Anti-Tp1 monoclonal Ab | 85.96 |
| Anti-Tp4 polyclonal Ab | 92.52 |
| Anti-Tp5 polyclonal Ab | 96.04 |
| Control | 0.32 |

FIG. 16B

```
Tp1              MATSIAFAADPGFCYFLLIPGPDSKPIFFKNDGDKFLRCVGYPKVKEEMLEMATKFNRLPKGVEIPAPPGVK
Kakuz521         MATSIAFAADPGFCYFLLIPGPDSKPIFFKNDGDKFLRCVGYPKVKEEMLEMATKFNRLPKGVEIPAPPGVK
NyairoIL02       MATSIAFAADPGFCYFLLIPGPDSKPIFFKNDGDKFLRCVGYPKVKEEMLEMATKFNRLPKGVEIPAPPGVK
NyairoIL17       MATSIAFAADPGFCYFLLIPGPDSKPIFFKNDGDKFLRCVGYPKVKEEMLEMATKFNRLPKGVEIPAPPGVK
Kakuzi521        MATSIAFAADPGFCYFLLIPGPDSKPIFFKNDGDKFLRCVGYPKVKEEMLEMATKFNRLPKGVEIPAPPGVK
Buffalo7344cl    MATSIAFAADPGFCYFLLIPGPDSKPIFLKNDGDKFLRCVGYPKVKEEMLEMATKFNRLPKGVEIPAPPGVK
KilifKL2         MATSIAFAADPGFCYFLLIPGPDSKPIFFKNDGDKFLRCVGYPKVKEEIIEMATKFNRLPKGVEIPAPPGVK
D409TpMariakani  MATSIAFAADPGICYFLLIPAP--KPIFFKNDGDKFLRCVGYPKVKEEIIEMATKFNRLPKGVEIPAPPGVK
KilifiBR305      MATSIAFAADPGFCYFLLIPGPDSKPIFFKNDGDKFLRCVGYPKVKEEILEMATKFNRLPKGVEIPAPPGVK
KilifiKL1        MATSIAFAADPGFCYFLLIPGPDSKPIFFKNDGDKFLRCVGYPKVKEEILEMATKFNRLPKGVEIPAPPGVK
Zambia2          MATSIAFAADPGFCYFLLIPGPDSKPIFFKNDGDKFLRCVGYPKVKEEIIEMATKFNRLPKGVEIPAPPGVK
Uganda           MATSIAFAADPGFCYFLLIPGPDSKPIFFKNDGDKFLRCVGYPKVKEEIIEMATKFNRLPKGVEIPAPPGVK

Tp1              PEAPTPTPTTITPSVPPTIPTPITPSAPPTTPPTGLNFNLTVQNKFMIGSQEVKLNITHEYEGVYEAHKYFI
Kakuzi521        PEAPTPTPTTITPSVPPTIPTPITPSAPPTTPPTGLNFNLTVQNKFMIGSQEVKLNITHEYEGVYEAHKYFI
NyairoIL02       PEAPTPTPTTITPSVPPTIPTPITPSAPPTTPPTGLNFNLTVQNKFMIGSQEVNLNITHEYEGVYEAHKYFI
NyairoIL17       PEAPTPTPTTITPSVPPTIPTPITPSAPPTTPPTGLNFNLTVQNKFMIGSQEVNLNITHEYEGVYEAHKYFI
Kakuzi521        PEAPTPTPTTITPSVPPTIPTPITPSAPPTTPPTGLNFNLTVQNKFMIGSQEVKLNITHEYEGVYEAHKYFI
Buffalo7344cl    PEAPTPTPTPITPSAPPTT--------PPTTPPKGLNFNLTLQNKFMIGSQEVKLSITHEYDGVYEAHKYFI
KilifKL2 ,       PEAPTPTPTTITPSVPPTIPTPITPSAPPTTPPTGLNFNLTVQNKFMVGSQEVKLNITHEYDGVYEAHKYFI
D409TpMariakani  PEAPTPTPTTITPSVPPTIPTPITPSAPPTTPPTGLNFNLTVQNKFMVGSQEVKLNITHEYDGVYEAHKYFI
KilifiBR305      PEAPTPTPTTITPSVPPTIPTPITPSAPPTTPPTGLNFNLTVQNKFMVGSQEVKLNITHEYEGVYEAHKYFI
KilifiKL1        PEAPTPTPTTITPSVPPTIPTPITPSAPPTTPPTGLNFNLTVQNKFMIGSPEVKLNITHEYEGVYEAHKYFI
Zambia2          PEAPTPTPTTITPSVPPTIPTPITPSAPPTTPPTGLNFNLTVQNKFMVGSQEVKLNIPHEYDGVYEAHKYFI
Uganda           PEAPTPTPTTITPSVPPTIPTPITPSAPPTTPPTGLNFNLTVQNKFMVGSQEVKLNITHEYDGVYEAHKYFI
```

FIG. 17

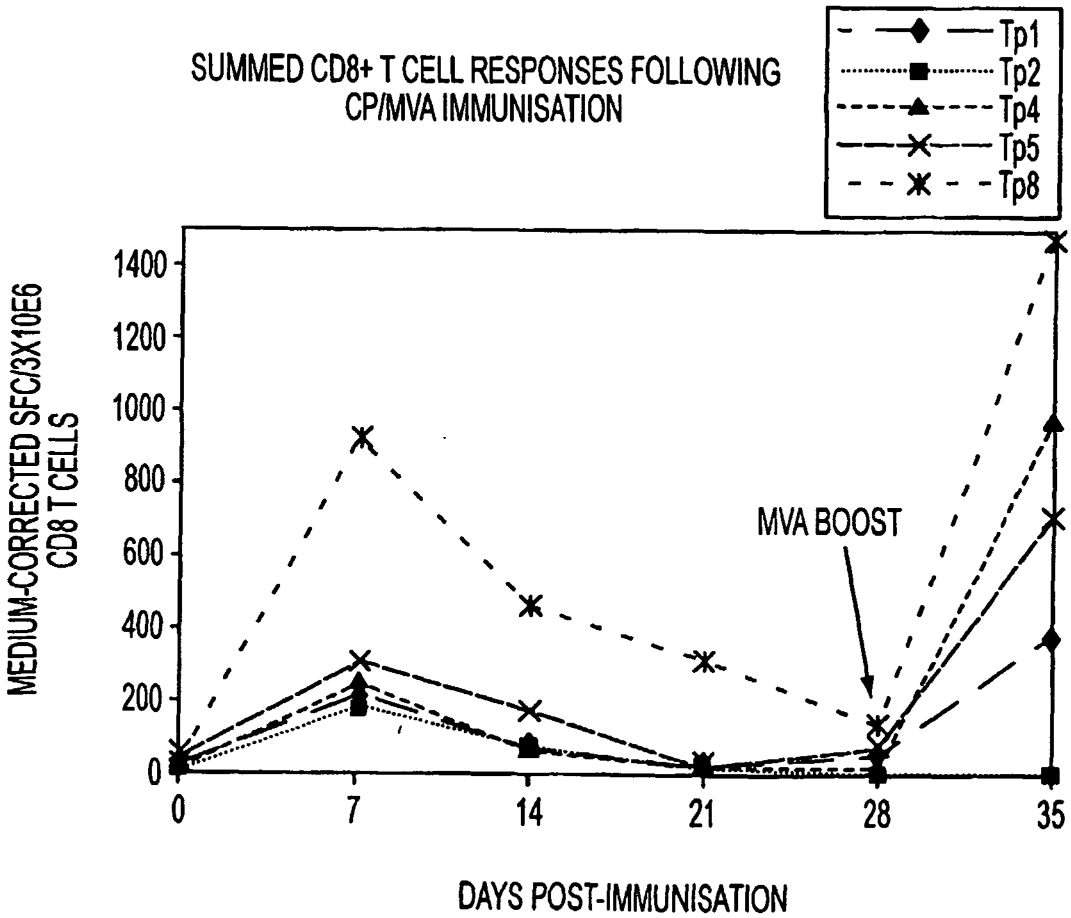
SUMMED CD8+ T CELL RESPONSES FOLLOWING
CP/MVA IMMUNISATION
Tp1
Tp2
Tp4
Tp5
Tp8
MEDIUM-CORRECTED SFC/3X10E6
CD8 T CELLS
1400
1200
1000
800
600
400
200
0
MVA BOOST
0
7
14
21
28
35
DAYS POST-IMMUNISATION

FIG. 18

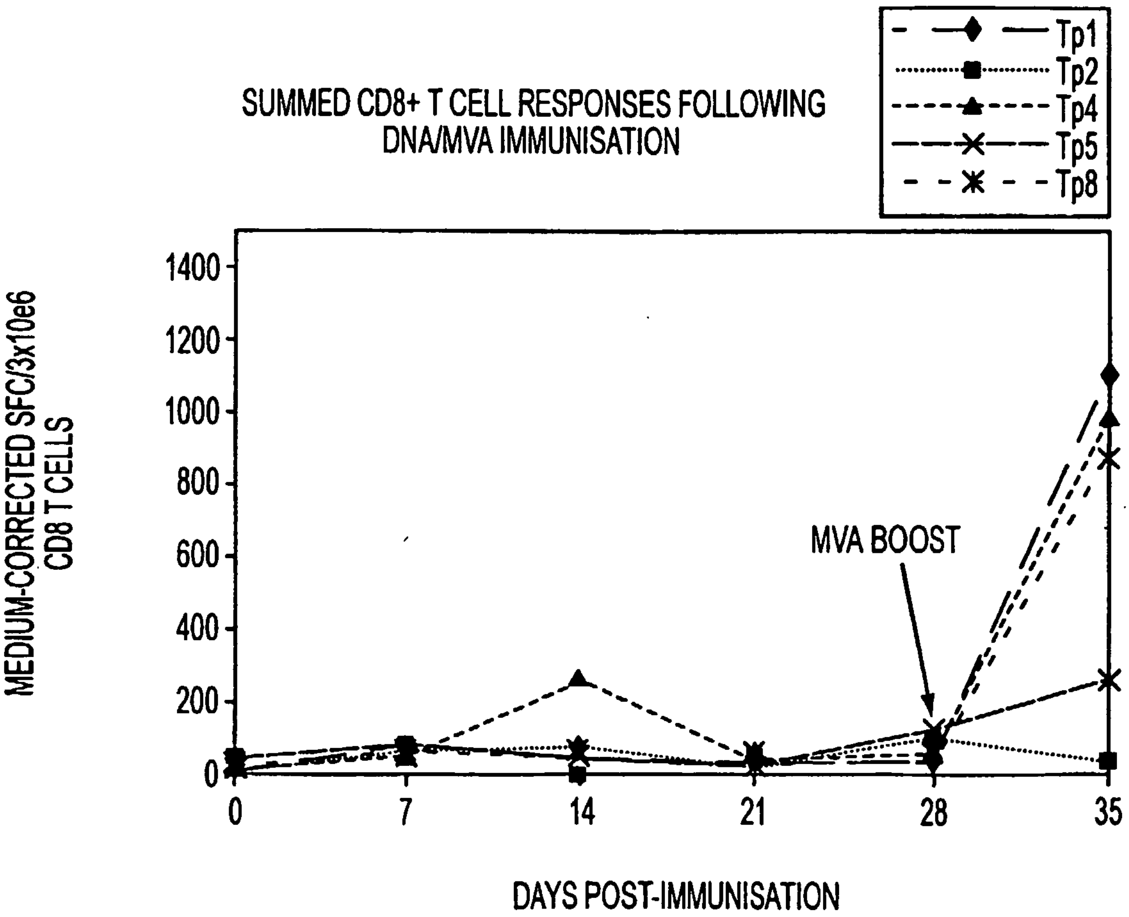
SUMMED CD8+ T CELL RESPONSES FOLLOWING DNA/MVA IMMUNISATION
Tp1
Tp2
Tp4
Tp5
Tp8
MEDIUM-CORRECTED SFC/3x10e6 CD8 T CELLS
1400
1200
1000
800
600
400
200
0
MVA BOOST
0
7
14
21
28
35
DAYS POST-IMMUNISATION

FIG. 19

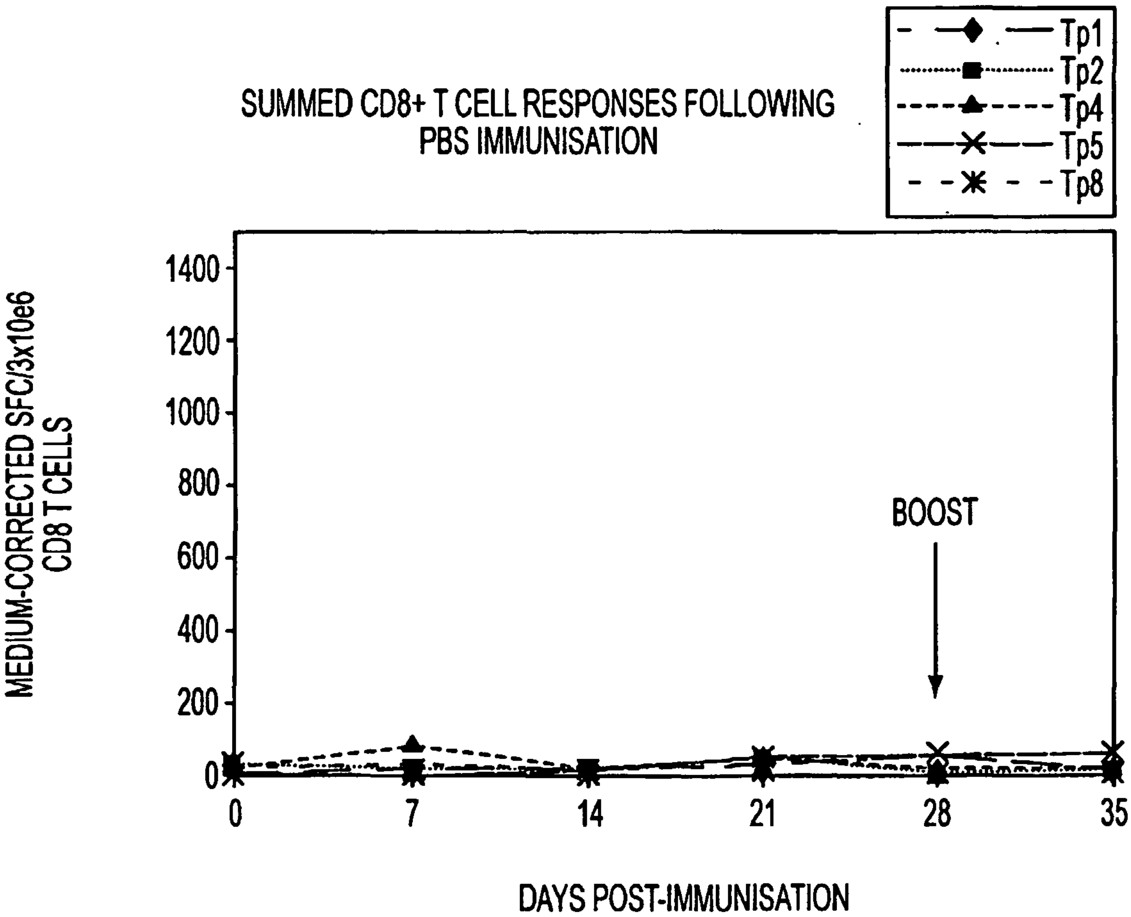
SUMMED CD8+ T CELL RESPONSES FOLLOWING
PBS IMMUNISATION
Tp1
Tp2
Tp4
Tp5
Tp8
MEDIUM-CORRECTED SFC/3x10e6
CD8 T CELLS
1400
1200
1000
800
600
400
200
0
BOOST
0
7
14
21
28
35
DAYS POST-IMMUNISATION

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5273744 A
- WO 9316094 A
- US 4683195 A
- US 4683202 A
- US 4800159 A, Saiki
- US 5837832 A, Chee
- WO 9511995 A, Chee
- US 5807522 A, Brown
- WO 95251116 A, Baldeschweiler
- US 60486750 B


### Non-patent literature cited in the description


- **HOLDER et al.** *Nature,* 1981, vol. 294, 361
- **MAGARIAN et al.** *J. Immunol,* 1984, vol. 132, 3131
- **LEW.** *Proc. Natl. Acad. Sci. USA,* 1989, vol. 86, 3768
- **SAMBROOK J. et al.** Molecular Cloning: A Laboratory Manual. Cold Spring Harbor Laboratory, 1989
- **AUSUBEL F. M et al.** Current Protocols in Molecular Biology. John Wiley & Sons, 1989
- **GOEDDEL.** Gene Expression Technology, Methods and Enzymology. Academic Press, 1990, vol. 185
- **KELLER, G. H. ; M. M. MANAK.** DNA Probes. Stockton Press, 1987, 169-170
- **GOBINDA et al.** *PCR Methods Applic,* 1993, vol. 2, 318-22
- **TRIGLIA T. et al.** *Nucleic Acids Res,* 1988, vol. 16, 8186
- **LAGERSTROM M. et al.** *PCR Methods Applic,* 1991, vol. 1, 111-19
- **PARKER J. D. et al.** *Nucleic Acids Res,* 1991, vol. 119, 3055-60
- **RUIZ-MARTINEZ M. C. et al.** *Anal Chem,* 1993, vol. 65, 2851-8
- **SAMBROOK et al.** Molecular Cloning: A Laboratory Manual. Cold Spring Harbor Laboratory Press, 1989
- **SMITH et al.** *Gene,* 1988, vol. 67, 31-40
- **AMANN et al.** *Gene,* 1988, vol. 69, 301-315
- **STUDIER et al.** *Gene Expression Technology: Methods in Enzymology,* 1990, vol. 185, 60-89
- **GOTTESMAN, S.** Gene Expression Technology: Methods in Enzymology. Academic Press, 1990, vol. 185, 119-128
- **WADA et al.** *Nucleic Acids Res.,* 1992, vol. 20, 2111-2118
- **BALDARI et al.** *EMBO J.,* 1987, vol. 6, 229-234
- **KUJAN et al.** *Cell,* 1982, vol. 30, 933-943
- **SCHULTZ et al.** *Gene,* 1987, vol. 54, 113-123
- **SMITH et al.** *Mol. Cell Biol.,* 1983, vol. 3, 2156-2165
- **LUCKLOW et al.** *Virology,* 1989, vol. 170, 31-39
- **SEED, B.** *Nature,* 1987, vol. 329, 840
- **KAUFINAN et al.** *EMBO J.,* 1987, vol. 6, 187-195
- **SAMBROOK, J. ; FRITSH, E. F. ; MANIATIS, T.** Molecular Cloning: A Laboratory Manual. Cold Spring Harbor Laboratory Press, 1989
- **SAMBROOK et al.** Molecular Cloning: A Laboratory Manual. Cold Spring Harbor Laboratory, Cold Spring Harbor Laboratory Press, 1989
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **CUNMINGHAM ; WELLS.** *Science,* 1989, vol. 244, 1081-1085
- **CREIGHTON.** The Proteins. W.H. Freeman & Co,
- **CHOTHIA.** *J. Mol. Biol.,* 1976, vol. 150, 1
- Proteins-Structure and Molecular Properties. W. H. Freeman and Company, 1993, vol. 2
- **WOLD, F.** Posttranslational Covalent Modification of Proteins. Academic Press, 1983, 1-12
- **SEIFTER et al.** *Meth Enzymol.,* 1990, vol. 182, 626-646
- **RATTAN et al.** *Ann. N.Y Acad. Sci.,* 1992, vol. 653, 48-62
- **ORLANDI R et al.** *PNAS,* 1989, vol. 86, 3833-3837
- **HUSE W. D. et al.** *Science,* 1989, vol. 256, 1275-1281
- **WINTER G. ; MILSTEIN C.** *Nature,* 1991, vol. 349, 293-299

- **HARLOW.** Antibodies. Cold Spring Harbor Press, 1989
- Molecular Cloning: A Laboratory Manual. Cold Spring Harbor Laboratory Press, 1989
- Methods in Enzymology: Guide to Molecular Cloning Techniques. Academic Press, 1987
- **LOCKHART, D. J. et al.** *Nat. Biotech.,* 1996, vol. 1.4, 1675-1680
- **SCHENA, M. et al.** *Proc. Natl. Acad. Sci.,* 1996, vol. 93, 10614-10619
- **CHARD, T.** An Introduction to Radioimmunoassay and Related Techniques. Elsevier Science Publishers, 1986
- **BULLOCK, G. R. et al.** Techniques in Immunocytochemistry. Academic Press, 1982, vol. 1
- TECHNIQUES IN IMMUNOCYTOCHEMISTRY. 1983, vol. 2
- TECHNIQUES IN IMMUNOCYTOCHEMISTRY. 1985, vol. 3
- **TIJSSEN, P.** Practice and Theory of Enzyme Immunoassays: Laboratory Techniques in Biochemistry and Molecular Biology. Elsevier Science Publishers, 1985

SEQUENCE LISTING

<110> INTERNATIONAL LIVESTOCK RESEARCH INSTITUTE

<120> EAST COAST FEVER VACCINE BASED ON CTL-SPECIFIC SCHIZONT ANTIGENS

<130> 41860-205200

<140>
<141>

<150> 60/486,750
<151> 2003-07-14

<160> 77

<170> PatentIn Ver. 3.2

<210> 1
<211> 543

<212> PRT
<213> Theileria parva

<400> 1

```
Met Arg Val Lys Lys Val Leu Leu Tyr Thr Leu Pro Val Val Gly Ile
 1               5                  10                  15

Leu Leu Ala Gly Ser Leu Ile Ile Phe Asn Phe Val Arg Lys Arg Pro
            20                  25                  30

Glu Lys Glu Glu Glu Leu Lys Pro Pro Ser Ala Leu Glu Asp Glu Leu
        35                  40                  45

Lys Lys Arg Glu Glu Glu Ser Arg Lys Arg Met Glu Glu Met Gln Lys
    50                  55                  60

Glu Ile Leu Glu Lys Lys Leu Arg Glu Gly Lys Lys Ala Leu Glu Glu
65                  70                  75                  80

Leu Glu Lys Arg Glu Lys Glu Val Val Asp Glu Phe Ala Lys His Leu
                85                  90                  95

Lys Lys Pro Glu Glu Arg Leu Pro Lys Ile Ile Leu Thr Leu Asp Ser
            100                 105                 110

Gly Phe Pro Thr Val Asp Pro Ile Thr Tyr Thr Ser Gly Val Tyr Met
        115                 120                 125

Val Ala Val Ser Lys Thr Thr Phe Thr Ser Asp Ser Asp Leu Val Asp
    130                 135                 140

Phe Thr His Thr Leu Leu Gly Ile Lys Phe Leu Val Thr Gly Val Gln
145                 150                 155                 160

Phe Gly Gly Lys Thr Tyr Thr Ile Lys Pro Ile Glu Ala Thr Met Ala
                165                 170                 175
```

Thr Ser Ile Ala Phe Ala Ala Asp Pro Gly Phe Cys Tyr Phe Leu Leu
180 185 190

Ile Pro Gly Pro Asp Ser Lys Pro Ile Phe Phe Lys Asn Asp Gly Asp
195 200 205

Lys Phe Leu Arg Cys Val Gly Tyr Pro Lys Val Lys Glu Glu Met Leu
210 215 220

Glu Met Ala Thr Lys Phe Asn Arg Leu Pro Lys Gly Val Glu Ile Pro
225 230 235 240

Ala Pro Pro Gly Val Lys Pro Glu Ala Pro Thr Pro Thr Pro Thr Thr
245 250 255

Ile Thr Pro Ser Val Pro Pro Thr Ile Pro Thr Pro Ile Thr Pro Ser
260 265 270

Ala Pro Pro Thr Thr Pro Pro Thr Gly Leu Asn Phe Asn Leu Thr Val
275 280 285

Gln Asn Lys Phe Met Ile Gly Ser Gln Glu Val Lys Leu Asn Ile Thr
290 295 300

His Glu Tyr Glu Gly Val Tyr Glu Ala His Lys Tyr Phe Ile Glu Arg
305 310 315 320

Gly Ser Phe Thr Pro Thr Ser Phe Ser Ile Gly Asp Leu Pro Gln Thr
325 330 335

Gly Leu Pro Val Asn Gln Thr Val Asp Thr Ile Val Val Tyr Phe His
340 345 350

Arg Val Thr Met Gly Glu Pro Val Gly Ile Pro Leu Ile Val Leu Ile
355 360 365

Phe Tyr Lys Asn Gln Ser Arg Lys Tyr Leu Asn Lys Gly Asn Gly Asn
370 375 380

Trp Glu Glu Ser Lys Ala Leu Leu Phe Arg Glu Glu Leu Asp Tyr Leu
385 390 395 400

Asp Ser Ile Phe Asn Asp Phe Val Thr Val Asn Leu Ser Arg Arg Ser
405 410 415

Asp Tyr Tyr Arg Asn Gly Thr Gly Thr Ser Glu Ile Glu Gln Thr Leu
420 425 430

Asp Met Asn Val Tyr Val Glu Pro Asp Thr Pro Cys Ala Gly Trp Thr
435 440 445

Thr Tyr Ile His Lys Leu Glu Glu Gly Gly Glu Gly Gly Ile Glu Lys
450 455 460

Pro Phe Gln Ile Arg Gln Leu Trp Phe Ser Lys Gln Lys Phe Asp Ile
465 470 475 480

Phe Pro Met Gly Lys Val Ser Ile Val Asn Val Tyr Gly Lys Asn Asp
485 490 495

Glu Pro Leu Ser Tyr Ala Pro Ser Ile Phe Ser Val Ile Arg Glu Asp
500 505 510

Gly Ile Gln Ile Phe Tyr Val Arg Ala Tyr Ser Gln Tyr Leu Leu Asp
515 520 525

Ser Ser Val Asn Pro Gln Asn Leu Pro Gln Lys Leu Asn Thr Leu
530 535 540

<210> 2
<211> 579
<212> PRT
<213> Theileria parva

<400> 2

Met Ser His Leu Met Asn Leu Pro Ile Leu Val Leu Lys Glu Gly Thr
1 5 10 15

Asp Thr Ser Gln Gly Gln Ala Gln Ile Ile Ser Asn Ile Asn Ala Cys
20 25 30

Gln Ala Ile Val Asp Cys Val Lys Thr Thr Leu Gly Pro Arg Gly Met
35 40 45

Asp Lys Leu Ile His Thr Glu Arg Asp Val Thr Ile Thr Asn Asp Gly
50 55 60

Ala Thr Val Leu Lys Leu Leu Asp Ile Thr His Pro Ala Ala Ser Val
65 70 75 80

Leu Val Asp Ile Ala Lys Ser Gln Asp Asp Glu Val Gly Asp Gly Thr
85 90 95

Thr Ser Val Thr Val Leu Ala Gly Glu Leu Leu Asn Glu Ala Lys Ala
100 105 110

Phe Ile Leu Asp Gly Ile Ser Pro Gln Val Ile Ile Lys Tyr Tyr Arg
115 120 125

Glu Ala Cys Gln Val Ala Leu Asn Leu Ile Asp Lys Val Ala Ile His
130 135 140

Leu Ser Asn Lys Ser Ser Thr Asp Lys Lys Glu Leu Leu Ile Lys Cys
145 150 155 160

Ala Glu Thr Thr Phe Asn Ser Lys Leu Leu Ser Gly Tyr Lys Thr Phe
165 170 175

Phe Ala Lys Met Val Val Glu Ala Val Ala Thr Leu Asp Glu Asp Leu
180 185 190

```
Asp Glu Asp Met Ile Gly Val Lys Lys Val Thr Gly Gly Ser Cys Glu
        195                 200                 205

Asp Ser Leu Leu Val Lys Gly Val Ala Phe Lys Lys Thr Phe Ser Tyr
    210                 215                 220

Ala Gly Ala Glu Gln Gln Pro Lys Lys Phe Val Asn Pro Lys Ile Leu
225                 230                 235                 240

Leu Leu Asn Leu Glu Leu Glu Leu Lys Ser Glu Lys Glu Asn Ala Glu
                245                 250                 255

Ile Val Ile Asn Asn Pro Gln Glu Tyr Gln Lys Ile Ile Asp Ala Glu
            260                 265                 270

Tyr Arg Ile Ile Phe Glu Lys Leu Glu Asn Ala Val Lys Leu Gly Ala
        275                 280                 285

Asn Val Val Leu Ser Lys Leu Pro Ile Gly Asp Leu Ala Thr Gln Tyr
    290                 295                 300

Phe Ala Asp Lys Asn Val Phe Cys Ala Gly Arg Val Asp Glu Asn Asp
305                 310                 315                 320

Leu Ile Arg Thr Ser Lys Ala Thr Gly Ala Ser Ile Gln Thr Thr Leu
                325                 330                 335

Asn Asn Leu Ser Val Asp Val Leu Gly Thr Cys Gly Val Phe Glu Glu
            340                 345                 350

Val Gln Ile Gly Ser Glu Arg Tyr Asn Met Phe Thr Asp Cys Lys Ser
        355                 360                 365

Ala Lys Thr Cys Thr Ile Val Leu Arg Gly Gly Gly Gln Gln Phe Ile
    370                 375                 380

Asp Glu Ser Glu Arg Ser Leu His Asp Ala Ile Met Ile Val Arg Arg
385                 390                 395                 400

Ala Thr Lys Cys Asn Thr Ile Leu Pro Gly Ala Gly Ala Ile Glu Met
                405                 410                 415

Leu Leu Ser Thr Tyr Leu Leu His Tyr Ser Leu Asn Thr Ile Asn Pro
            420                 425                 430

Thr Asp Ser Val Asn His Val Asn Cys Val Asn Ser Val Asn His Val
        435                 440                 445

Asn Gly Val Thr Gly Val Asn Lys Ser Leu Val Gly Lys Arg His Ile
    450                 455                 460

Ile Met Asn Gly Phe Ala Lys Ala Leu Glu Cys Ile Pro Arg Asn Leu
465                 470                 475                 480

Ala Thr Asn Ser Gly Tyr Asn Ser Asn Asp Leu Leu Ser Ile Leu Arg
                485                 490                 495
```

Asn Lys Tyr Asn Gln Leu Glu Ile Val Asn Gly Glu Ile Lys Val Asn
500 505 510

Asn Glu Glu Ser Trp Tyr Gly Ile Asp Cys Tyr Lys Gly Ser Val Cys
515 520 525

Asn Ala Tyr Lys Ala Cys Ile Trp Glu Pro Ser Leu Val Lys Lys Asn
530 535 540

Ser Ile Tyr Ser Ala Thr Glu Ala Ala Cys Leu Val Leu Ser Val Asp
545 550 555 560

Glu Thr Val Lys Asn Gln Ser Arg Gln Gln Leu Gln Ser Ala Leu Pro
565 570 575

Gln Pro Lys

<210> 3
<211> 155
<212> PRT
<213> Theileria parva

<400> 3

Met Pro Lys Asn Lys Gly Lys Gly Gly Lys Asn Arg Arg Arg Gly Lys
1 5 10 15

Asn Asp Asn Glu Gly Glu Lys Arg Glu Leu Val Phe Lys Met Glu Asp
20 25 30

Gln Glu Tyr Ala Gln Val Leu Arg Met Leu Gly Asn Gly Arg Leu Glu
35 40 45

Ala Tyr Cys Phe Asp Gly Thr Lys Arg Leu Cys His Ile Arg Gly Lys
50 55 60

Met Arg Lys Arg Val Trp Val Asn Ala Gly Asp Ile Ile Leu Val Ser
65 70 75 80

Leu Arg Asp Phe Gln Asp Ser Lys Ala Asp Val Ile Ala Lys Tyr Thr
85 90 95

Ala Glu Glu Ala Arg Thr Leu Lys Ala Tyr Gly Glu Leu Pro Glu Ala
100 105 110

Thr Lys Ile Asn Glu Thr Asp Val Tyr Asp Asp Glu Ala Asp Asn Cys
115 120 125

Ile Asp Phe Gln Asp Val Ser Ser Glu Ser Glu Pro Glu Asp Glu Ser
130 135 140

Gln Glu Glu Ser Asp Phe Asp Ile Asp Asp Leu
145 150 155

<210> 4
<211> 721
<212> PRT
<213> Theileria parva

<400> 4

Met Thr Ser Lys Asp Glu Thr Pro Asp Gln Glu Val Tyr Ala Phe Asn
1               5                   10                  15

Ala Asp Ile Ser Gln Leu Leu Ser Leu Ile Ile Asn Ala Phe Tyr Ser
            20                  25                  30

Asn Lys Glu Ile Phe Leu Arg Glu Leu Ile Ser Asn Ala Ser Asp Ala
        35                  40                  45

Leu Glu Lys Ile Arg Tyr Glu Ala Ile Lys Asp Pro Lys Gln Ile Glu
    50                  55                  60

Asp Gln Pro Asp Tyr Tyr Ile Arg Leu Tyr Ala Asp Lys Asn Asn Asn
65                  70                  75                  80

Thr Leu Thr Ile Glu Asp Ser Gly Ile Gly Met Thr Lys Ala Asp Leu
                85                  90                  95

Val Asn Asn Leu Gly Thr Ile Ala Lys Ser Gly Thr Arg Ala Phe Met
            100                 105                 110

Glu Ala Leu Gln Ala Gly Ser Asp Met Ser Met Ile Gly Gln Phe Gly
        115                 120                 125

Val Gly Phe Tyr Ser Ala Tyr Leu Val Ala Asp Lys Val Thr Val Val
    130                 135                 140

Ser Lys Asn Asn Ala Asp Asp Gln Tyr Val Trp Glu Ser Thr Ala Ser
145                 150                 155                 160

Gly His Phe Thr Val Lys Lys Asp Asp Ser His Glu Pro Leu Lys Arg
                165                 170                 175

Gly Thr Arg Leu Ile Leu His Leu Lys Glu Asp Gln Thr Glu Tyr Leu
            180                 185                 190

Glu Glu Arg Arg Leu Lys Glu Leu Val Lys Lys His Ser Glu Phe Ile
        195                 200                 205

Ser Phe Pro Ile Ser Leu Ser Val Glu Lys Thr Gln Glu Thr Glu Val
    210                 215                 220

Thr Asp Asp Glu Ala Glu Leu Asp Glu Asp Lys Lys Pro Glu Glu Glu
225                 230                 235                 240

Lys Pro Lys Asp Asp Lys Val Glu Asp Val Thr Asp Glu Lys Val Thr
                245                 250                 255

Asp Val Thr Asp Glu Glu Glu Lys Lys Glu Glu Lys Lys Lys Lys Lys
            260                 265                 270

Arg Lys Val Thr Asn Val Thr Arg Glu Trp Glu Met Leu Asn Lys Gln
275 280 285

Lys Pro Ile Trp Met Arg Leu Pro Ser Glu Val Thr Asn Glu Glu Tyr
290 295 300

Ala Ala Phe Tyr Lys Asn Leu Thr Asn Asp Trp Glu Asp His Leu Ala
305 310 315 320

Val Lys His Phe Ser Val Glu Gly Gln Leu Glu Phe Lys Ala Leu Leu
325 330 335

Phe Val Pro Arg Arg Ala Pro Phe Asp Met Phe Glu Ser Arg Lys Lys
340 345 350

Lys Asn Asn Ile Lys Leu Tyr Val Arg Arg Val Phe Ile Met Asp Asp
355 360 365

Cys Glu Glu Leu Ile Pro Glu Trp Leu Ser Phe Val Lys Gly Val Val
370 375 380

Asp Ser Glu Asp Leu Pro Leu Asn Ile Ser Arg Glu Thr Leu Gln Gln
385 390 395 400

Asn Lys Ile Leu Lys Val Ile Arg Lys Asn Leu Val Lys Lys Cys Leu
405 410 415

Glu Leu Phe Asn Glu Leu Thr Glu Lys Lys Glu Asp Phe Lys Lys Phe
420 425 430

Tyr Glu Gln Phe Ser Lys Asn Leu Lys Leu Gly Ile His Glu Asp Asn
435 440 445

Ala Asn Arg Ser Lys Ile Ala Glu Leu Leu Arg Phe Glu Thr Thr Lys
450 455 460

Ser Gly Asp Glu Leu Val Ser Leu Lys Glu Tyr Val Asp Arg Met Lys
465 470 475 480

Ser Asp Gln Lys Tyr Val Tyr Tyr Ile Thr Gly Glu Ser Lys Gln Ser
485 490 495

Val Ala Ser Ser Pro Phe Leu Glu Thr Leu Arg Ala Arg Asp Tyr Glu
500 505 510

Val Leu Tyr Met Thr Asp Pro Ile Asp Glu Tyr Ala Val Gln Gln Ile
515 520 525

Lys Glu Phe Glu Gly Lys Lys Leu Lys Cys Cys Thr Lys Glu Gly Leu
530 535 540

Asp Leu Asp Glu Gly Glu Asp Glu Lys Lys Ser Phe Glu Ala Leu Lys
545 550 555 560

Glu Glu Met Glu Pro Leu Cys Lys His Ile Lys Glu Val Leu His Asp
565 570 575

Lys Val Glu Lys Val Val Cys Gly Thr Arg Phe Thr Asp Ser Pro Cys
580 585 590

Ala Leu Val Thr Ser Glu Phe Gly Trp Ser Ala Asn Met Glu Arg Ile
595 600 605

Met Lys Ala Gln Ala Leu Arg Asp Ser Ser Ile Thr Ser Tyr Met Leu
610 615 620

Ser Lys Lys Ile Met Glu Ile Asn Pro Arg His Ser Ile Met Lys Glu
625 630 635 640

Leu Lys Thr Arg Ala Ala Asn Asp Lys Thr Asp Lys Thr Val Lys Asp
645 650 655

Leu Val Trp Leu Leu Tyr Asp Thr Ala Leu Leu Thr Ser Gly Phe Asn
660 665 670

Leu Asp Glu Pro Thr Gln Phe Gly Asn Arg Ile Tyr Arg Met Ile Lys
675 680 685

Leu Gly Leu Ser Leu Asp Asp Glu Glu His Val Glu Glu Asp Ser Ser
690 695 700

Met Pro Pro Leu Asp Glu Pro Val Val Asp Ser Lys Met Glu Glu Val
705 710 715 720

Asp

<210> 5
<211> 440
<212> PRT
<213> Theileria parva

<400> 5

Met Leu Gly Asn His Val Met Gly Ser Asn Ser Pro His Ile Lys Ile
1 5 10 15

Leu Ser Ser Val Thr Phe Leu His Ile Ala Lys Met Glu Glu Val Glu
20 25 30

Asn Val Lys Val Asp Ala Leu Glu Arg Val Asp Thr Glu Ser Val Leu
35 40 45

Asn Tyr Asp Thr Val Leu Glu Lys Lys Pro Leu Arg Ser Ser Val Ala
50 55 60

Ser Phe Phe Lys Arg Tyr Ser Ala Val Leu Val Ile Leu Thr Ala Val
65 70 75 80

Leu Leu Phe Thr Phe Thr Phe Ala Ala Ile Ala Leu Ser Ser Gly Arg
85 90 95

Ser Ala Ile Arg Lys Asn Arg Glu Leu Leu Ser Val Glu Phe Glu Lys
100 105 110

Leu Gln Phe Asp Asn Phe Val Thr Ile Lys Gly Glu Arg Glu Glu Asp
115 120 125

Phe Pro Lys Met Val Ala Glu Val Leu Tyr Lys Val Ala Val Glu Phe
130 135 140

Asp Pro Lys Glu Glu Ala Leu Ile Tyr Val Gln Phe Asn Asp Phe Asn
145 150 155 160

Lys Gln His Asp Lys Lys His Asn Asn Tyr Arg His Lys Lys Thr Ser
165 170 175

Tyr Thr Asn Phe Arg Asn Asn Leu Asn Asp Ile Asn Glu His Asn Ala
180 185 190

Lys Pro Asn Leu Ser Tyr Thr Lys Asn Met Asn His Phe Gly Asp Ile
195 200 205

Ser Ser Lys Asp Phe Met Lys Arg Tyr Thr Lys Lys Val Leu Leu Asn
210 215 220

Leu Pro Lys Asp His Val Ser Thr Tyr Asn Asn Asn Arg Pro Met Ser
225 230 235 240

Val Asp Leu Arg Ser His Gly Val Leu Thr Pro Val Lys Cys Gln Glu
245 250 255

Glu Asn Glu Leu Ser Trp Pro Tyr Ser Val Val Ala Val Ala Glu Ser
260 265 270

Phe Val Lys Lys Thr Ser Gln Lys Thr Val Ser Leu Ser Glu Lys Gln
275 280 285

Leu Val Asp Cys Val Thr Asp Lys Lys Ser Ala Asn Asn Pro Phe Leu
290 295 300

Gly Tyr Lys Tyr Leu Lys Asp Leu Gly Leu Phe Glu Ser Glu Leu Val
305 310 315 320

Asp Lys Ser Thr Thr Lys Cys Pro Ala Leu Glu Gly Glu Arg Phe Lys
325 330 335

Val Pro Ser Tyr Ser Tyr Ser Tyr Glu Pro Asp Leu Val Ala Leu Leu
340 345 350

Leu Asn Ala Gly Pro Leu Thr Val Pro Val Ala Val Ser Glu Asp Trp
355 360 365

Gln Phe Tyr Ala Asp Gly Thr Leu Asp Val Cys Gly Ala Glu Leu Asn
370 375 380

His Phe Leu Thr Leu Val Gly Val Ser Phe Asp Glu Lys Gly Asn His
385 390 395 400

Trp Ile Leu Lys Asn Ser Phe Gly Glu Gly Trp Gly Asn Lys Gly Tyr
405 410 415

Leu Leu Leu Thr Arg Asn Ser Lys Glu Tyr Lys Asp Asp Cys Gly Leu
420 425 430

Thr Ser Phe Ala Val Tyr Ala Val
435 440

<210> 6
<211> 543
<212> PRT
<213> Theileria parva

<400> 6

```
Met Arg Val Lys Lys Val Leu Leu Tyr Thr Leu Pro Val Val Gly Ile
1               5                   10                  15

Leu Leu Ala Gly Ser Leu Ile Ile Phe Asn Phe Val Arg Lys Arg Pro
            20                  25                  30

Glu Lys Glu Glu Glu Leu Lys Pro Pro Ser Ala Leu Glu Asp Glu Leu
        35                  40                  45

Lys Lys Arg Glu Glu Glu Ser Arg Lys Arg Met Glu Glu Met Gln Lys
    50                  55                  60

Glu Ile Leu Glu Lys Lys Leu Arg Glu Gly Lys Lys Ala Leu Glu Glu
65                  70                  75                  80

Leu Glu Lys Arg Glu Lys Glu Val Val Asp Glu Phe Ala Lys His Leu
                85                  90                  95

Lys Lys Pro Glu Glu Arg Leu Pro Lys Ile Ile Leu Thr Leu Asp Ser
            100                 105                 110

Gly Phe Pro Thr Val Asp Pro Ile Thr Tyr Thr Ser Gly Val Tyr Met
        115                 120                 125

Val Ala Val Ser Lys Thr Thr Phe Thr Ser Asp Ser Asp Leu Val Asp
    130                 135                 140

Phe Thr His Thr Leu Leu Gly Ile Lys Phe Leu Val Thr Gly Val Gln
145                 150                 155                 160

Phe Gly Gly Lys Thr Tyr Thr Ile Lys Pro Ile Glu Ala Thr Met Ala
                165                 170                 175

Thr Ser Ile Ala Phe Ala Ala Asp Pro Gly Phe Cys Tyr Phe Leu Leu
            180                 185                 190

Ile Pro Gly Pro Asp Ser Lys Pro Ile Phe Phe Lys Asn Asp Gly Asp
        195                 200                 205

Lys Phe Leu Arg Cys Val Gly Tyr Pro Lys Val Lys Glu Glu Met Leu
    210                 215                 220
```

Glu Met Ala Thr Lys Phe Asn Arg Leu Pro Lys Gly Val Glu Ile Pro
225 230 235 240

Ala Pro Pro Gly Val Lys Pro Glu Ala Pro Thr Pro Thr Pro Thr Thr
245 250 255

Ile Thr Pro Ser Val Pro Pro Thr Ile Pro Thr Pro Ile Thr Pro Ser
260 265 270

Ala Pro Pro Thr Thr Pro Pro Thr Gly Leu Asn Phe Asn Leu Thr Val
275 280 285

Gln Asn Lys Phe Met Ile Gly Ser Gln Glu Val Lys Leu Asn Ile Thr
290 295 300

His Glu Tyr Glu Gly Val Tyr Glu Ala His Lys Tyr Phe Ile Glu Arg
305 310 315 320

Gly Ser Phe Thr Pro Thr Ser Phe Ser Ile Gly Asp Leu Pro Gln Thr
325 330 335

Gly Leu Pro Val Asn Gln Thr Val Asp Thr Ile Val Val Tyr Phe His
340 345 350

Arg Val Thr Met Gly Glu Pro Val Gly Ile Pro Leu Ile Val Leu Ile
355 360 365

Phe Tyr Lys Asn Gln Ser Arg Lys Tyr Leu Asn Lys Gly Asn Gly Asn
370 375 380

Trp Glu Glu Ser Lys Ala Leu Leu Phe Arg Glu Glu Leu Asp Tyr Leu
385 390 395 400

Asp Ser Ile Phe Asn Asp Phe Val Thr Val Asn Leu Ser Arg Arg Ser
405 410 415

Asp Tyr Tyr Arg Asn Gly Thr Gly Thr Ser Glu Ile Glu Gln Thr Leu
420 425 430

Asp Met Asn Val Tyr Val Glu Pro Asp Thr Pro Cys Ala Gly Trp Thr
435 440 445

Thr Tyr Ile His Lys Leu Glu Glu Gly Gly Glu Gly Gly Ile Glu Lys
450 455 460

Pro Phe Gln Ile Arg Gln Leu Trp Phe Ser Lys Gln Lys Phe Asp Ile
465 470 475 480

Phe Pro Met Gly Lys Val Ser Ile Val Asn Val Tyr Gly Lys Asn Asp
485 490 495

Glu Pro Leu Ser Tyr Ala Pro Ser Ile Phe Ser Val Ile Arg Glu Asp
500 505 510

Gly Ile Gln Ile Phe Tyr Val Arg Ala Tyr Ser Gln Tyr Leu Leu Asp
515 520 525

Ser Ser Val Asn Pro Gln Asn Leu Pro Gln Lys Leu Asn Thr Leu
530 535 540

<210> 7
<211> 241
<212> PRT

<213> Theileria parva

<400> 7

```
Met Arg Val Lys Lys Val Leu Leu Tyr Thr Leu Pro Val Val Gly Ile
1               5                   10                  15

Leu Leu Ala Gly Ser Leu Ile Ile Phe Asn Phe Val Arg Lys Arg Pro
            20                  25                  30

Glu Lys Glu Glu Glu Leu Lys Pro Pro Ser Ala Leu Glu Asp Glu Leu
        35                  40                  45

Lys Lys Arg Glu Glu Glu Ser Arg Lys Arg Met Glu Glu Met Gln Lys
    50                  55                  60

Glu Ile Leu Glu Lys Lys Leu Arg Glu Gly Lys Lys Ala Leu Glu Glu
65                  70                  75                  80

Leu Glu Lys Arg Glu Lys Glu Val Val Asp Glu Phe Ala Lys His Leu
                85                  90                  95

Lys Lys Pro Glu Glu Arg Leu Pro Lys Ile Ile Leu Thr Leu Asp Ser
            100                 105                 110

Gly Phe Pro Thr Val Asp Pro Ile Thr Tyr Thr Ser Gly Val Tyr Met
        115                 120                 125

Val Ala Val Ser Lys Thr Thr Phe Thr Ser Asp Ser Asp Leu Val Asp
    130                 135                 140

Phe Thr His Thr Leu Leu Gly Ile Lys Phe Leu Val Thr Gly Val Gln
145                 150                 155                 160

Phe Gly Gly Lys Thr Tyr Thr Ile Lys Pro Ile Glu Ala Thr Met Ala
                165                 170                 175

Thr Ser Ile Ala Phe Ala Ala Asp Pro Gly Phe Cys Tyr Phe Leu Leu
            180                 185                 190

Ile Pro Gly Pro Asp Ser Lys Pro Ile Phe Phe Lys Asn Asp Gly Asp
        195                 200                 205

Lys Phe Leu Arg Cys Val Gly Tyr Pro Lys Val Lys Glu Glu Met Leu
    210                 215                 220

Glu Met Ala Thr Lys Phe Asn Arg Leu Pro Lys Gly Val Glu Ile Pro
225                 230                 235                 240

Ala
```

<210> 8
<211> 14
<212> PRT
<213> Theileria parva

<400> 8

```
Arg Cys Val Gly Tyr Pro Lys Val Lys Glu Glu Met Leu Glu
1               5                   10
```

<210> 9

<211> 11
<212> PRT
<213> Theileria parva

<400> 9

Val Gly Tyr Pro Lys Val Lys Glu Glu Met Leu
1 5 10

<210> 10
<211> 11
<212> PRT
<213> Theileria parva

<400> 10

Ser His Glu Glu Leu Lys Lys Leu Gly Met Leu
1 5 10

<210> 11
<211> 11
<212> PRT
<213> Theileria parva

<400> 11

Lys Ser Ser His Gly Met Gly Lys Val Gly Lys
1 5 10

<210> 12
<211> 9
<212> PRT
<213> Theileria parva

<400> 12

Phe Ala Gln Ser Leu Val Cys Val Leu
1 5

<210> 13
<211> 9
<212> PRT
<213> Theileria parva

<400> 13

Gln Ser Leu Val Cys Val Leu Met Lys
1 5

<210> 14
<211> 9
<212> PRT
<213> Theileria parva

<400> 14

Thr Gly Ala Ser Ile Gln Thr Thr Leu
1 5

<210> 15
<211> 9
<212> PRT
<213> Theileria parva

<400> 15

Ser Lys Ala Asp Val Ile Ala Lys Tyr
1 5

<210> 16
<211> 9
<212> PRT
<213> Theileria parva

<400> 16

Glu Phe Ile Ser Phe Pro Ile Ser Leu
1 5

<210> 17
<211> 9
<212> PRT
<213> Theileria parva

<400> 17

Cys Gly Ala Glu Leu Asn His Phe Leu
1 5

<210> 18
<211> 1807
<212> DNA
<213> Theileria parva

<400> 18

aaggttaagt atagattttt tgtgactttt atttacttac ctgtttgtat aaaattataa 60
ggttataaaa tttaaccaaa taaccgattt aattgtaata tttaaagttt tgtacagtat 120
atgagggtca aaaaagtttt attatataca cttccggttg tcggtatttt actggctgga 180
tctttgatta tatttaattt cgttaggaaa agaccggaaa aagaagagga actcaaacct 240
ccttctgcat tagaagatga acttaaaaaa cgtgaagaag aaagccgaaa acgcatggaa 300
gaaatgcaaa aggaaattct cgaaaaaaag ttaagagaag gtaaaaaagc cttggaagaa 360

```
cttgaaaaac gtgaaaaaga agtggtagat gagtttgcaa aacacctcaa aaaacctgaa 420
gaaagacttc ctaaaattat tcttacattg gattccggtt ttccaacagt tgatcctatt 480
acatatactt caggagttta tatggtagca gttagtaaaa caacttttac ctcagattca 540
gatcttgttg attttactca cacactgctg ggcataaagt ttctagttac tggtgtacaa 600
tttggtggga aaacatacac aattaaaccg attgaagcta ctatggccac ttcaattgca 660
tttgccgctg atcctggatt ctgttatttt ctattaatac caggccctga ctcgaaacca 720
atattcttca aaaacgacgg agataaattt ttacgttgcg tagggtatcc aaaggttaaa 780
gaagaaatgc tagaaatggc tacaaaattc aatagactac caaagggcgt ggaaatacct 840
gcacctccag gagtaaaacc agaggctccc acacctacac caacgacaat aactccttct 900
gtacctccta ctataccaac gccaataact ccttcggcac ctcctactac accacctacg 960
ggactaaatt ttaacttgac agttcagaac aaattcatga taggttcgca agaagttaag 1020
ttaaatataa ctcacgaata cgagggtgta tacgaagctc ataaatattt cattgaaagg 1080
ggcagcttta cccctacctc attctcaatc ggtgatcttc cacaaacagg tcttccagta 1140
aatcaaacag tggatacaat tgtggtatat ttccatcgag taacgatggg tgaacccgtt 1200
ggtatacctc taattgtgtt aatcttttat aaaaaccaat ctagaaaata tttaaataag 1260
ggaaatggaa actgggaaga atctaaagct ctattatttc gtgaggaact tgattactta 1320
gattccattt ttaatgattt tgtgacagta aacctttcta gacgttctga ttattatcgt 1380
aatggaactg gcacatcaga aattgagcaa acgttagata tgaatgttta cgttgaacct 1440
gatacaccct gtgctggatg gacaacgtat atacataaat tagaagaagg aggtgaagga 1500
ggaattgaaa aaccttttca aattagacaa ttatggttta gtaaacagaa atttgatata 1560
ttcccaatgg gaaaagtttc aatcgttaat gtttatggga aaaacgatga accgctatct 1620
tacgctccct caattttcag tgtaatacgc gaagatggaa ttcaaatatt ttatgttcgt 1680
gcttactcac agtacttgct tgattcaagt gttaatcccc aaaatttacc tcaaaaatta 1740
aacacccttt agattttttt taaaaaaatc atgtaatata attgtttttg aaaaaaaaaa 1800
aaaaaaa                                                           1807
```

<210> 19
<211> 1740
<212> DNA
<213> Theileria parva

<400> 19

```
atgagtcatt taatgaacct accaatcctt gtattgaagg aaggcactga tacatcccaa 60
ggccaagctc aaatcattag taatatcaac gcctgtcaag ctattgtcga ttgtgttaaa 120
actactctag gtcctagagg aatggacaag ttgatacata cggagagaga tgtgacgata 180
accaatgacg gtgctactgt tttgaaatta cttgatatta ctcatcctgc cgcttctgtt 240
cttgttgata tcgctaaatc acaagatgat gaagtcggtg atgggactac ttccgttact 300
gttctagcag gtgagttatt gaatgaagct aaggcgttta tattggatgg gataagtcct 360
caggttatca taaaatacta tcgtgaagcc tgtcaagttg ctttaaatct cattgataaa 420
gttgccattc atctctccaa caaatcctca actgataaga aagaactact gataaaatgt 480
gctgaaacta cttttaattc aaagttattg tctggttata aaaccttttt tgccaagatg 540
gttgtggagg cagtggctac tttggatgag gacttggatg aggatatgat tggtgttaaa 600
aaagtcactg gtggttcctg tgaggactca ctcctagtca agggtgtagc attcaagaaa 660
actttcagct acgctggggc tgaacaacag ccaaagaaat tcgtcaatcc aaagatttta 720
ttacttaatt tggaattgga actcaaatcc gaaaaagaaa acgcagaaat tgttatcaat 780
aatccacaag aatatcagaa gataatagat gccgagtata ggataatatt tgagaagctt 840
gagaatgcag tgaaactcgg tgctaatgta gttttatcta aattgccaat tggtgattta 900
gcaacacaat actttgcaga taaaaatgta ttttgtgccg gccgggttga tgaaaatgat 960
cttataagaa cgagtaaagc tactggtgct tctattcaaa ccactctcaa taacctttca 1020
gttgacgtct taggaacttg tggtgtgttt gaggaagtgc aaattgggtc tgaacgttac 1080
aatatgttca cagattgcaa gagtgcaaaa acctgtacaa ttgtgttgag aggtggaggt 1140
cagcagttca ttgatgaatc tgaacgttca ctccatgacg cgattatgat tgtcagaaga 1200
gcaactaaat gtaatactat ccttcccgga gctggtgcca ttgagatgtt gctctcaact 1260
tatctcctcc actattctct caacactatt aatcccacag actctgtcaa ccatgttaac 1320
tgcgttaact ccgtaaatca tgttaatgga gttactgggg tgaataagag tctggtgggt 1380
aagaggcaca taataatgaa cgggtttgca aaggcattgg agtgtattcc aaggaattta 1440
```

```
gccactaatt ctggctacaa ttcaaatgat ttattatcga tactaagaaa taaatacaat 1500
caattggaaa tagtcaatgg agagataaag gtgaataatg aggagagttg gtatggaata 1560
gattgttaca agggaagtgt atgtaacgca tacaaggctt gtatttggga gccgagtttg 1620
gtgaaaaaaa actcaattta ctcagctact gaagcagctt gccttgttct ctcagttgat 1680
gaaactgtca aaaaccaatc cagacaacag ttacaaagcg cactaccaca acccaaataa 1740
```

<210> 20
<211> 468
<212> DNA
<213> Theileria parva

<400> 20

```
atgccgaaaa ataaaggcaa aggaggaaag aaccggagac gcggtaaaaa tgacaatgaa 60
ggcgaaaaaa gagaattagt cttcaaaatg gaagatcaag aatatgctca agtttacgt 120
atgctcggta atggcagact tgaagcctac tgttttgacg gcactaaacg tctttgccat 180
attagaggaa agatgaggaa gcgagtttgg gtaaatgccg gcgatattat tttggtatcg 240
cttagagatt tccaggacag caaggctgac gtgatcgcaa agtacactgc tgaggaggct 300
cgtactctga aggcttacgg cgagttgcct gaagcgacca aaatcaacga aactgacgtg 360
tacgacgacg aggccgacaa ctgcattgac ttccaggacg tatcgtctga atcagaacct 420
gaggatgagt cacaagagga gtcggatttc gatatcgatg atttataa             468
```

<210> 21
<211> 2166
<212> DNA
<213> Theileria parva

<400> 21

```
atgacatcaa aggacgagac acctgatcag gaggtctacg cttttaatgc tgatatctcc 60
cagcttttaa gcttgatcat caacgcattt tatagtaaca aggagatttt ccttcgtgaa 120
ctcattagca acgctagcga cgcactggaa aaaattaggt atgaggcaat caaggatcca 180
aagcaaatcg aggatcaacc cgattactat atcaggctgt atgccgacaa gaacaacaac 240
accctcacaa tcgaagattc cggtattggc atgaccaaag ccgacctcgt gaacaacctc 300
ggtacaattg ccaaatccgg cacaagagca ttcatggagg cactgcaagc aggctcggac 360
atgtcaatga tcggacagtt tggtgtcggt ttctactcag catacctggt cgcagataag 420
gtgacagtag tgtccaagaa caacgcagac gaccagtacg tctgggagtc aacagcctca 480
ggccacttta cagtgaagaa ggacgactcg cacgagccgc tcaaaagagg aactagacta 540
atactgcact tgaaggagga ccaaactgag taccttgagg agagaaggct gaaagagctt 600
gttaagaagc acagcgagtt catttcattc ccaatctcgc tctcagtaga gaagacccag 660
gagaccgagg tcactgacga cgaggcagag ctagacgagg acaagaagcc cgaggaggag 720
aagcccaagg acgataaggt ggaggacgtt actgacgaga aagtgaccga cgtcactgac 780
gaggaggaga aaaaggagga aaagaaaaag aagaagagga aggtcaccaa cgtaacgcgt 840
gagtgggaaa tgcttaacaa gcagaagcca atttggatga gactcccgtc tgaagtcacc 900
aacgaagaat atgcagcgtt ctacaagaac ttaaccaacg attgggaaga ccacttggcc 960
gtgaaacact tcagcgttga gggtcagctt gagttcaaag ctctactgtt cgtcccaaga 1020
agagcgccgt ttgacatgtt cgagtcccgc aaaaagaaaa acaacatcaa gttgtacgtc 1080
agacgcgtat ttatcatgga cgactgtgag gagctcatcc cggagtggct ttcctttgtg 1140
aagggtgtgg tagactcaga ggacctgccc ttgaatattt ctagggaaac tctccagcag 1200
aacaagatcc tcaaggtcat caggaagaac ttggtgaaaa agtgcctcga gctcttcaat 1260
gaactcactg agaagaagga ggacttcaag aagttctacg agcagttcag caagaacctg 1320
aagctgggaa tccacgagga caacgctaat cgctcaaaga tcgccgaact gttgaggttc 1380
gagacaacca agagcggaga cgaactcgtg tcactcaagg agtacgttga caggatgaag 1440
agtgaccaga agtatgtgta ctacatcacg ggagagtcga agcagagcgt agcctcaagt 1500
cctttccttg agaccctgag ggctcgcgac tacgaagtcc tgtacatgac tgacccaatt 1560
gatgagtacg cagttcagca gatcaaggag tttgaaggca agaaactcaa gtgctgtacc 1620
aaggagggcc tggaccttga tgagggcgag gatgaaaaga agtcctttga agcgctcaag 1680
```

gaagaaatgg aacctctttg caagcacatc aaggaagtgc tccacgacaa ggtggaaaag 1740
gtcgtgtgtg gaacaaggtt taccgactct ccatgcgcac ttgtcaccag cgagttcggc 1800
tggagcgcga acatggagcg tatcatgaaa gcacaagctc tcagagactc gtccataaca 1860
agctacatgc tgagcaagaa gatcatggag attaacccga gacatagcat catgaaggag 1920
ctcaaaacta gagctgcaaa cgacaaaaca gataaaaccg tcaaggacct agtctggctt 1980
ctctacgaca cagcgctctt aacctcaggg tttaacctcg atgagcccac ccagtttgga 2040
aacaggatct acaggatgat caagctcgga ctctcattgg acgacgagga acacgtagaa 2100
gaggactcat caatgccgcc gctggatgag cccgttgtcg actccaaaat ggaggaagtt 2160
gactaa 2166

<210> 22
<211> 1323
<212> DNA
<213> Theileria parva

<400> 22

atgcttggaa atcatgtcat gggatctaat tccccccaca ttaaaatttt atcatctgtt 60
acattcttac atattgctaa aatggaagaa gtagaaaacg taaaagtcga cgccttggag 120
cgtgttgaca ctgagtctgt ccttaattat gacactgtgt tagaaaagaa accattgcgc 180
agcagtgttg cctctttctt caaaagatac agtgctgttc tcgtaatatt aactgccgtg 240
ctattattca cattcacttt tgcagcaata gcattgtcat caggcagaag cgcaatcaga 300
aagaacagag aactcctgtc agtcgaattt gaaaagcttc agttcgataa tttcgtgaca 360
attaagggag aaagggaaga ggacttcccc aagatggtag ctgaagttct ttacaaggtt 420
gcagtcgagt ttgacccaaa agaagaggcc ttgatctacg tccagttcaa tgacttcaac 480
aagcaacacg acaagaagca caacaattac aggcacaaga agacctcgta caccaacttc 540
agaaacaacc ttaatgatat aaacgagcac aacgcaaaac caaacctgtc gtacaccaag 600
aacatgaacc acttcggtga catatcatcc aaggatttca tgaagagata caccaagaaa 660
gtactcttga acttgccaaa agaccacgtg tccacctata acaacaacag accaatgtca 720
gttgatctca gaagccatgg tgtattgact ccagtcaagt gccaagaaga aaatgaactc 780
tcatggccat actccgtagt agcagtcgcc gagtcattcg ttaagaagac atcacaaaag 840
accgtatccc tcagcgaaaa acaattagta gattgcgtta cagataagaa atctgcaaac 900
aacccattct tgggttacaa ataccttaag gacttgggtc tgttcgaatc agaactcgta 960
gacaaatcca caaccaagtg cccagcattg gaaggtgaaa gattcaaagt cccatcatac 1020
tcatactcat atgagccaga tttggtggca ctcttgttga atgcaggacc actcactgta 1080
ccagttgcag tgagcgagga ttggcaattc tacgctgatg gaaccttgga tgtatgcggt 1140
gctgaattga accacttctt gaccctagta ggtgtcagct ttgacgaaaa aggcaatcac 1200
tggatactca aaaactcatt cggtgaaggc tggggaaaca agggatacct actgttgact 1260
cgcaatagca aggaatacaa agatgattgt ggattgacct ccttcgcagt gtacgcagtt 1320
taa 1323

<210> 23
<211> 33
<212> DNA
<213> Theileria parva

<400> 23
gtagggtatc caaaggttaa agaagaaatg cta 33

<210> 24
<211> 33
<212> DNA
<213> Theileria parva

<400> 24
agtcatgaag aactaaaaaa attgggaatg cta 33

<210> 25
<211> 33
<212> DNA

<213> Theileria parva

<400> 25
aaatcatcac atggtatggg aaaggtagga aaa 33

<210> 26
<211> 27
<212> DNA
<213> Theileria parva

<400> 26
tttgcacaaa gcctagtgtg cgtatta 27

<210> 27
<211> 27
<212> DNA
<213> Theileria parva

<400> 27
caaagcctag tgtgcgtatt aatgaaa 27

<210> 28
<211> 27
<212> DNA
<213> Theileria parva

<400> 28
actggtgctt ctattcaaac cactctc 27

<210> 29
<211> 27
<212> DNA
<213> Theileria parva

<400> 29
agcaaggctg acgtgatcgc aaagtac 27

<210> 30
<211> 27
<212> DNA
<213> Theileria parva

<400> 30
agcaaggctg acgtgatcgc aaagtac 27

<210> 31
<211> 27
<212> DNA
<213> Theileria parva

<400> 31
tgcggtgctg aattgaacca cttcttg 27

<210> 32
<211> 16
<212> PRT
<213> Theileria parva

<400> 32

Phe Leu Val Gly Tyr Pro Lys Val Lys Glu Glu Met Leu Glu Met Ala
1               5                   10                  15

<210> 33
<211> 27
<212> DNA
<213> Artificial Sequence

<220>
<223> Description of Artificial Sequence: Synthetic Primer

<400> 33
ggatccccgg aaaaagaaga ggaactc 27

<210> 34
<211> 24
<212> DNA
<213> Artificial Sequence

<220>
<223> Description of Artificial Sequence: Synthetic Primer

<400> 34
aatgtagttt tatctaaatt gcca 24

<210> 35
<211> 33
<212> DNA
<213> Artificial Sequence

<220>
<223> Description of Artificial Sequence: Synthetic Primer

<400> 35
ggatccgaaa tggcgaaaaa taaaggcaaa gga 33

<210> 36
<211> 30
<212> DNA
<213> Artificial Sequence

<220>
<223> Description of Artificial Sequence: Synthetic Primer

<400> 36
gccaagaatt cgatgacatc aaaggacgag 30

<210> 37
<211> 28
<212> DNA
<213> Artificial Sequence

<220>
<223> Description of Artificial Sequence: Synthetic Primer

<400> 37

ctgcagttaa tttttgaggt aaattttg 28

<210> 38
<211> 25
<212> DNA
<213> Artificial Sequence

<220>
<223> Description of Artificial Sequence: Synthetic Primer

<400> 38
gaggagataa gttgagagca acatc 25

<210> 39
<211> 31
<212> DNA
<213> Artificial Sequence

<220>
<223> Description of Artificial Sequence: Synthetic Primer

<400> 39
ctgcagttat aaatcatcga tatcgaaatc t 31

<210> 40
<211> 30
<212> DNA
<213> Artificial Sequence

<220>
<223> Description of Artificial Sequence: Synthetic Primer

<400> 40
ggcgcggccg cgtcaacttc ctccattttg 30

<210> 41
<211> 24
<212> DNA
<213> Artificial Sequence

<220>
<223> Description of Artificial Sequence: Synthetic Primer

<400> 41
atggccactt caattgcatt tgcc 24

<210> 42
<211> 24
<212> DNA
<213> Artificial Sequence

<220>
<223> Description of Artificial Sequence: Synthetic Primer

<400> 42
ttaaatgaaa tatttatgag cttc 24

<210> 43

<211> 157
<212> PRT
<213> Theileria parva

<400> 43

```
Met Arg Val Lys Lys Val Leu Leu Tyr Thr Leu Pro Val Val Gly Ile
1               5                   10                  15

Leu Leu Ala Gly Ser Leu Ile Ile Phe Asn Phe Val Arg Lys Arg Pro
            20                  25                  30

Glu Lys Glu Glu Glu Leu Lys Pro Pro Ser Ala Leu Glu Asp Glu Leu
        35                  40                  45

Lys Lys Arg Glu Glu Glu Ser Arg Lys Arg Met Glu Glu Met Gln Lys
    50                  55                  60

Glu Ile Leu Glu Lys Lys Leu Arg Glu Gly Lys Lys Ala Leu Glu Glu
65                  70                  75                  80

Leu Glu Lys Arg Glu Lys Glu Val Val Asp Glu Phe Ala Lys His Leu
                85                  90                  95

Lys Lys Pro Glu Glu Arg Leu Pro Lys Ile Ile Asp Ser Gly Phe Pro
            100                 105                 110

Thr Val Asp Pro Ile Thr Tyr Thr Ser Gly Val Tyr Met Val Ala Val
        115                 120                 125

Ser Lys Thr Thr Phe Thr Ser Asp Ser Asp Leu Val Asp Phe Thr His
    130                 135                 140

Thr Leu Leu Gly Ile Lys Phe Leu Val Thr Gly Val Gln
145                 150                 155
```

<210> 44
<211> 107
<212> PRT
<213> Theileria parva

<400> 44

Met Arg Val Lys Lys Val Leu Leu Tyr Thr Leu Pro Val Val Gly Ile
1 5 10 15

Leu Leu Ala Gly Ser Leu Ile Ile Phe Asn Phe Val Arg Lys Arg Pro
20 25 30

Glu Lys Glu Glu Glu Leu Lys Pro Pro Ser Ala Leu Glu Asp Glu Leu
35 40 45

Lys Lys Arg Glu Glu Glu Ser Arg Lys Arg Met Glu Glu Met Gln Lys
50 55 60

Glu Ile Leu Glu Lys Lys Leu Arg Glu Gly Lys Lys Ala Leu Glu Glu
65 70 75 80

Leu Glu Lys Arg Glu Lys Glu Val Val Asp Glu Phe Ala Lys His Leu
85 90 95

Lys Lys Pro Glu Glu Arg Leu Pro Lys Ile Ile
100 105

<210> 45
<211> 103
<212> PRT
<213> Theileria parva

<400> 45

Met Arg Val Lys Lys Val Leu Leu Tyr Thr Leu Pro Val Val Gly Ile
1 5 10 15

Leu Leu Ala Gly Ser Leu Ile Ile Phe Asn Phe Val Arg Lys Arg Pro
20 25 30

Glu Lys Glu Glu Glu Leu Lys Pro Pro Ser Ala Leu Glu Asp Glu Leu
35 40 45

Lys Lys Arg Glu Glu Glu Ser Arg Lys Arg Met Glu Glu Met Gln Lys
50 55 60

Glu Ile Leu Glu Lys Lys Leu Arg Glu Gly Lys Lys Ala Leu Glu Glu
65 70 75 80

Leu Glu Lys Arg Glu Lys Glu Val Val Asp Glu Phe Ala Lys His Leu
85 90 95

Lys Lys Pro Glu Glu Arg Leu
100

<210> 46
<211> 37
<212> PRT
<213> Theileria parva

<400> 46

Met Arg Val Lys Lys Val Leu Leu Tyr Thr Leu Pro Val Val Gly Ile
1               5                   10                  15

Leu Leu Ala Gly Ser Leu Ile Ile Phe Asn Phe Val Arg Lys Arg Pro
            20                  25                  30

Glu Lys Glu Glu Glu
        35

<210> 47
<211> 66
<212> PRT
<213> Theileria parva

<400> 47

Met Pro Gly Pro Asp Ser Lys Pro Ile Phe Phe Lys Asn Asp Gly Asp
1               5                   10                  15

Lys Phe Leu Arg Cys Val Gly Tyr Pro Lys Val Lys Glu Glu Met Leu
            20                  25                  30

Glu Met Ala Thr Lys Phe Asn Arg Leu Pro Lys Gly Val Glu Ile Pro
        35                  40                  45

Ala Pro Pro Gly Val Lys Pro Glu Ala Pro Thr Pro Thr Pro Thr Thr
    50                  55                  60

Ile Thr
65

<210> 48
<211> 68
<212> PRT
<213> Theileria parva

<400> 48

Met Pro Thr Pro Thr Thr Ile Thr Pro Ser Val Pro Pro Thr Ile Pro
1               5                   10                  15

Thr Pro Ile Thr Pro Ser Ala Pro Pro Thr Thr Pro Pro Thr Gly Leu
            20                  25                  30

Asn Phe Asn Leu Thr Val Gln Asn Lys Phe Met Ile Gly Ser Gln Glu
        35                  40                  45

Val Lys Leu Asn Ile Thr His Glu Tyr Glu Gly Val Tyr Glu Ala His
    50                  55                  60

Lys Tyr Phe Ile
65

<210> 49
<211> 62
<212> PRT
<213> Theileria parva

<400> 49

Met Gly Val Tyr Glu Ala His Lys Tyr Phe Ile Glu Arg Gly Ser Phe
1 5 10 15

Thr Pro Thr Ser Phe Ser Ile Gly Asp Leu Pro Gln Thr Gly Leu Pro
20 25 30

Val Asn Gln Thr Val Asp Thr Ile Val Val Tyr Phe His Arg Val Thr
35 40 45

Met Gly Glu Pro Val Gly Ile Pro Leu Ile Val Leu Ile Phe
50 55 60

<210> 50
<211> 148
<212> PRT
<213> Theileria parva

<400> 50

Met Pro Gly Pro Asp Ser Lys Pro Ile Phe Phe Lys Asn Asp Gly Asp
1 5 10 15

Lys Phe Leu Arg Cys Val Gly Tyr Pro Lys Val Lys Glu Glu Met Leu
20 25 30

Glu Met Ala Thr Lys Phe Asn Arg Leu Pro Lys Gly Val Glu Ile Pro
35 40 45

Ala Pro Pro Gly Val Lys Pro Glu Ala Pro Thr Pro Thr Thr Ile Thr
50 55 60

Pro Ser Val Pro Pro Thr Ile Pro Thr Pro Ile Thr Pro Ser Ala Pro
65 70 75 80

Pro Thr Thr Pro Pro Thr Gly Leu Asn Phe Asn Leu Thr Val Gln Asn
85 90 95

Lys Phe Lys Phe Met Ile Gly Ser Gln Glu Val Lys Leu Asn Ile Thr
100 105 110

His Glu Tyr Glu Gly Val Tyr Glu Ala His Lys Tyr Phe Ile Glu Arg
115 120 125

Gly Ser Phe Thr Pro Thr Ser Phe Ser Ile Gly Asp Leu Pro Gln Thr
130 135 140

Gly Leu Pro Val
145

<210> 51
<211> 121
<212> PRT
<213> Theileria parva

<400> 51

Met Pro Thr Pro Thr Thr Ile Thr Pro Ser Val Pro Pro Thr Ile Pro
1 5 10 15

Thr Pro Ile Thr Pro Ser Ala Pro Pro Thr Thr Pro Pro Thr Gly Leu
20 25 30

Asn Phe Asn Leu Thr Val Gln Asn Lys Phe Lys Phe Met Ile Gly Ser
35 40 45

Gln Glu Val Lys Leu Asn Ile Thr His Glu Tyr Glu Gly Val Tyr Glu
50 55 60

Ala His Lys Tyr Phe Ile Glu Arg Gly Ser Phe Thr Pro Thr Ser Phe
65 70 75 80

Ser Ile Gly Asp Leu Pro Gln Thr Gly Leu Pro Val Asn Gln Thr Val
85 90 95

Asp Thr Ile Val Val Tyr Phe His Arg Val Thr Met Gly Glu Pro Val
100 105 110

Gly Ile Pro Leu Ile Val Leu Ile Phe
115 120

<210> 52
<211> 177
<212> PRT
<213> Theileria parva

<400> 52

Met Pro Gly Pro Asp Ser Lys Pro Ile Phe Phe Lys Asn Asp Gly Asp
1 5 10 15

Lys Phe Leu Arg Cys Val Gly Tyr Pro Lys Val Lys Glu Glu Met Leu
20 25 30

Glu Met Ala Thr Lys Phe Asn Arg Leu Pro Lys Gly Val Glu Ile Pro
35 40 45

Ala Pro Pro Gly Val Lys Pro Glu Ala Pro Thr Pro Thr Thr Ile Thr
50 55 60

Pro Ser Val Pro Pro Thr Ile Pro Thr Pro Ile Thr Pro Ser Ala Pro
65 70 75 80

Pro Thr Thr Pro Pro Thr Gly Leu Asn Phe Asn Leu Thr Val Gln Asn
85 90 95

Lys Phe Lys Phe Met Ile Gly Ser Gln Glu Val Lys Leu Asn Ile Thr
100 105 110

His Glu Tyr Glu Gly Val Tyr Glu Ala His Lys Tyr Phe Ile Glu Arg
115 120 125

Gly Ser Phe Thr Pro Thr Ser Phe Ser Ile Gly Asp Leu Pro Gln Thr
130 135 140

Gly Leu Pro Val Asn Gln Thr Val Asp Thr Ile Val Val Tyr Phe His
145 150 155 160

Arg Val Thr Met Gly Glu Pro Val Gly Ile Pro Leu Ile Val Leu Ile
165 170 175

Phe

<210> 53
<211> 543
<212> PRT
<213> Theileria parva

<400> 53

Met Arg Val Lys Lys Val Leu Leu Tyr Thr Leu Pro Val Val Gly Ile
1 5 10 15

Leu Leu Ala Gly Ser Leu Ile Ile Phe Asn Phe Val Arg Lys Arg Pro
20 25 30

Glu Lys Glu Glu Glu Leu Lys Pro Pro Ser Ala Leu Glu Asp Glu Leu
35 40 45

Lys Lys Arg Glu Glu Glu Ser Arg Lys Arg Met Glu Glu Met Gln Lys
50 55 60

Glu Ile Leu Glu Lys Lys Leu Arg Glu Gly Lys Lys Ala Leu Glu Glu
65 70 75 80

Leu Glu Lys Cys Glu Lys Glu Met Val Asp Glu Phe Glu Lys His Leu
85 90 95

Lys Lys Pro Glu Glu Arg Leu Pro Lys Ile Ile Leu Ile Leu Asp Ser
100 105 110

Gly Phe Pro Thr Val Asp Pro Ile Thr Tyr Thr Ser Gly Val Tyr Met
115 120 125

Val Ala Val Ser Lys Thr Thr Phe Thr Ser Asp Ser Asp Leu Val Asp
130 135 140

Phe Thr His Thr Leu Leu Gly Ile Lys Phe Leu Val Ala Gly Val Gln
145 150 155 160

Phe Gly Gly Lys Thr Tyr Thr Ile Lys Pro Ile Glu Ala Thr Met Ala
165 170 175

Thr Ser Ile Ala Phe Ala Ala Asp Pro Gly Phe Cys Tyr Phe Leu Leu
180 185 190

Ile Pro Gly Pro Asp Ser Lys Pro Ile Phe Phe Lys Asn Asp Gly Asp
195 200 205

Lys Phe Leu Arg Cys Val Gly Tyr Pro Lys Val Lys Glu Glu Ile Ile
210 215 220

Glu Met Ala Thr Lys Phe Asn Arg Leu Pro Lys Gly Val Glu Ile Pro
225 230 235 240

Ala Pro Pro Gly Val Lys Pro Glu Ala Pro Thr Pro Thr Pro Thr Thr
245 250 255

Ile Thr Pro Ser Val Pro Pro Thr Ile Pro Thr Pro Ile Thr Pro Ser
260 265 270

Ala Pro Pro Thr Thr Pro Pro Thr Gly Leu Asn Phe Asn Leu Thr Val
275 280 285

Gln Asn Lys Phe Met Val Gly Ser Gln Glu Val Lys Leu Asn Ile Thr
290 295 300

His Glu Tyr Asp Gly Val Tyr Glu Ala His Lys Tyr Phe Ile Glu Lys
305 310 315 320

Gly Arg Phe Thr Pro Thr Ser Phe Ser Ile Gly Ala Asp Pro Gln Thr
325 330 335

Gly Leu Pro Val Asn Gln Thr Val Asp Thr Ile Val Val Tyr Phe His
340 345 350

Arg Val Thr Met Gly Glu Pro Val Gly Ile Pro Leu Ile Val Leu Val
355 360 365

Phe Tyr Lys Asn Gln Ser Thr Lys Tyr Leu Asn Lys Gly Asn Gly Asn
370 375 380

Trp Glu Glu Ser Lys Ala Leu Leu Phe Arg Glu Glu Leu Asp Phe Leu
385 390 395 400

Asp Ser Met Phe Asn Gly Tyr Val Thr Val Asn Leu Ser Arg Arg Ser
405 410 415

Asp Tyr Tyr Arg Asn Gly Thr Gly Thr Ser Glu Ile Glu Lys Thr Leu
420 425 430

Asp Met Asn Val Tyr Val Glu Pro Asp Thr Pro Cys Leu Gly Trp Thr
435 440 445

Thr Tyr Ile His Lys Leu Glu Glu Gly Gly Glu Gly Gly Ile Glu Lys
450 455 460

Pro Phe Gln Ile Arg Gln Leu Trp Phe Ser Lys Gln Lys Phe Asp Ile
465 470 475 480

Phe Pro Met Gly Lys Val Ser Ile Val Asn Val Tyr Gly Lys Asn Asp
485 490 495

Glu Pro Leu Ser Tyr Ala Pro Ser Ile Phe Ser Val Ile Arg Glu Asp
500 505 510

Gly Ile Gln Ile Phe Tyr Val Arg Ala Tyr Ser Gln Tyr Leu Leu Asp
515 520 525

Ser Ser Val Asn Pro Gln Asn Leu Pro Gln Lys Leu Thr Ala Glu
530 535 540

<210> 54
<211> 72
<212> PRT
<213> Theileria parva

<400> 54

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Met Leu Glu Met Ala Thr Lys Phe Asn Arg Leu Pro Lys Gly Val Glu
50 55 60

Ile Pro Ala Pro Pro Gly Val Lys
65 70

<210> 55
<211> 72
<212> PRT
<213> Theileria parva

<400> 55

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Met Leu Glu Met Ala Thr Lys Phe Asn Arg Leu Pro Lys Gly Val Glu
50 55 60

Ile Pro Ala Pro Pro Gly Val Lys
65 70

<210> 56
<211> 72
<212> PRT
<213> Theileria parva

<400> 56

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Met Leu Glu Met Ala Thr Lys Phe Asn Arg Leu Pro Lys Gly Val Glu
50 55 60

Ile Pro Ala Pro Pro Gly Val Lys
65 70

<210> 57
<211> 72
<212> PRT
<213> Theileria parva

<400> 57

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Met Leu Glu Met Ala Thr Lys Phe Asn Arg Leu Pro Lys Gly Val Glu
50 55 60

Ile Pro Ala Pro Pro Gly Val Lys
65 70

<210> 58

<211> 72
<212> PRT
<213> Theileria parva

<400> 58

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Met Leu Glu Met Ala Thr Lys Phe Asn Arg Leu Pro Lys Gly Val Glu
50 55 60

Ile Pro Ala Pro Pro Gly Val Lys
65 70

<210> 59
<211> 72
<212> PRT
<213> Theileria parva

<400> 59

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Leu Lys Asn Asp
20 25 30

Gly Asp Lys Phe Leu Arg Cys Val Gly Tyr Pro Lys Val Lys Glu Glu
35 40 45

Met Leu Glu Met Ala Thr Lys Phe Asn Arg Leu Pro Lys Gly Val Glu
50 55 60

Ile Pro Ala Pro Pro Gly Val Lys
65 70

<210> 60
<211> 72
<212> PRT
<213> Theileria parva

<400> 60

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Ile Ile Glu Met Ala Thr Lys Phe Asn Arg Leu Pro Lys Gly Val Glu
50 55 60

Ile Pro Ala Pro Pro Gly Val Lys
65 70

<210> 61
<211> 70
<212> PRT
<213> Theileria parva

<400> 61

Met Ala Thr Ser Ile Ala Phe Ala Ala Asp Pro Gly Ile Cys Tyr Phe
1 5 10 15

Leu Leu Ile Pro Ala Pro Lys Pro Ile Phe Phe Lys Asn Asp Gly Asp
20 25 30

Lys Phe Leu Arg Cys Val Gly Tyr Pro Lys Val Lys Glu Glu Ile Ile
35 40 45

Glu Met Ala Thr Lys Phe Asn Arg Leu Pro Lys Gly Val Glu Ile Pro
50 55 60

Ala Pro Pro Gly Val Lys
65 70

<210> 62
<211> 72
<212> PRT
<213> Theileria parva

<400> 62

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Ile Leu Glu Met Ala Thr Lys Phe Asn Arg Leu Pro Lys Gly Val Glu
50 55 60

Ile Pro Ala Pro Pro Gly Val Lys
65 70

<210> 63

<211> 72
<212> PRT
<213> Theileria parva

<400> 63

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Ile Leu Glu Met Ala Thr Lys Phe Asn Arg Leu Pro Lys Gly Val Glu
50 55 60

Ile Pro Ala Pro Pro Gly Val Lys
65 70

<210> 64
<211> 72
<212> PRT
<213> Theileria parva

<400> 64

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Ile Ile Glu Met Ala Thr Lys Phe Asn Arg Leu Pro Lys Gly Val Glu
50 55 60

Ile Pro Ala Pro Pro Gly Val Lys
65 70

<210> 65
<211> 72
<212> PRT
<213> Theileria parva

<400> 65

Met Ala Thr Ser Ile Ala Phe Ala Ala Asp Pro Gly Phe Cys Tyr Phe
1 5 10 15

Leu Leu Ile Pro Gly Pro Asp Ser Lys Pro Ile Phe Phe Lys Asn Asp
20 25 30

Gly Asp Lys Phe Leu Arg Cys Val Gly Tyr Pro Lys Val Lys Glu Glu
35 40 45

Ile Ile Glu Met Ala Thr Lys Phe Asn Arg Leu Pro Lys Gly Val Glu
50 55 60

Ile Pro Ala Pro Pro Gly Val Lys
65 70

<210> 66
<211> 72
<212> PRT
<213> Theileria parva

<400> 66

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Ile
35 40 45

Gly Ser Gln Glu Val Lys Leu Asn Ile Thr His Glu Tyr Glu Gly Val
50 55 60

Tyr Glu Ala His Lys Tyr Phe Ile
65 70

<210> 67
<211> 72
<212> PRT
<213> Theileria parva

<400> 67

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Ile
35 40 45

Gly Ser Gln Glu Val Lys Leu Asn Ile Thr His Glu Tyr Glu Gly Val
50 55 60

Tyr Glu Ala His Lys Tyr Phe Ile
65 70

<210> 68
<211> 72

<212> PRT
<213> Theileria parva

<400> 68

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Ile
35 40 45

Gly Ser Gln Glu Val Asn Leu Asn Ile Thr His Glu Tyr Glu Gly Val
50 55 60

Tyr Glu Ala His Lys Tyr Phe Ile
65 70

<210> 69
<211> 72
<212> PRT
<213> Theileria parva

<400> 69

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Ile
35 40 45

Gly Ser Gln Glu Val Asn Leu Asn Ile Thr His Glu Tyr Glu Gly Val
50 55 60

Tyr Glu Ala His Lys Tyr Phe Ile
65 70

<210> 70
<211> 72
<212> PRT
<213> Theileria parva

<400> 70

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Ile
35 40 45

Gly Ser Gln Glu Val Lys Leu Asn Ile Thr His Glu Tyr Glu Gly Val
50 55 60

Tyr Glu Ala His Lys Tyr Phe Ile
65 70

<210> 71
<211> 64
<212> PRT
<213> Theileria parva

<400> 71

Pro Glu Ala Pro Thr Pro Thr Pro Thr Pro Ile Thr Pro Ser Ala Pro
1 5 10 15

Pro Thr Thr Pro Pro Thr Thr Pro Pro Lys Gly Leu Asn Phe Asn Leu
20 25 30

Thr Leu Gln Asn Lys Phe Met Ile Gly Ser Gln Glu Val Lys Leu Ser
35 40 45

Ile Thr His Glu Tyr Asp Gly Val Tyr Glu Ala His Lys Tyr Phe Ile
50 55 60

<210> 72
<211> 72
<212> PRT
<213> Theileria parva

<400> 72

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Val
35 40 45

Gly Ser Gln Glu Val Lys Leu Asn Ile Thr His Glu Tyr Asp Gly Val
50 55 60

Tyr Glu Ala His Lys Tyr Phe Ile
65 70

<210> 73
<211> 72
<212> PRT
<213> Theileria parva

<400> 73

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Val
35 40 45

Gly Ser Gln Glu Val Lys Leu Asn Ile Thr His Glu Tyr Asp Gly Val
50 55 60

Tyr Glu Ala His Lys Tyr Phe Ile
65 70

<210> 74
<211> 72
<212> PRT
<213> Theileria parva

<400> 74

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Val
35 40 45

Gly Ser Gln Glu Val Lys Leu Asn Ile Thr His Glu Tyr Glu Gly Val
50 55 60

Tyr Glu Ala His Lys Tyr Phe Ile
65 70

<210> 75
<211> 72
<212> PRT
<213> Theileria parva

<400> 75

Pro Glu Ala Pro Thr Pro Thr Pro Thr Thr Ile Thr Pro Ser Val Pro
1 5 10 15

Pro Thr Ile Pro Thr Pro Ile Thr Pro Ser Ala Pro Pro Thr Thr Pro
20 25 30

Pro Thr Gly Leu Asn Phe Asn Leu Thr Val Gln Asn Lys Phe Met Ile
35 40 45

Gly Ser Pro Glu Val Lys Leu Asn Ile Thr His Glu Tyr Glu Gly Val
50 55 60

Tyr Glu Ala His Lys Tyr Phe Ile
65 70

<210> 76

<211> 72
<212> PRT
<213> Theileria parva

<400> 76

```
Pro Glu Ala Pro Thr Pro Thr Pro Thr Thr Ile Thr Pro Ser Val Pro
1               5                   10                  15

Pro Thr Ile Pro Thr Pro Ile Thr Pro Ser Ala Pro Pro Thr Thr Pro
            20                  25                  30

Pro Thr Gly Leu Asn Phe Asn Leu Thr Val Gln Asn Lys Phe Met Val
        35                  40                  45

Gly Ser Gln Glu Val Lys Leu Asn Ile Pro His Glu Tyr Asp Gly Val
    50                  55                  60

Tyr Glu Ala His Lys Tyr Phe Ile
65                  70
```

<210> 77
<211> 72
<212> PRT
<213> Theileria parva

<400> 77

```
Pro Glu Ala Pro Thr Pro Thr Pro Thr Thr Ile Thr Pro Ser Val Pro
1               5                   10                  15

Pro Thr Ile Pro Thr Pro Ile Thr Pro Ser Ala Pro Pro Thr Thr Pro
            20                  25                  30

Pro Thr Gly Leu Asn Phe Asn Leu Thr Val Gln Asn Lys Phe Met Val
        35                  40                  45

Gly Ser Gln Glu Val Lys Leu Asn Ile Thr His Glu Tyr Asp Gly Val
    50                  55                  60

Tyr Glu Ala His Lys Tyr Phe Ile
65                  70
```